(19) Europäisches Patentamt
European Patent Office
Office européen des brevets

(11) EP 2 492 283 A1

(12) **EUROPEAN PATENT APPLICATION**

(43) Date of publication:
**29.08.2012 Bulletin 2012/35**

(51) Int Cl.:
***C07K 16/12*** *(2006.01)* ***G01N 33/569*** *(2006.01)*

(21) Application number: **11156131.2**

(22) Date of filing: **28.02.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Université de Strasbourg**
**67000 Strasbourg (FR)**

(72) Inventors:
- **Prevost, Gilles**
  **67170 Brumath (FR)**
- **Barasino, Charline**
  **68200 Mulhouse (FR)**
- **Keller, Daniel**
  **67200 Strasbourg (FR)**
- **Masoud, Khaidoun**
  **Afamnia Latakia (SY)**

(74) Representative: **Novagraaf Technologies**
**122 rue Edouard Vaillant**
**92593 Levallois-Perret Cedex (FR)**

(54) **Immunoabsorbed anti-staphylococcal enterotoxins polyclonal antibodies, preparation and uses thereof**

(57) The present invention relates to the preparation of immunoabsorbed polyclonal antibodies raised against staphylococcal enterotoxins, and their use for multiplex detection.

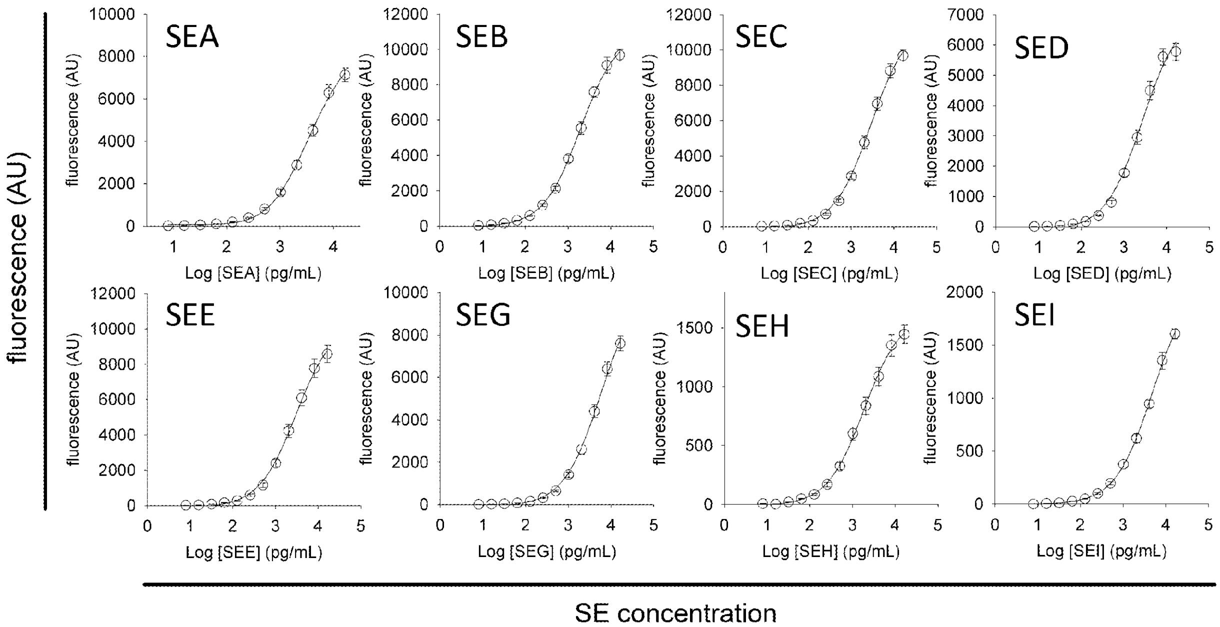


FIGURE 4

EP 2 492 283 A1

## Description

### Technical domain

The present invention relates to the multiplex titration of staphylococcal enterotoxins allowing simultaneous specific and quantitative detections with lower limits and with a higher magnitude of response. To this aim, the present invention uses affinity-purified and immunoabsorbed polyclonal antibodies raised against staphylococcal enterotoxins.
The present invention finds an application in dairy products but also in human clinics, veterinary clinics, drugs control, meat and egg products.
In the specification below, references in square brackets ([ ]) refer to the list of references presented at the end of the text.

### State of the art

An enterotoxin is a protein toxin released by a microorganism in the intestine. Enterotoxins are chromosomally encoded exotoxins that are produced and secreted from several bacterial organisms, for example *Escherichia coli O157: H7*, *Clostridium perfringens*, *Vibrio cholerae*, *Staphylococcus aureus* (also known as golden cluster seed, seed gold, or golden staph), *Yersinia enterocolitica*, *Shigella dysenteriae.* They are often heat-stable, and are of low molecular weight and water-soluble. Enterotoxins are frequently cytotoxic and kill cells by altering the apical membrane permeability of the mucosal (epithelial) cells of the intestinal wall. They are mostly pore-forming toxins (mostly chloride pores), secreted by bacteria, that assemble to form pores in cell membranes. This causes the cells to die.
Staphylococcal enterotoxins (SEs) are basic proteins highly toxic produced by certain *Staphylococcus* strains in a variety of environments, including food substrates. SEs are exoproteins with a molecular weight between 22 and 29kDa showing neurotoxic properties in humans. In addition, SEs are powerful superantigens that stimulate non-specific T-cell proliferation, and share close phylogenetic relationships, with similar structures and activities. To date, 23 different SEs have been identified. Types of toxins SEA, SEB, SEC, SED and SEE represent "conventional" toxins because well-characterized and identified for many years, and which involvement in cases of food poisoning has been shown by many authors [Jones and Kahn, J. Bacteriol., 166 : 29-33, 1986; Betley and Mekalanos, J. Bacteriol., 170(1) : 34-41, 1988; Couch and al., J. Bacteriol., 170 : 2954-2960, 1988; Bayles and Iandolo, J. Bacteriol., 171 : 4799-4806, 1989; Dingues and al., Clin. Microbiol. Rev., 13 : 16-34, 2000] [1-5]. Serotype C was subdivided in groups (SEC1, SEC2, SEC3, $SEC_{bovine}$, $SEC_{ovine}$, $SEC_{goat}$ and $SEC_{canine}$) classified according to differences of superantigen activity and depending on the host to which they are associated [Bergdoll and al., J. Bacteriol., 90(5) : 1481-1485, 1965; Marr and al., Infect. Immun., 61 : 4254-4262, 1993] [6, 7]. A staphylococcal enterotoxin SEF was described in 1981 [Bergdoll, Lancet, 1 : 1017-1021, 1981] [8], but was renamed a few years later TSST1 given its lack of emetic activity unlike other conventional enterotoxins [Blomster-Hautamaa and al., J. Biol. Chem., 261 : 15783-15786, 1986] [9]. Since the mid-1990s, from the genome analysis of *S. aureus,* several genes with strong sequence homology with genes of conventional enterotoxins have been identified. These genes encode proteins with structural properties similar to those of enterotoxins whose emetic activity has rarely been demonstrated. In 2004, a new nomenclature for the superantigens expressed by *S. aureus* has been proposed for the designation of these new enterotoxins [Lina and al., J. Infect. Dis., 189(12) : 2334-2336, 2004] [10]. Accordingly, only the toxins inducing emetic activity after oral administration in animals have been designated staphylococcal enterotoxins (SEs), namely types of toxins SEA, SEB, SEC, SED, SEE, SEG, SEH and SEI. Other toxins, for which no emetic activity has been demonstrated *in vivo,* have been called "staphylococcal enterotoxin-like" to indicate that their role in food poisoning was not confirmed [Ren et al., J. Exp. Med., 180(5) : 1675-1683, 1994; Jarraud and al., J. Immunol., 166(1) : 669-677, 2001; Orwin and al., Infect. Immun., 69(1) : 360-366, 2001; Letertre and al., Mol. Cell Probes, 17 : 227-235, 2003; Omoe and al., J. Clin. Microbiol., 40 : 857-862, 2002; Su and Wong, J. Food Prot., 59(3) : 327-330, 1996; Munson and al., Infect. Immun., 66 : 3337-3348, 1998] [11-17]. In 2008, two new enterotoxins SES and SET were cloned and purified which show an emetic activity in animals, as newly confirmed for the enterotoxin SER [Ono and al., Infect. Immun., 76(11) : 4999-5005, 2008] [18].
The most sensitive methods to detect toxins are the polymerase chain reaction (PCR) and enzyme-linked immunoabsorbent assay (ELISA). The PCR-related methods are more suitable for detection of organisms, such as bacteria and viruses, from which nucleic acids can be extracted for specific amplification. The ELISA-based methods are more robust for detection of toxins, which are often proteins in nature, using specific polyclonal or monoclonal antibodies when available. In comparison with the PCR method, the ELISA-based detection is less sensitive to the matrix effect and presumably gives fewer false-positive results, and ELISA can be addressed to thermostable proteins while bacteria may disappear in slightly heated foods.
All the staphylococcal toxins share similarities in amino acid sequence homology ranging from 15,5% (between SEB and SEK) to 81 % (between SEA and SEE) which can lead to lack of specificity of tools used for their detection. Moreover up to date, the titration of staphylococcal enterotoxins is achieved antigen by antigen (which is extremely time consuming) and it is proposed to a limited number (4 or 5) of staphylococcal enterotoxins involved in food-born intoxination issued from dairy products. Commercial kits are qualitative but when quantitative, they generally lack specificity (which does not allow to easily conduct epidemiological links) and/or sensitivity (which puts the tests over the limits of human toxicity threshold of staphylococcal enterotoxins). Furthermore no commercial kit has been able to fulfil national and international standards (e.g. AFNOR in France).

Therefore, there is a real need to identify new methods for detection and specific titration of staphylococcal enterotoxins, that overcomes the shortcomings, disadvantages and obstacles of prior art, and thereby improving the management of staphylococcal enterotoxins contaminations, notably those induced by enterotoxins from *Staphylococcus aureus*, while reducing costs and time consumption.

**Description of the invention**

The Inventors have now developed unexpectedly a multiplex assay that allows simultaneously the specific titration of at least staphylococcal enterotoxins A, B, C, D, E, G, H and I. Any of the cited antigens are expressed in a recombinant form that remains functional and does not harbour significant difference in their epitopes. These antigens are used for immunizations, and serve as controls in the test assays. The titration was based on the Luminex® technology and uses rabbit polyclonal antibodies that were affinity-purified, and secondarily immunoabsorbed against cross-reacting enterotoxins to reach a strict specificity which preserves affinity and sensitivity of a bulk of specific polyclonal antibodies only for the corresponding antigen. The multiplex titration finally allows simultaneous specific and quantitative detections with lower limits close to 50-80pg/g of initial products, e.g. culture supernatants, dairy products, human serum and with at least a more than two logarithmic magnitude of response. Such a simultaneous titration offers homogeneity and possibilities to detect toxin concentrations that would remain below the human toxicity threshold. Such a simultaneous titration conjoined to a low consummation of developed product bring decreased cost compared to those actually observed for these assays.

The present invention, therefore, relates to an immunological composition comprising a mixture of at least two immunoabsorbed polyclonal antibodies each raised against a staphylococcal enterotoxin, and a pharmaceutically acceptable carrier.

According to the present invention, it may be in particular immunoabsorbed polyclonal antibodies each raised against a staphylococcal enterotoxin chosen from the group consisting of the staphylococcal enterotoxins A, B, C, D, E, G, H, and I.

"Immunoabsorbed polyclonal antibodies" within the meaning of the present invention means a set of polyclonal antibodies each raised against a given staphylococcal enterotoxin (immunogen) that are affinity-purified and secondarily immunoabsorbed against cross-reacting enterotoxin(s) to reach a strict specificity which preserves affinity and sensitivity of a bulk of specific polyclonal antibodies only for the corresponding given staphylococcal enterotoxin (immunogen).

"Pharmaceutically acceptable carrier" within the meaning of the present invention means any and all solvents, disintegrating agents, binders, excipients, lubricants, absorption delaying agents and the like.

"Staphylococcal enterotoxins A, B, C, D,E, G, H and I" within the meaning of the present invention means the staphylococcal enterotoxins (SEs), namely types of toxins SEA, SEB, SEC, SED, SEE, SEG, SEH and SEI, produced by *Staphylococcus aureus*.

The present invention also relates to a method for multiplex detection of staphylococcal enterotoxins, comprising:

a) contacting a sample with an immunological composition of the invention ;
b) detecting potential immunological complexes formed.

"A sample" within the meaning of the present invention means any culture media, biological samples (e.g. human clinical samples) or food extracts that are susceptible of being contaminated by staphylococcal enterotoxins. For example, it includes culture supernatants, human serum, dairy/egg/meat/prepared meals/sea foods products /spices/ drugs and surface of medical devices, and any extracts or protein preparation issued from the cited stuffs.

"Immunological complexes" within the meaning of the present invention means the complexes formed between the polyclonal antibody and its immunogen (namely the given staphylococcal enterotoxin used for immunization).

The detection of said immunological complexes can be performed by any means known in the art. For example, it includes protein mass - based technologies (*e.g.* protein CHIPS assisted by SELDI-TOF or MALDI-TOF, fluorescence-based technologies (*e.g.* Luminex technology), surface plasmon resonnance-based technologies (biosensors).

According to a particular embodiment of the present invention, said method for multiplex detection can also include a step c) wherein the detection results obtained in step b) are compared to a negative and / or positive control.

"Negative and/or positive control" within the meaning of the present invention means a control sample comprising or not at least one staphylococcal enterotoxin. This control allows to compare the detection results obtained in step b) of the method of the invention and to detect a false positive or false negative, if any.

According to a particular embodiment of the present invention, said method of multiplex detection can also include a step $c_{bis}$) wherein the detection results obtained in step b) are compared to a standard of measurement; allowing a qualitative and / or quantitative analysis of staphylococcal enterotoxins.

"Standard of measurement" within the meaning of the present invention means one or more analysis carried out on solutions manufactured for analytical purposes according to the invention and comprising a known staphylococcal enterotoxin(s) content. From the analysis results, it is thus possible to determine the presence of staphylococcal enterotoxin(s) in the sample as well as its concentration by comparison, for example using a standard curve made from standards or measurement.

According to the present invention, steps c) and $c_{bis}$) can be both implemented, before or after steps a) and b) of the method of the invention. According to the present invention, steps c) and $c_{bis}$) can be implemented simultaneously or sequentially, including to steps a) and b), for example on a multi-well plate for performing several analysis simultaneously or sequentially, including measures of standards.

The present invention also relates to the use of an immunological composition of the invention, as a diagnostic tool of a staphylococcal enterotoxin(s) contamination.

Such a diagnostic tool allows a qualitative and/or quantitative diagnosis of a staphylococcal enterotoxin(s) contamination.

The present invention also relates to a kit for the multiplex detection of staphylococcal enterotoxins, comprising an immunological composition of the invention and means for the detection of immunological complexes.

"Means for the detection of immunological complexes" within the meaning of the present invention means any means well known in the art. For example, it includes Cy5, phycoerythrin or any other fluorescent dyes, fluorescent metabolites issued from enzymes activity, Matrix assisted laser desorption ionization procedures, Fluorescent resonance energy transfer, surface plasmon resonance.

The present invention also relates to a method for the preparation of an immunoabsorbed polyclonal antibody raised against a staphylococcal enterotoxin, comprising :

a) providing an anti-enterotoxin polyclonal antibody previously obtained from the immunization of a non-human animal with a given staphylococcal enterotoxin ;

b) purification of said anti-enterotoxin polyclonal antibody using at least two successive immunoabsorption steps against immunization-unrelated staphylococcal enterotoxins and which order is chosen to abolish cross-reactions with said immunization-unrelated staphylococcal enterotoxins from the strongest cross-reaction to the weaker cross-reaction.

"Immunization-unrelated staphylococcal enterotoxins" within the meaning of the present invention means given staphylococcal enterotoxin(s) Y (SEY) that differ(s) from a given staphylococcal enterotoxin X (SEX), used as immunogen for obtaining a polyclonal antibody raised against said given staphylococcal enterotoxin X (SEX), but that is responsible for a cross reaction with said anti-SEX polyclonal antibody.

For example, step b) is carried out by purification of an affinity purified anti-SE polyclonal antibody on an immunoabsorption column with a first immunization-unrelated staphylococcal enterotoxin immobilized responsible for a cross reaction. If a cross reaction remains, a new purification is carried out on an immunoabsorption column with the same or another immunization-unrelated staphylococcal enterotoxin responsible for the cross reaction. These purification steps are repeated until obtaining monospecificity of immunoabsorbed Ab with respect to its immunogen. The order of successive columns can be easily determined by one skilled in the art according to the extent of each cross reaction detected, namely from the stronger cross reactivity to the weaker cross-reactivity. Preferably, said method for the preparation of an immunoabsorbed anti-SE polyclonal antibody can also include washing steps between each purification step.

According to a particular embodiment of the present invention, said method for the preparation of an immunoabsorbed anti-SE polyclonal antibody can also include a step c) wherein the specificity of said polyclonal antibody is monitored by any means well known in the art. For example, the specificity of the immunoabsorbed anti-SE polyclonal antibody can be controlled by ELISA, DOT-BLOT against its immunogen and/or one or more parent staphylococcal enterotoxin(s) to detect the presence of remaining cross reaction, if any. Preferably, one or more analysis is(are) carried out on solutions manufactured for analytical purposes according to the invention and comprising a known immunogen or immunization-unrelated staphylococcal enterotoxin content. From the analysis results, it is thus possible to determine the presence of any cross reaction as well as its extent by comparison, for example using a standard curve made from standards of measurement.

According to the present invention, step c) can be implemented between each immunoabsorption step and/or at the end of step b). Preferably step c) is implemented between each immunoabsorption step to detect remaining cross reaction(s), determine the extent of each cross reaction detected, and thus determine the necessary next immunoabsorption step(s) to achieve monospecificity.

According to a particular embodiment of the present invention, the method for the preparation of an immunoabsorbed polyclonal antibody against SEA comprises a step b) wherein 5 successive immunoabsorption steps against the immunization-unrelated staphylococcal enterotoxin E, E, I, B, then D are implemented.

According to a particular embodiment of the present invention, the method for the preparation of an immunoabsorbed polyclonal antibody against SEB comprises a step b) wherein 3 successive immunoabsorption steps against the immunization-unrelated staphylococcal enterotoxin C1, C1, then G are implemented.

According to a particular embodiment of the present invention, the method for the preparation of an immunoabsorbed polyclonal antibody against SEC comprises a step b) wherein 3 successive immunoabsorption steps against immunization-unrelated staphylococcal enterotoxin B, B, then G are performed.

According to a particular embodiment of the present invention, the method for the preparation of an immunoabsorbed polyclonal antibody against SED comprises a step b) wherein 3 successive immunoabsorption steps against the immunization-unrelated staphylococcal enterotoxin A, A, then E are performed.

According to a particular embodiment of the present invention, the method for the preparation of an immunoabsorbed polyclonal antibody against SEE comprises a step b) wherein 4 successive immunoabsorption steps against the immunization-unrelated staphylococcal enterotoxin A, A, I, then C1 are performed.

According to a particular embodiment of the present invention, the method for the preparation of an immunoabsorbed polyclonal antibody against SEG comprises a step b) wherein 4 successive immunoabsorption steps against the immunization-unrelated staphylococcal enterotoxin A, B, I, then C1 are performed.

According to a particular embodiment of the present invention, the method for the preparation of an immunoabsorbed polyclonal antibody against SEH comprises a step b) wherein 2 successive immunoabsorption steps against the immunization-unrelated staphylococcal enterotoxin B, then D.

According to a particular embodiment of the present invention, the method for the preparation of an immunoabsorbed polyclonal antibody against SEI comprises a step b) wherein 5 successive immunoabsorption steps against immunization-unrelated staphylococcal enterotoxin E, C1, G, B, then A are performed.

The present invention also relates to an immunoabsorbed anti-SE polyclonal antibody raised against a staphylococcal enterotoxin obtained by a method for the preparation an immunoabsorbed anti-enterotoxin polyclonal antibody of the invention. Such immunoabsorbed anti-enterotoxin polyclonal antibodies differ from those of the art by a different population of antibodies, affinity and specificity to the corresponding antigen.

For example immunoabsorbed anti-SE polyclonal antibodies of the invention are available free in solutions or adsorbed on a support, for example a bead (microsphere or nanoparticle), but can be adsorbed also onto a protein microarray, a functionalized multiwell plate, etc.... Preferably, such polyclonal antibodies are adsorbed on beads, more preferably on beads of an unique color-code according to the strict specificity of the immunoabsorbed anti-SE polyclonal antibody of the invention adsorbed thereon.

**Brief description of the figures**

- Figure 1 represents the pGEX-6P-1 expression vector containing a resistance gene to ampicillin and two restriction sites for BamH1 (loc.945) and EcoR1 (loc. 954).
- Figure 2 represents the amino acids sequences of staphylococcal enterotoxinsSEA-1.
- Figure 3 represents the synthesis of the calibration curves for plates 100 to 104.
- Figure 4 represents the titration curves of staphylococcal enterotoxins SEA-I.
- Figure 5 represents the LOD and LOQ for plates 100 to 104 +/- standard deviation.
- Figure 6 represents the linearity of standard for plates 100 to104.
- Figure 7 represents SEs titration on Munster after caseins precipitation and delipidation
- Figure 8 represents SEs titration on concentrated matrix after caseins precipitation and delipidation.

**EXAMPLES**

**EXAMPLE 1: ORIGINS OF THE 8 STAPHYLOCOCCAL ENTEROTOXINS**

**Sources of the basic *Staphylococcus aureus* strains used for cloning**

The genes fragments corresponding to the 8 Staphylococcal enterotoxins (SEs) secreted sequences were obtained from strict amplification (Phusion™ High Fidelity DNA Polymerase) of genes from strains listed in the table 1 below.

Table 1

| Enterotoxin | Strain (genotype) | Source |
|---|---|---|
| **SEA** | FRI722 (sea) | DeBuyser |
| **SEB** | S6 | DeBuyser |
| **SEC** | FRI137 (*sec1, seg, seh, sei)* | DeBuyser / AFSSA |
| **SED** | FRI361 (*sec*, *sed*, *seg*, *sei*) / FRI1157m | DeBuyser |
| **SEE** | FRI 326 | DeBuyser |
| **SEG** | FRI137 (*sec*, *seg*, *seh*, *sei*) | AFSSA |
| **SEH** | FRI137 (*sec*, *seg*, *seh*, *sei*) | AFSSA |
| **SEI** | FRI137 (*sec*, *seg*, *seh*, *sei*) | AFSSA |

Cloned genes have been checked by nucleotide sequencing. Oligonucleotides adjustable to the plasmid pGEX-6P-1 were used for an insertion either in the *Eco*R1 cut site (protein + 8 amino acids), or in the *Bam*H1 cut site (protein +5 amino acids). Antigens are over-expressed from recombinant clones pGEX-6P-1 (SEA+5, SEB+5, SEC1+8, SEE+5, SEG+8, SEH+5 and SEI+5) transforming BL21 as Glutathion S-Transferase (GST) fusion proteins, then purified by glutathion affinity (Sepharose4B GSH) followed by a cation exchange chromatography (Mono S), adapted to each SE. All SEs have purity over 90-95% in SDS-PAGE. SEA+5, SEB+5, SEC1 +8 are recognized by the kit Oxoid SET RPLA and SEE+5 by Oxoid anti-SEA from the same kit.

**Bacterial strains**

*Escherichia. coli* XL1-Blue

Genotype : *recA1 endA1 gyrA96 thi-1 hsdR17 supE44 relA1 lac* [F' *proAB lacI*$^q$Z$\Delta$*M15* Tn*10* (Tet$^r$)]

*Escherichia coli* BL21

Genotype : *E. coli* B F- *dcm ompT hsdS* ($r_B$- $m_B$-) *gal*

**Plasmid Vector (pGEX-6P-1)**

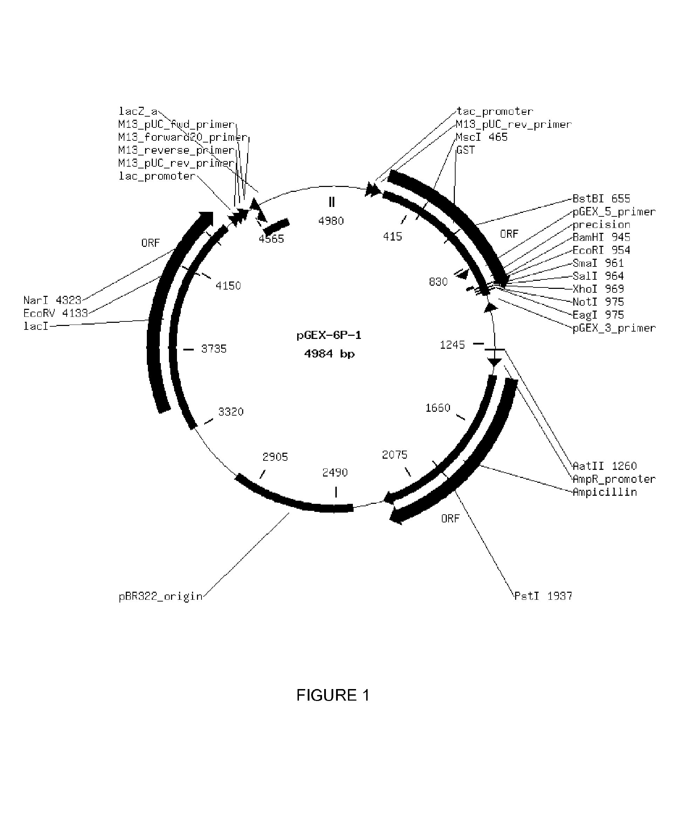

It is a 4984 pb vector (figure 1) containing an ampicillin resistance gene, a gene coding to the Glutathion-S-Transferase (GST) and a multiple cloning region located in 3' of that gene.

Cloned sequences in multiple restriction/insertion sites of pGEX-6P-1 vector can be expressed as GST fusion proteins located at its N-terminal extremity [Smith and Johnson, Gene, 67(1) : 31-40, 1988] [19]. Expression is under the control of the *ptac* promoter, upstream the GST coding gene, which is induced by the isopropyl β-D thiogalactopyranoside (IPTG). The fusion protein expressed in the pGEX-6P-1™ can easily be purified by affinity chromatography using a Glutathion Sepharose 4B™ column (GE Healthcare, Orsay). The fusion protein contains a specific proteolysis site using PreScission™ Protease (GE Healthcare, Orsay), and is located between the GST and its fusion partner.

**Recombined vectors**

Recombined vectors used are made in the first part of work by the cloning method (table 2).

Table 2

| Plasmids | Relatives characteristics (Origin) |
|---|---|
| pGEX-6P-1 | Ap$^r$, GST expression vector (Pharmacia) |
| pPAX1 | Ap$^r$, pGEX-6P-1 carrying *sea* (Gravet et al.) |
| pKBX1 | Ap$^r$, pGEX-6P-1 carrying *seb* (That work) |
| pKCX1 | Ap$^r$, pGEX-6P-1 carrying *sec1* (That work) |
| pPDX1 | Ap$^r$, pGEX-6P-1 carrying *sed* (That work) |
| pKEX1 | Ap$^r$, pGEX-6P-1 carrying *see* (That work) |

(continued)

| Plasmids | Relatives characteristics (Origin) |
|---|---|
| pKGX1 | $Ap^r$, pGEX-6P-1 carrying *seg* (That work) |
| pKHX1 | $Ap^r$, pGEX-6P-1 carrying *seh* (That work) |
| pKIX1 | $Ap^r$, pGEX-6P-1 carrying *sei* (That work) |

**DNA primers used for genetic amplification (PCR)**

Oligonucleotides used for the genetic amplification of the genes coding the staphylococcal enterotoxins are shown in the table 3 below.

Table 3

| Gene | Accession N° | Position | Primers | Primers sequence 5'→3' | Tm (°C) |
|---|---|---|---|---|---|
| *seb* | M11118 | 325-1041 | *Bam*H1-setBf | AGTAAGGATCCGAGAGAGTCAAC CAGATCCTAAACCA (SEQ ID NO: 1) | 75 |
| | | | *EcoR*1-setBr | AAGTATAGAATTCACTAACACTTC ATATTTACTTTCTAAAAT (SEQ ID NO: 2) | 64 |
| *sec1* | XO5815 | 199-915 | *EcoR*1-setC1f | AAGTATAGAATTCGAGAGCCAAC CAGACCCTACGCCAGA (SEQ ID NO: 3) | 79 |
| | | | *EcoR*1-setC1r | AAGTATAGAATTCTACTTTCACTT TTTATATCAAAATGG (SEQ ID NO: 4) | 67 |
| *sed* | M28521 | 445-1065 | *EcoR*1-setDf | AAGTATAGAATTCAAACATTCTTAT GCAGATAAAAATCCAAT (SEQ ID NO: 5) | 64 |
| | | | *EcoR*1-setDr | AAGTATAGAATTCTTGAAATGGCT TTAGTGTCTGATGTT (SEQ ID NO: 6) | 63 |

(continued)

| Gene | Accession N° | Position | Primers | Primers sequence 5'→3' | Tm (°C) |
|---|---|---|---|---|---|
| *see* | M21319 | 82-771 | *Bam*H1-setEf | AGTAAGGATCCAGCGAAGAAATA AATGAAAAAGATTT (SEQ ID NO: 7) | 65 |
| | | | *Eco*R1-setEr | AAGTATAGAATTCAGTTGTGTATA AATACAAATCAATAT (SEQ ID NO: 8) | 57 |
| *seg* | AF064773 | 229-927 | *Eco*R1-seGf | AGTAAGGATCCCAACCCGATCCT AAATTAGACGAAC (SEQ ID NO: 9) | 68 |
| | | | *Eco*R1-seGr | AAGTATGAATTCTCAGTGAGTATT AAGAAATACTTCCATTTT (SEQ ID NO: 10) | 62 |
| *seh* | U11702 | 280-930 | *Bam*H1-setHf | AGTAAGGATCCGAAGATTTACAC GATAAAAGTGAGTT (SEQ ID NO: 11) | 64 |
| | | | *Eco*R1-setHr | AAGTATAGAATTCTGATCTAGAAA TTTTCATTGATTATA (SEQ ID NO: 12) | 58 |
| *sei* | AF064774 | 226-879 | *Bam*H1-setIf | AGTAAGGATCCCAAGGTGATATT GGTGTAGGTAACTT (SEQ ID NO: 13) | 65 |
| | | | *Eco*R1-setIr | AAGTATAGAATTCGTTACTATCTA CATATGATATTTCG (SEQ ID NO: 14) | 57 |

**Media**

- 2 x TY (Trypcase-Yeast extract) medium : 1.6% (p/v) bio-trypcase (BioMérieux), 1% (p/v) yeast extract (Bio-Rad), 0.5% (p/v) NaCl pH 7.4. The agar medium contains 1.5% (p/v).

- 2 x TYA medium : 2 x TY medium containing 100µg/mL of ampicilline.

- M9 medium : 15 g/L $Na_2HPO_4$-$12H_2O$, 3 g/L $KH_2PO_4$, 1 g/L $NH_4Cl$, 0.5 g/L NaCl, 15 g/L agar. Following the autoclave add : 1 mL 0.1 M $CaCl_2$, 1 mL 1M $MgSO_4$, 1 mL 1 M Thiamine-HCl, 2mL 20% (p/v) Glucose

- Strains freezing medium : 90% (v/v) Brain Heart Infusion (Difco), 10% (v/v) glycerol (87%).

**Reagents and buffers**

TEB 10 X : 0.89 M Tris-base, 0,89 M boric acid, 25 mM EDTA-$Na_2$ (Titriplex III), pH 8.3.
TE : 10 mM Tris-HCl, 1 mM EDTA-Na2, pH 8.0.
TEG : 25 mM Tris-HCl, 50 mM Glucose, 10 mM EDTA; pH 8.0.
Alkaline Lysis Buffer(ALB): 0.2 M NaOH, 1% (m/v) SDS.
Plasmid DNA preparation reagents (Plasmid-combi-kit, Qiagen) :

- Buffer P1 : 50 mM Tris-HCl, 10 mM EDTA-$Na_2$ pH 8.0; 100 μg/mL RNase A.
- Buffer P2 : 200 mM NaOH, 1% (m/v) SDS.
- Buffer P3 : 3.0 M Potassium acetate, pH 5.5.
- Buffer QC : 50 mM MOPS, 1 M NaCl, pH 7.0, 15% (v/v) ethanol.
- Buffer QBT : 50 mM MOPS, 750 mM NaCl, pH 7.0, 15% (v/v) ethanol, 0.15% (v/v) Triton X100.
- Buffer QF : 50 mM Tris-HCl, 1,25 M NaCl, pH 8.5, 15% (v/v) ethanol.

x 5 DNA denaturating solution : 25 mM EDTA-$Na_2$ pH 8.0, 20% (m/v) glycerol, 0.5% (m/v) lauroyl sarcosine, 0.1% (m/v) Bromophenol blue. This solution leads to DNA denaturation and can be used as a migration control in agarose gel electrophoresis.

- SDS-PAGE loading buffer: 0.2 M Tricine, 0.2 M Tris-base, 0.55% (m/v) SDS, pH 8.0.
- Coomassie blue coloration stock solution : 0.2 g Coomassie blue G250 R (GE Healthcare), 60% (v/v) methanol, QSP 200 mL with $H_2O$.
- Coomassie Blue coloration finale solution: 50 % (v/v) of filtered stock solution, 10% (v/v) acetic acid, 30 % (v/v) methanol.
- PBS : 2.7 mM KCl, 10 mM, $Na_2HPO_4$, 1.8 mM $KH_2PO_4$, 140 mM NaCl, pH 7.4.
- Lysis BufferA: 20 mM HEPES, NaCl 150 mM, EDTA 1 mM pH 7.2. Buffer B : 100 mM Acetic acid, 500 mM NaCl pH 4.0.
- Elution buffer C : 50mM Tris-hydroxyamino methyl-HCl, 500 mM, NaCl, 30 mM GSH, pH 8.0.

**Preparation of *Staphylococcus aureus* total DNA**

From a 5 mL preculture of the corresponding strain of *S. aureus*, inoculate 2x 10 mL of 2x TY medium at 37˚C overnight. Spin 10 min at 5000 xg. Wash the pellet with water. Resuspend in 7 mL of lysis buffer [50mM Tris; 25% Sucrose; 2 mg/mL lysozyme; 0.05 mg/mL Lysostaphine; pH 7.5] and 100μL of 2 μg/mL of boiled RNase A. Incubate 45 min at 37˚C. Spin 10 min at 5000 xg. Resuspend the pellet in 3852μL of water, 300μL of EDTA,NA2 250 mM pH 8.0 and 300μL of SDS 10% (v/v). Agitate vigorously. Add 10 mL of phenol, equilibrated at pH 8.0. Agitate vigorously. Spin 5 min at 5000 x *g*. The upper aqueous phase undergoes a second extraction with 10mL of phenol/chloroform (1/1). Eventually repeat that step. Precipitate the DNA with 3 volumes of absolute ethanol. Save the precipitate and move it into a new tube. Wash with 70% ethanol (v/v) and spin 5min at 5000 x *g*. Repeat that step. Dry the DNA pellet. Dissolve in 300μL of TE buffer. Store at -20˚C.

**DNA amplification by Polymerase Chain Reaction (PCR)**

Assemble the reaction in ice, in that order : 35.5μL of MQ water, 10μL of PCRx5 buffer (which contains 7.5mM MgCl2), 1 μL of 0.2mM dNTP, 1μL of each corresponding 25μM primer solutions (forward and reverse), 1μL of the corresponding *S. aureus* total DNA (about 100μg) and 0.5μL of the DNA polymerase (Phusion High Fidelity DNA polymerase). PCR cycles for the amplification of the SEs genes are shown in the table 4 below.

Table 4

| Phase | Length | Temperature | N˚ of cycles |
|---|---|---|---|
| Initial denaturation | 40 s | 94˚C | 1 |
| Initial denaturation | 20 s | 94˚C | |
| Hybridization | 30 s | 52˚C | 35 |

(continued)

| Phase | Length | Temperature | N° of cycles |
|---|---|---|---|
| Elongation | 2 min | 72°C | |
| Final elongation | 3 min | 72°C | 1 |

Control of PCR product on agarose gel (1 % w/v)

The agarose is dissoluted in TBE buffer x1 (89mM Tris-borate, 2mM EDTA, pH 8.0) by heating. Ethidium Bromide is included in the gel matrix at 0.5μg/mL to enable fluorescent visualisation of the DNA fragment under UV light (254 nm to 300 nm). The migration occurs at 110V during about 1 h.

Electroelution of the PCR product in low melting point agarose gel 1,8% at 100V

Make a phenol/chloroform extraction, then one ether extraction. Add 3μL of 5M NaCl and precipitate the DNA with ice-cold absolute ethanol. Wash the pellet with 70% ethanol (v/v) and let dry. Resuspend the DNA in TE and store at -20°C.

**Ligation of the insert in the plasmid**

Restriction hydrolysis

Assemble in a Eppendorf tube : 15μL of the restriction enzyme buffer x10, 110μL of water, 20μL (10μg) of the plasmid or insert from PCR and 5μL of the corresponding enzyme restriction. Incubate 2h at 37°C. Add 6μL of 250mM EDTA pH8.0. Make a phenol extraction, keep the aqueous phase (upper phase). Make three ether extractions, keep the aqueous phase (lower phase). Add 3μL of 5M NaCl and 400μL of ice-cold absolute ethanol. Spin 5 min at 7 000 x *g* at 4°C, and discard the supernatant. Wash the DNA pellet with 70% ethanol (v/v), spin and discard the supernatant. Dry the DNA pellet. Continue to the following step : plasmid dephosphorylation.

Plasmid dephosphorylation

To the dried DNA pellet, add 134μL of MQ water, 15μL of the buffer x10 and 1μL of the CAIP enzyme. Incubate 1h at 37°C. Add the same volumes and re-incubate 1h at 37°C. Add 12μL of 250mM EDTA pH 8, vortex. Add 250μL of phenol. Vortex and incubate 10 min at 65°C. Vortex and re-incubate 10 min at 65°C. Discard the phenol. Make a phenol/chloroform extraction, then four ether extractions. Add 3μL of 5M NaCl and precipitate the DNA with ice-cold absolute ethanol. Wash the pellet with 70% ethanol (v/v) and let dry. Resuspend the DNA in 125μL of TE and store at -20°C.

Ligation of the plasmid and the DNA fragment

In a final volume of 15 μL, assemble ligase buffer; a molar ratio insert/vector of 3 to 5 and 0.2U of T4 DNA ligase. Incubate 16h at 14°C.

**Transformation of competent bacteria with the ligation product**

Preparation of competent cells

From 3mL of 2xTY preculture, incubate a 100mL culture of *E. coli* XL1 Blue at 37°C under 200rpm shaking in the same medium. When $OD_{600nm}$ reach 0.5 to 0.7, spin 10min at 2500 x *g*. Resuspend the pellet in 10mL of 50mM $CaCl_2$. Leave the bacteria 30min at 4°C, then spin 10min at 2500 x *g* at 4°C. Resuspend the pellet in 2.5mL of 50mM $CaCl_2$.

Transformation of bacteria by the thermal shock method

Add 4μL of ligation product to 150μL of competent *E. coli* XL1 Blue cells. Incubate 40min at 4°C, then a thermal shock of 2min at 42°C. After 2min of chilling at 4°C, spread out the preparation on TYA plate and incubate at 37°C overnight.

**Control of ligation**

Mini preparation of plasmid DNA from *E.coli*

Inoculate a 2x TYA medium (3 mL) with an isolated colony and grow overnight at 37°C. Pellet cells at 12 000 xg for 30 sec. and discard the supernatant. Add 100μL of TEG buffer [25mM Tris, 50mM glucose, 10mM EDTA, pH 8.0] and resuspend cells. Add 200μL of Alkaline Lysis Buffer [0.2N NaOH, 1% SDS], mix by inverting 5-6 times. Add 150μL of 3M Potassium Acetate (pH 5.5). Spin 10min at 12 000 x *g*. Transfer 400μL of supernatant to a new tube containing 1mL of 24:1 Chloroform:Isoamyl Alcohol ($CHCl_3$:IAA) and mix by inverting. Spin at 12 000 x *g* for 10 min. Transfer aqueous phase to 800μL of ice-cold absolute ethanol. Precipitate at -20°C for at least 30min. Spin at 7 000 x *g* for 10 min. Discard the supernatant and wash the pellet with 1mL 70% ethanol (v/v), then 1mL 95% ethanol (v/v). Discard ethanol and dry the pellet (air-dry). Dissolve in 40μL of TE containing 20μg/mL of RNase A (Ribonuclease A SERVA®). Store at - 20°C.

Control of ligation product by agarose gel electrophoresis

DNA minipreps are digested by the restriction enzyme corresponding to the insertion sites. The restriction product is then analyzed by agarose gel electrophoresis.

Maxi preparation of DNA by chromatography (Plasmid Combi kit, Qiagen)

Inoculate 100mL of 2x TYA medium with transformed bacteria and grow overnight at 37°C. Spin 10 min at 5000 x *g*. Resuspend the cells in 6mL of P1 buffer. Transfer the solutions into ultracentrifugation tubes. Add 6mL of P2 buffer in order to lyse the bacteria. Incubate 5 min and add 6mL of P3 buffer (SDS is precipited and the mixture is becoming viscous). Leave the medium 5min at room temperature then spin 40min at 25 000 x *g* at 4°C in angular rotor (Ti 60 - Beckman). Supernatant is then used for the chromatography.

Equilibrate the anions exchange column (DEAE-cellulose groups) with 10mL of QBT buffer. Apply the supernatant to the column and then wash with 2 x 10mL of QC buffer. Eluate the DNA with 7mL of QF buffer. Add 20mL of ethanol and spin 10min at 5000 xg at 4°C. Wash the pellet with 70% ethanol (v/v). Let the pellet dry and resuspend in 350μL of TE buffer. Add 350μL phenol, shake vigorously and spin 5min at 5000 x *g*. The upper phase, containing the DNA, undergoes an other phenol extraction. Followed four ether extractions, in order to get rid of phenol in the aqueous phase. Precipitate the DNA by adding 15μL of 5M NaCl and 1mL of ice-cold absolute ethanol. Wash the pellet with 70% (v/v) ethanol. Resuspend the dried pellet in a necessary TE volume to get a DNA concentration of 1μg/μL.

Control of the insertion direction of the fragment

The insertion direction of the fragment of interest in the vector is checked by sequencing, with the help of vector specific primers.

The surexpression of the SE is estimated by GST enzyme activity titration (see below).

Transformation of *E.coli* BL-21 with $CaCl_2$ treatment

When ligation product are checked, *E. coli* BL21 cells can be transformed in order to get a significant surexpression of SE. Make a 100mL 2xTY culture of *E. coli* BL21, grow overnight at 37°C. Inoculate an other 100mL 2x TY with the *E. coli* preculture. Let grow at 37°C until $OD_{600nm}$ reach 0.5 to 0.7. Transfer the culture into a 50mL tube. Spin at 3000rpm 10 min at 4°C, discard the supernatant and add 10mL 50mM $CaCl_2$ to the pellet. Spin 10min, 3000rpm and at 4°C and discard the supernatant. Repeat that step : add $CaCl_2$ and spin. Dissolve the pellet in 5mL 50mM $CaCl_2$. Transfer 150μL of competent BL21 bacteria into a new eppendorf tube. Add 1 to 3μL (2ng) of transformed plasmid DNA. Transform bacteria by thermal shock method. Incubate 40min at 4°C, then 2min at 42°C. After 2min chilling at 4°C, spread out the cells on a 2x TYA plate. Incubate 24h at 37°C.

### 1.1. *E. coli* production of Staphylococcal enterotoxins (apart from SED)

#### 1.1.1. Preparation of bacterial lysate

Two 2L flasks containing 400mL of 2xTYA medium are inoculated with 50 mL of a BL21 *E. coli* preculture transformed by the pGEX-SE recombined strains, and incubated at 37°C under 200rpm shaking. When the $OD_{600nm}$ reaches 0.4 to 0.6, fusion gene expression is induced with 0.2mM IPTG at 25°C overnight. Next day (18-22h), bacteria are centrifuged at 5000 x *g* during 10 min, at 4°C. The pellet is resuspended in 35mL of PBS-EDTA 1mM buffer. Bacteria cells are then lysed by French Press (French Pressure Cell Press, SLM AMINCO®) under a 600 bars pressure. Bacterial lysate is centrifuged during 30 min at 29000 x *g*, at 4°C in a Ti60 rotor (Beckman).

### 1.1.2. GST enzyme activity titration

GST enzyme activity leads to an estimation of the fusion protein concentration in the environment. GST catalyzes a transfer reaction of the GSH on the CDNB (Chloro-1,2,4 Dinitrobenzene) [Habig and al., J. Biol. Chem., 249 : 7130-7139, 1974] [20]. The product of the reaction absorbs at 340nm whereas the free CDNB has its maximum at 270 nm. The kinetic occurs in a spectrophotometric bowl.

The enzyme buffer contains:

| | |
|---|---|
| $H_2O$ (Milli Q) | 880μL |
| x 10 reaction buffer (1 M potassium phosphate, pH 6.5) | 100μL |
| CDNB (100mM in Ethanol) | 10μL |
| Reduced Glutathion (100mM in distilled water) | 10μL |
| Sample or dilution 1/10, 1/20 | 10μL |

The bowl is then placed into the spectrophotometer and the absorption evolution is measured at 340 nm every minutes during 5 minutes against a negative control, containing all the components apart from the 10μL sample, replaced by 10μL of distilled water (even without GST, there is a degradation of the substrate). This reaction is linear until $A_{340nm}$ = 0.8. The GST concentration must be adjusted in order to get a linear reaction during 5 minutes.

In those conditions, the GST concentration is proportional to the speed reaction (ΔA/min) in accordance to the following relation :

[GST] = $v_I$ x k x 1/d for 10μL of sample
Where : [GST] in μg/mL
$v_I = (\Delta D_{340} - \Delta OD_{340}\text{blanc})/\text{min}$
1/d = dilution inverse
k = constant = 545 $\mu g.min.mL^{-1}$
k is determined from a commercial GST solution (Sigma) with a known concentration.

### 1.1.3. Purification of the fusion protein to GST, by GSH affinity chromatography

4 mL of gel Gluthathione Sepharose 4B GSH™ (GE Healthcare) (capacity of 20mg of GST) is packed in a PD10 column (GE Healthcare). The column is equilibrated with 20 mL of buffer : PBS + EDTA 1mM pH 7.5 per gravity. 16mg equivalent GST (determined by the GST titration above) are applied in batch and left 30 min at +4°C under smooth shaken. Then the gel is 10 min centrifuged at 200 x g and at +4°C, and washed in PD10™ with 20mL of PBS-EDTA.

6x2mL fractions are eluted with the buffer: Tris 50mM, GSH 30mM, NaCl 500mM pH8.0. Fractions with a significant $D0_{280nm}$ (>0.2) are assembled, 10μL (20u) of PreScission protease™ (GE Healthcare) are added to cleave the fusion protein and incubation one night at +4°C.

Note 1 : in order to avoid contaminations, a different column for each enterotoxin should be used. Gels should also need to be washed with 10mL of the solution: $NaH_2PO_4$ 30mM, NaCl 2M pH6.5 between two runs.

Note 2 : For SEG+8 and SEH+5, the sample may be applied a second time on the column GSH affinity, after the protease action and desalting against PBS, in order to get a maximum of GST ; because those toxins are hardly separable of the GST, in the conditions of exchange ions chromatography presented below.

### 1.1.4. SEs purification by exchange cations chromatography

After the PreScission protease™ action, the sample is diluted at 40mL with 10mM of start buffer containing 40mM (MES or Hac) adjusted with NaOH (see table 5 below) and then dialyzed against that buffer during 2h at + 4°C , with a Spectra Porl™ membrane (Spectrum Laboratories) with a nominal molecular weight limit from 6 to 8000 Da. Then the sample is completed with 30mM MES pH5.7 [from MES 500mM pH5.7] and with 3mM DTT [from DTT 1 M]. After 5 min centrifugation at 5000 x *g* at +4°C, different samples are applied on a 6mL ReSource S™ or MonoS™ column (GE Healthcare), equilibrated in start buffer. After column washing, elution is made by a linear gradient of NaCl, supplied by the end buffer, *i.e.* start buffer + 1 M NaCl. The NaCl gradient slope goes from 0 to 300 mM maximum in 63 mL (flow rate 4mL/min).

Note: between two runs and for each SE, the gel is washed with 2mL de NaOH 0.5M.

Table 5

| | SEA+5 | SEB+5 | SEC1+8 | SEE+5 | SEG+8 | SEH+5 | SEI+5 |
|---|---|---|---|---|---|---|---|
| Start buffer | MES pH5.7 | MES pH6.1 | MES pH5.7 | MES pH5.7 | Hac pH4.8 | Hac pH4.8 | MES pH6.1 |
| Elution (mM NaCl) | 120 | 90 | 90 | 120 | 120 | 80 | 170 |
| MES : 2-(N-Morpholino)ethane - sulfonic acid<br>Hac : acetic acid<br>DTT: 1,4 Dithiothreitol | | | | | | | |

Note: Exchange ions separation shows different forms (enterotoxins found in several elution peaks), the prevalent form is kept.

Toxins are stored at -80°C in aliquots of 2mL maximum.

The purity is estimated in SDS-PAGE on a Phast System™ apparatus following the instructions of GE Healthcare. Briefly, 500ng in 1μL of SE (50μL denatured in SDS 2.5% and β-mercaptoethanol) are applied on a PhastGel™ Gradient 10-15 and after electrophoretic migration, the gel is colored with blue coomassie (PhastGel Blue R).

**1.2. SED production**

Some troubles appear upon the expression of the *sed* gene, that changed the protocole to an antigen purification from a referred strain of *Staphylococcus aureus.* SED is purified from *S. aureus* (87109 (FRI1157M) of the Institut Pasteur, Mrs Dr Debuyser collection) :

- BHI culture

From a preculture (6H), twelve 2L flasks containing 200 mL of BHI are inoculated with 400μL and incubated at 37°C under 250rpm overnight (15-18H). Bacteria are discarded by centrifugation at 7 000 x *g* 10min at +4°C.

- Supernatant precipitation with ammonium sulfate

The supernatant is precipitated with solid ammonium sulfate (Sigma) : 667g to 1 L (90% of saturation) overnight (20-24H) at +4°C

- Capture by cations exchange chromatography at pH5.7 SP Sepharose FF gel.

After centrifugation, the pellet is dissolved with 100mL of water and dialysed in a SpectraPor1™ membrane (Spectrum Laboratories) first 2H at +4°C against 4L of water then against 2L of MES 10mM pH5.7. The sample is then completed to 30mM MES pH5.7 with MES 500mM pH5.7 solution and to 3mM DTT with a 1 M solution and centrifugated at 5000 x *g* 5 min at +4°C. The sample is applied on a 50mL SP Sepharose FF™ in a XK26 column (GE Healthcare) equilibrated with MES 40mM + DTT 1mM, pH5.7 buffer. After column washing, elution is made by direct application of a MES 40mM + DTT 1mM + 300mM NaCl, pH5.7 buffer.

- Cations exchange chromatography at pH 5.7 Ressource S™ or MonoS™ column

After the first exchange chromatography, the sample is dialysed again in the same conditions as above. Then, it is applied on a 8 mL MonoS™ Tricorn column (GE Healthcare) equilibrated in the same start buffer as above. After washing, elution is made by a linear gradient of NaCl in the same end buffer. The NaCl gradient slope goes from 0 to 160mM in 60 mL.

- Hydrophobic interactions chromatography at pH 7.0 Source-Phenyl gel.

This step is delicate, SED highly hydrophobic is barely holded by the column. Now the pH of the sample is adjusted to 7,0 with a $K_2HPO_4$ 1.5M solution. Finally, the phosphate concentration of the sample is 350mM ($KH_2PO_4$ 1,5M pH7.0) with 1.7M ammonium sulfate (($(NH_4)2SO_4$ 3M pH7,0). The sample is applied on a 1mL ReSource S™ or MonoS™ PHE column (GE Healthcare) equilibrated in $KH_2PO_4$ 50mM + 1.7M $(NH_4)2SO_4$ + DTT 1mM, pH7.0 buffer. After a short column washing with 3 mL, elution is made by a linear decreasing gradient of ammonium sulfate, supplied by the end buffer: $KH_2PO_4$ 50mM + DTT 1 mM pH7.0. The gradient slope goes from 1700mM to 0 in 10mL.

- Storage

Several runs of SED purification are put together (~4mg) and after dialysis, SED is concentrated on a MonoS column as above. The toxin is stored at -80°C after checking the purity on SDS PAGE as above.

SED is recognized by the Oxoid SET RPLA™ kit (following the Oxoid instructions).

Enterotoxins A, B, C, D, E, G, H, and I are kept inside a box placed in a -80°C freezer, located in a secured room. Amounts of enterotoxins stored and removed are recorded in a saved file and on a paper signed by the project responsible, all kept locked. A statement to the AFSSAPS for the B enterotoxin of *Staphylococcus aureus* is made. Every wild and recombinant microorganisms (even if they should be induced to get a toxin expression) are strictly controlled and used under a microbiological security post. Microorganisms used and toxins amounts which have to be used by the experience are denatured and sterilized, then confined on SharpSafe boxes before being evacuated to the Institute DASRI. Toxins amounts used and rejected are always less than 1 mg.

**EXAMPLE 2: ANTIBODIES ANTI-8SEs ORIGINS**

**2.1. Procurement and affinity purification of the antibodies**

**2.1.1. Generation of toxoids**

The 8 SEs (1 to 2 mg/mL) were turned into a toxoid by a 60mM or 330mM formalin treatment during 48H at 37°C in buffer : 50mM $NaH_2PO_4$ + 150 mM NaCl pH 7.5 [protocol inspired from the thesis of M. PIEMONT or inspirited from Gampfer and al. [Vaccine, 20 : 3675-3684, 2002] [21]. The excess of formaldehyde was discarded by desalting on PD 10™ column (following the instructions of GE Healthcare) against PBS and the toxoids were stored at -20°C. During a second immunization drive, the 8 SEs were treated with 1% formol [inspirited from Gampfer, 2002, fore-mentioned] [21] in order to get bigger immune-serum stocks and antibodies for SEH and SEI with a better affinity. For each SE, two New-Zealand rabbits were injected in subcutaneous with 50 μg/500μL (apart from SED: 25μg then 100 μg) with the complete Freund's adjuvant to start and then the incomplete formulation was used.

**2.1.2. Toxoids preparations for injection**

Animals were immunized with 10 to 200μg per rabbit (50μg in a first generation of antibodies). Separately, the antigens were emulsified in a 1 :1 mixture of complete or incomplete Freund's adjuvant (CFA or IFA) and PBS with a 2mL seringue and a 1.1mm needle. Rabbits were shaved off and the preparations were injected with 0.7mm needles in three subcutaneous sites with 500μL. Three booster immunizations were given at about 3-weeks intervals (for a second generation of antibodies IFA was used after the first injection).

**2.1.3. Collect of sera**

Before the rabbits were bled, a sample of veinous blood from the ear was taken, about 500μL. The sera was used in an Ouchterlony precipitation test. Briefly, in a plate with 0.6% agar in PBS, 40μL of sera or 40μL of SE at 150μg/mL were put into 2 wells separeted from 1cm. After overnight incubation at +4°C, a line of precipitation was considered like a hyper- immunisation. After that, rabbits were bled of 35mL maximum under anesthesia : ketamin (20-40mg/kg) and xylazin (3-5mg/kg) [Borkowski and al., Clin. Tech. Small Anim. Pract., 14.(1): 44-49, 1999] [22] at the ear artery with a vaccum pump device. If necessary, the rabbits were killed with Dolethal™. After overnight blood clot retraction at +4°C, sera were centrifuged 5000 x *g* 5 min and filtered (0.45μm), and stored at -80°C by 10 mL fractions.

Antibodies were purified by affinity on an activated "HiTrap NHS" support, where a native SE had been immobilized by injection-chromatography (Elution buffer: CitrateNa 0.1 M + NaCl 0.2 M, pH 2.5, then neutralization at pH 7.5 with $Na_2CO_3$ 1M).

**2.1.4. Affinity purification of antibodies anti-SE**

The Aktä Purifier™ device (GE Healthcare) and Hi-TrapNHS gel 1 mL (GE Healthcare) were used wherein antigen corresponding to the antibody to purify was immobilized (see GE Healthcare protocol, "Ligand coupling procedure for HiTrap NHS-activated HP") (see table 6 below).

Table 6

| Ligand | Immobilized amount (mg) | Date |
|---|---|---|
| **SEA** | 6,7 | 08/02/2008 |
| **SEB** | 5,4 | 26/10/2007 |
| **SEC1** | 3 | 02/04/2008 |
| **SED** | 4 | 15/04/2010 |
| **SEE** | 6,8 | 14/03/2008 |
| **SEG** | 5,8 | 23/04/2008 |
| **SEH** | 8,5 | 28/03/2008 |
| **SEI** | 5,3 | 28/01/2008 |

The column was equilibrated with 10mL of PBS with a flow rate of 1mL/min. The serum provided by the hyper-immunized rabbit, was centrifuged 5min at 5000 x g, and 5mL of serum were applied at the flow rate of 0.5mL/min. The column was washed with PBS. The antibodies were evaluated with citrate buffer [0.2M NaCl, 0.1 M citric acid, pH 2.5], at a flow rate of 1 mL/min. Fractions were then neutralized to pH 8 with 1M $Na_2CO_3$ pH 9.5. The $OD_{280nm}$ was measured. Fractions with a significant $OD_{280nm}$ (>0.2) were assembled. Antibodies were then dialyzed against 2L of 30mM $NaH_2PO_4$, 100mM NaCl, pH 7.3 at 4˚C overnight.

The antibodies purified by affinity were frozen and stored at -80˚C at a minimal concentration of 1mg/mL.

## 2.2. Specificity of affinity purified antibodies and achievements of mono SE-specific antibodies

### 2.2.1. DOT-BLOT

A piece of nitrocellulose (Protan $^{BA83TM}$ 0.22μm Schleicher et Schuell) of about 7x3cm was cut. 1μL, i.e. 10ng, of each SE (stock solutions at 10μg/mL) and a blank (BSA) were put and left to dry several minutes. The membrane was drenched (~20mL) in the blocking buffer [PBS, 5% skimmed milk(w/v)], left at 4˚C overnight, then washed with PBS + 0.05% Tween20 5min by immersion. The immunoabsorbed Ab antiSE, which the specificity is to check, was added at 1μg/mL in 20mL of PBS + 0.05% Tw20 + 1% skimmed milk and left 2h under smooth shaken, then washed 3 times. Goat Ab-POD anti-rabbit IgG purified by affinity (Sigma A6154), was added at a 1/800 dilution in the same buffer that primary Ab and incubated in the same conditions, then washed 3 times. The reaction was revealed according to the Opti 4 CN™ kit instructions (BioRad) : 9mL of water, 1 mL of the kit buffer and 0.2mL of the kit 4 CN, for 30 min max., depending on the blank. The membrane was washed with water several times and finally scanned.

### 2.2.2. Immunoabsorption of antibodies already affinity purified

The HiTrap™ gel (1mL) with an antigen (namely an immunization-unrelated staphylococcal enterotoxin) immobilized responsible for the cross reaction was chosen. The gel was equilibrated with 5 to 10 mL of PBS on AktaPurifier™ device (GE Healthcare) at flow rate 1mL/min. An amount of Ab was applied from one run of affinity chromatography (1mL of gel), at a flow rate 0.5mL/min, and Ab which passed through the immunoabsorption column were collected. Ab eluated by the citrate buffer were removed, about 10mL at flow rate 1mL/min. The column was washed with 5 to 10 mL of PBS. The column was stored in PBS + NaN3 0.1% at 4˚C. After control of their specificity (by DOT-BLOT) against the immunogen and/or cross reactivity with an antigen (namely an immunization-unrelated staphylococcal enterotoxin), immunoabsorbed Ab were stored at -80˚C at a concentration of 1 to 3 mg/mL.

If a cross reaction was still detected, the immunoabsorption step was again implemented as above described against the same antigen or another antigen as above defined (which is still not the immunogen) until obtaining the monospecificity of immunoabsorbed Ab. The order of successive columns could be determined by one skilled in the art according to the extent of each cross reaction detected, namely from the stronger cross reactivity to the weaker cross-reactivity.

Immunoabsorptions in the exact order of the successive columns made on the affinity purified antibodies are shown in the table 7 below.

Note 1 : For IgG, $DO_{280}$ = 1,0 means a concentration of 750μg/mL.

Note 2 : Concentration of Ab is possible by ultrafiltration, using Amicon® 4mL or 15mL, cut off 10kDa units (according to Millipore™ instructions).

Table 7

| Ab>SEA | Ab>SEB | Ab>SEC1 | Ab>SED | Ab>SEE | Ab>SEG | Ab>SEH | Ab>SEI |
|---|---|---|---|---|---|---|---|
| E E I B D | C1 C1 G | B B G | A A E | A AI C1 | A BI C1 | B D | E C1 G B A |

Those immunoabsorbed antibodies were frozen in the buffer: $NaH_2PO_4$ 30 mM + NaCl 100 mM, pH 7.3.

Such antibodies may be used downstream for several kinds of applications (beads-based multiplex technologies, chip-based protein microarray, immunocapture coupled to MALDI-TOF, etc....).

**EXAMPLE 3: COUPLING OF ANTI-SEs ANTIBODIES TO LUMINEX MICROSPHERES AND BIOTIN**

**3.1. Beads sensitization with the immunoabsorbed antibodies anti-SEs**

Each immunoabsorbed antibody anti-SEs was combined with a microsphere of an unique color-code according to the table 8 below.

**3.1.1 Protocol (inspired from Amine coupling kit of BioRad ref 171- 406001)**

For each SE, 19.5 x $10^6$ carboxylated beads were activated at pH6.0 (Buffer : MES 0.1 M + NaCl 0.15 M pH 6.0) by 50mg/mL of EDC with sNHS during 30 minutes under shaking at room temperature. After wash by centrifugation 10 min at 10 000 x g, 360 μg of immuno-absorbed antibodies (without $NaN_3$ and Amine) were added, i.e. 18.5 μg for 1 million of beads, and were incubated in PBS during 2H under shaking, at room temperature and in darkness. After wash, free sites were blocked with ethanolamine (Buffer : ethanolamine 50 mM + NaCl 150mM, pH 8.5) during 30 minutes. Then the blocking buffer was replaced by the storing one : PBS + $NaN_3$ 0.05% (p/v). The storage was made at +4˚C and in darkness during several months after assembling in one fraction the 8 types of beads at 1x10 beads of each color region/mL.

Finally, the fluorescence of the coupled beads was measured by the BioPlex- 100™ with biotin-coupled goat antigen (Sigma), rabbit anti-IgG and SEPE. The final suspension concentration was determined by an 1 $mm^3$ hemacytometer.

**3.1.2. Acquired results for the validation of the beads set**

Table 8

| Ab anti | Beads COOH color-region | Coupling date | [beads]/m L in 1.8mL | Coupling yield in % for the beads | SEPE control in UFI |
|---|---|---|---|---|---|
| **SEA(EEIBD)** | 8 | 09/02/2010 | $7.77.10^6$ | 72 | 22 798 |
| **SEB (C1 C1 G)** | 12 | 10/02/2010 | $9.06.10^6$ | 84 | 24066 |
| **SEC1 (B B G)** | 16 | 10/02/2010 | $8.95.10^6$ | 83 | 21665 |
| **SED (A A E)** | 20 | 23/04/2010 | $7.53.10^6$ | 70 | 23873 |
| **SEE (A A I C1)** | 30 | 19/02/2010 | $6.81.10^6$ | 63 | 14436 |
| **SEG (ABIC1)** | 34 | 12/02/2010 | $6.48.10^6$ | 60 | 8489 |
| **SEH (B D)** | 38 | 12/02/2010 | $8.78.10^6$ | 81 | 7050 |
| **SEI (E C1 G B A)** | 42 | 19/02/2010 | $7.74.10^6$ | 71 | 7949 |

According to the supplier BioRad, a bead was sufficiently coupled if UFI SEPE > 2000 and UFI blank < 100. This was the case for the 8 types of beads.

### 3.2. Biotin coupling of antibodies

Like the secondary antibodies, the simply affinity purified antibodies (8AbBt(X)) individually coupled to biotin were used. The test specificity was made by the primary antibody immobilized on the beads, since it ensured the antigens selection and all the test specificity.

The 8 immuno-absorbed Ab individually biotin-coupled (AbBt(-)) were also used for the need of the test specificity (see below). That specificity was compared to the one of AbBt(x) ; the specificity deviation was not significant, but the sensibility seemed to be lightly strengthened by the use of affinity purified antibodies, for a low production cost.

Protocol of biotin coupling:

The protocol was unchanged whatever the Ab chosen (without $NaN_3$ and Amine). 200μg of Ab at 1mg/mL in PBS were mixed to 10μL of sNHS-Lc-Bt (Molecular Probes) at 2.67 mM during 2H at room-temperature, i.e. a initial molar ratio [Bt] / [Ac] = 20. Free biotin was blocked by 10μL of ethanolamine 100mM, pH 7.5 during 30min at room-temperature, then 5.5μL of $NaN_3$ were added at 2%, i.e. a final concentration of 0.05% (p/v). The AbBt(-) and the AbBt (x) on either side were assembled in 2 fractions (x) and (-) only after the specificity test (see below). Each fraction was aliquoted and stored at -20°C at 100μg/mL.

### EXAMPLE 4: GENERAL INSTRUMENTAL PROTOCOL : LUMINEX xMAP TECHNOLOGY

As a preamble, a reminder of the protocol of BioPlex100™ utilization :

- A plan of the 96-wells plate for a maximum of 31 samples was drawn, made in duplicate (with 1 blank for each type of cheese matrix), 12 standards (from 16384 to 8 pg/mL, 2 in 2 dilution), 3 identical standard blanks and 2 controls at 4000 and 400pg/mL of the 8 SEs (those two points could be replaced by external control of the laboratory). Every points were in duplicate.
- Antigen aliquot was defrosted.
- From the stock suspension of beads coupled to the 8 immunoabsorbed Ab anti-SEs at 1. $10^6$ beads/region/mL, a final solution at $10^5$ was prepared and 50 μL were splitted out in a 96-wells multiscreen plate (Millipore), i.e. 5000 beads/region/well.
- Washing by aspiration with the manifold pump: 2 to 3 inches Hg of depression then 100μL of TBS (Tris-HCl 40 mM + NaCl 140 mM, pH 7.5) were added and re-aspirated.
- 50μL/well of antigen (sample, standard, blank and control) were transferred according to the plate plan and incubated under high shaking (600) during 1 h30, at room-temperature and in darkness.
- Then washed 3 times by aspiration.
- 50μL/well of the 8Ab-antiSEs-Bt(x) solution (non immuno-absorbed) were added at 0.25μg/mL in TBS and incubated 1h, at room-temperature and in darkness.
- Then washed 3 times by aspiration.
- 50μL/well of 0.5μg/mL SEPE solution (stock solution at 1mg/mL, Molecular Probes réf : S866) were added and incubated 15 min, at room-temperature and in darkness.
- Then washed 3 times by inspiration.
- Each well was resuspended in 125μL of TBS + Tween 20 at 0.05% and stirred 1 min.
- The plate was read at the BioPlex100™ according to the BioRad instructions (calibration and start up).
- Results were analyzed for each SE : the assays concentration was determined after subtraction of the cheese matrix blank, with the aid of the inverse function f-1 which is :

$$X = \left( \left( \frac{(a-d)}{(y-d)} \right)^{g^{-1}} - 1 \right)^{b^{-1}} \quad x \ c$$

x = concentration
y = response (FI)
a = estimated response at infinite concentration
b = slope of tangent at midpoint
c = midrange concentration or midpoint

d = estimated response at zero concentration
g = asymmetry factor

Note : Beads concentration (5000/SE/well) is chosen for a plate reading of 30 to 45 min. The sensibilization with 18.5μg of Ab/million of beads produces a satisfactory maximal signal (>2000 UFI), apart from SEH and SEI which have a lower signal. 8 AbBt(x) and SEPE concentration produce the lowest limit of quantification (LOQ).

**EXAMPLE 5 : TITRATION TEST VALIDATION**

Results exposed there concern the xMAP technology application for the 8SEs titration besides of cheese matrix.

**5.1. Specificity**

The aim was to estimate the degree of cross-reaction between the primary immunoabsorbed Ab coupled to beads on one hand, and the secondary biotin-coupled Ab in the other hand, towards the 8 SEs.

**5.1.1 Modification of the protocol exposed in EXAMPLE 4.**

In that aim, those following ELISA sandwiches were made up :

- A : beads 8Ab-antiSEs (immuno-absorbed) + 1 SE / well + 8AbBt(x) + SEPE, with the Ag at about 19.6 ng/mL, a concentration equal to the S1 point (16.4 ng/mL) of the standard range (table 9).
- B : beads 8Ab-antiSEs (immuno-absorbed) + 8 SE / well + 1AbBt(x) + SEPE (table10).

For results analyzing, the net value of fluorescence (Fl-blk) was noticed every time. The specificity, expressed in %,was given by the ratio of the detected signal (Fl-blk) by the specific signal for each SE.

- C : beads 8Ab-antiSEs (immuno-absorbed) + 1 SE / well + 8AbBt (immuno-absorbed) + SEPE, with the Ag at about 19.6 ng/mL , a concentration equal to the S1 point (16.4 ng/mL) of the standard range (table 11).

**5.1.2. Specificity - Results - Conclusions**

Table 9: ELISA specificity: A

| % | SEA | SEB | SEC | SED | SEE | SEG | SEH | SEI |
|---|---|---|---|---|---|---|---|---|
| **antiSEA (E E I B D)** | 100 | 0 | 0 | 0 | 0 | 0 | 0 | 0 |
| **antiSEB (C1 C1 G)** | 1 | 100 | 0 | 0 | 0 | 0 | 0 | 0 |
| **antiSEC1 (B B G)** | 1 | 0 | 100 | 0 | 0 | 0 | 0 | 0 |
| **antiSED (A A E)** | 0 | 0 | 0 | 100 | 0 | 0 | 0 | 0 |
| **antiSEE(A A I C1)** | 0 | 0 | 0 | 0 | 100 | 0 | 0 | 0 |
| **antiSEG (A B I C1)** | 1 | 0 | 0 | 0 | 0 | 100 | 0 | 0 |
| **antiSEH (B D)** | 2 | 0 | 1 | 1 | 0 | 0 | 100 | 0 |
| **antiSEI (E C1 G B A)** | 0 | 0 | 0 | 0 | 0 | 0 | 0 | 100 |

Conclusion : the 8 immunoabsorbed Ab anti-SEs did not produce significant cross-reaction between the 8 SEs. An immunoabsorbed Ab immobilized to a bead, only fixed its own Ag.

Table 10 : ELISA specificity: B

| % | SEA | SEB | SEC | SED | SEE | SEG | SEH | SEI |
|---|---|---|---|---|---|---|---|---|
| **antiSEA Bt(X)** | 100 | 2 | 5 | 1 | 62 | 3 | 2 | 7 |
| **AntiSEB** | 2 | 100 | 70 | 0 | 1 | 1 | 0 | 3 |

(continued)

| % | SEA | SEB | SEC | SED | SEE | SEG | SEH | SEI |
|---|---|---|---|---|---|---|---|---|
| **antiSEC1** | 3 | 66 | 100 | 0 | 4 | 12 | 0 | 3 |
| **antiSED** | 2 | 0 | 0 | 100 | 1 | 0 | 1 | 0 |
| **antiSEE** | 64 | 0 | 1 | 3 | 100 | 1 | 1 | 42 |
| **antiSEG** | 32 | 26 | 47 | 0 | 34 | 100 | 5 | 29 |
| **antiSEH** | 0 | 2 | 0 | 0 | 0 | 0 | 100 | -1 |
| **antiSEI** | 1 | 0 | 0 | -1 | 24 | 0 | -5 | 100 |

Conclusion : B ELISA revealed cross-reactions with some SEs for the 8 simply affinity purified Ab anti-SEs. However, as the bead-immobilized immunoabsorbed Ab were strictly specific, the simply affinity purified Ab as secondary Ab could be used. B ELISA also suggested a different immunoabsorption protocol from the one made up with the DOT BLOT results.

Table 11: ELISA specificity: C

| % | SEA | SEB | SEC | SED | SEE | SEG | SEH | SEI |
|---|---|---|---|---|---|---|---|---|
| **antiSEA (E E I B D)** | 100 | 0 | 0 | 0 | 1 | 0 | 0 | 0 |
| **antiSEB (C1 C1 G)** | 0 | 100 | 0 | 0 | 0 | 0 | 0 | 0 |
| **antiSEC1 (B B G)** | 1 | 0 | 100 | 0 | 0 | 0 | 0 | 0 |
| **antiSED (A A E)** | 0 | 0 | 0 | 100 | 0 | 0 | 0 | 0 |
| **antiSEE(A A I C1)** | 0 | 0 | 0 | 0 | 100 | 0 | 0 | 0 |
| **antiSEG (A B I C1)** | 1 | 0 | 0 | 0 | 0 | 100 | 0 | 0 |
| **antiSEH (B D)** | 1 | 0 | 0 | 0 | 0 | 0 | 100 | 0 |
| **antiSEI (E C1 G B A)** | 0 | 0 | 0 | 0 | 0 | 0 | 0 | 100 |

Conclusion : identical to the table A conclusion.
Note : That type of specificity test did not announce any specific cross-reaction in cheese matrix, like with protein A. Such interactions with protein A could easily be circumvent by the addition of 10 μg/mL aspecific rabbit IgG.

**5.2. Sensibility and detection limits**

Aim: from the standard curve of the antigens titration, deduce the LOD (limit of detection) and the LOQ (limit of quantification).

**5.2.1. Standard curve**

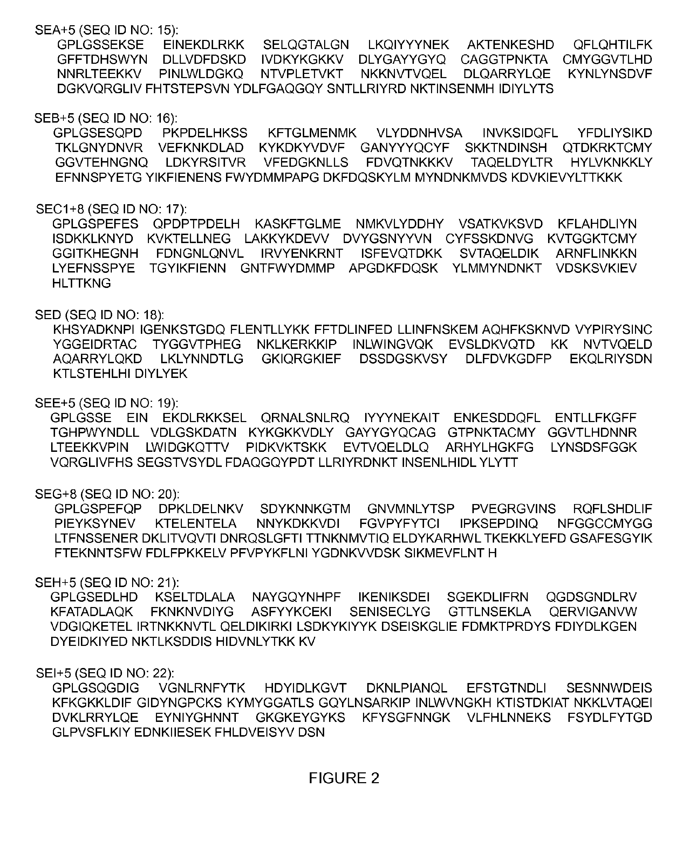

The reference concentrations (Cref) of the 8 SEs were obtained from spectrophotometry at $DO_{280nm}$ according to the Beer-Lambert law. The extinction coefficients (ε) used were those given by the server software http://expasy.org as Protparam (see table 12 below), according to the corresponding primary sequences (figure 2) :

Table 12

| SE | SEA | SEB | SEC1 | SED | SEE | SEG | SEH | SEI |
|---|---|---|---|---|---|---|---|---|
| ε **en $L.g^{-1}.cm^{-1}$** | 0,725 | 0,779 | 0,915 | 1,021 | 0,736 | 0,912 | 0,964 | 0,727 |
| 1mL of a stock solution of the 8SEs at 6.55μg/mL has been prepared in TBS and frozen at -40°C. | | | | | | | | |

In TBS+Tween20+IgAasp [aspecific IgG, i.e. prepared from a nonimmunized rabbit serum and affinity purified on Sepharose Fast Flow Protein G™ (GE healthcare)] Tris 40mM + NaCl 140 mM pH 7.5 + Tween 20 at 0.05% + aspecific

rabbit IgG at 10μg/mL, purified on G protein (stock solution at 5200μg/mL).

The stock solution of SEs was diluted at 1/100 with the same buffer, then a cascade dilution, 2 in 2 between 65.6 and 0.008 ng/mL. The standard range used was from 16.4 to 0.008 ng/mL, i.e. 12 points of measure.

A non linear Logistic-5PL regression was made with StatLIA™ Immunoassays software of Brendan Scientific, included in BioPlex Manager 4.0.

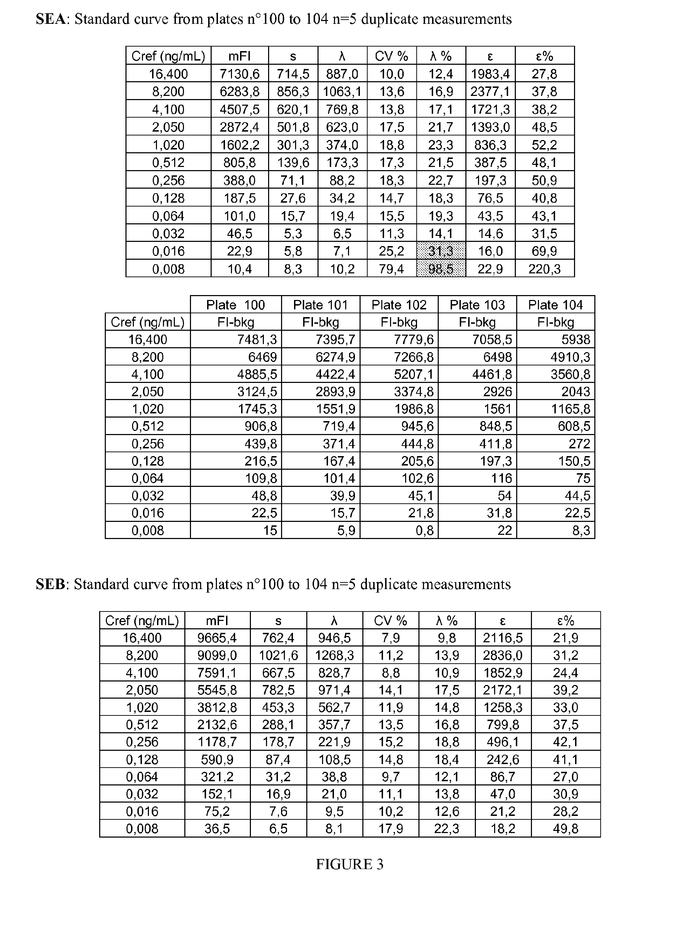

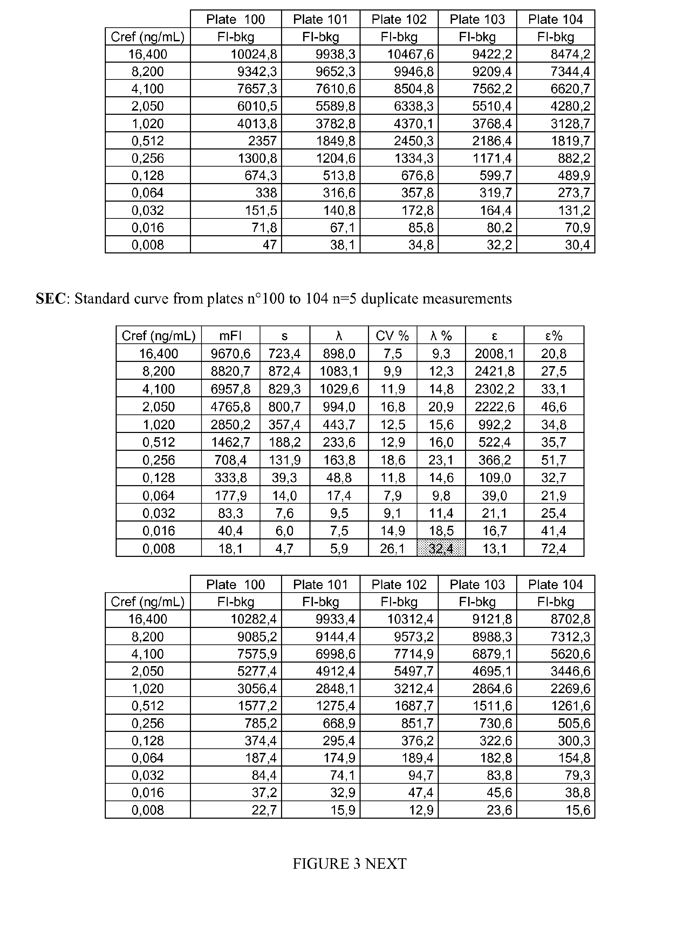

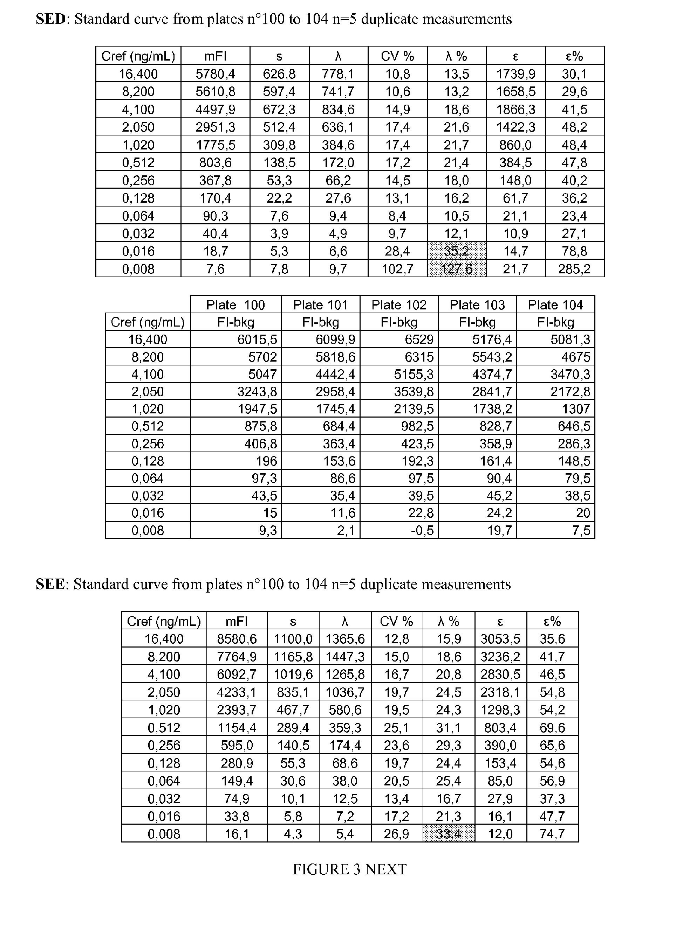

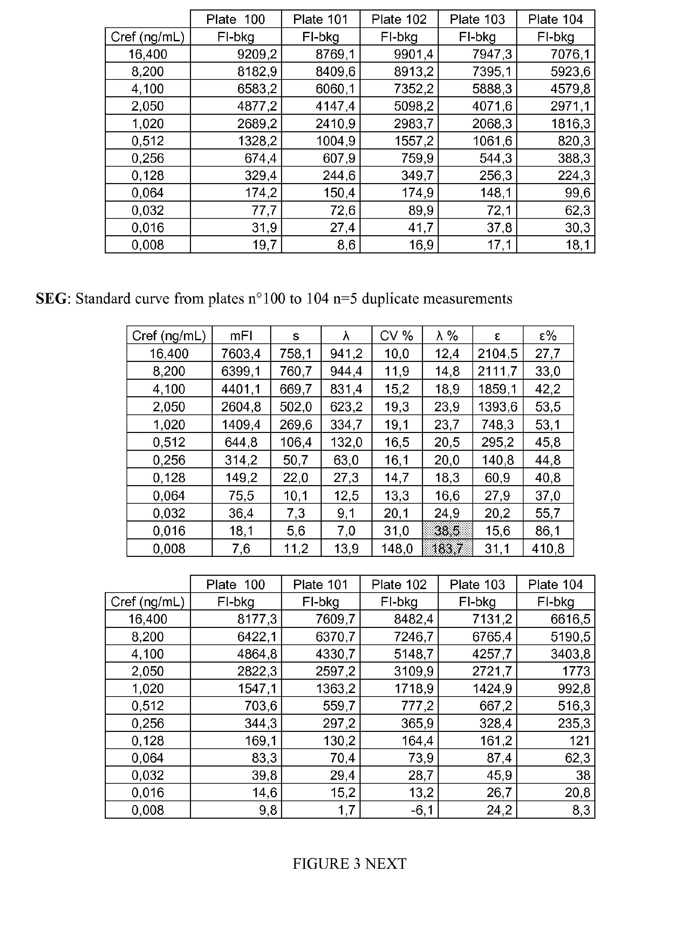

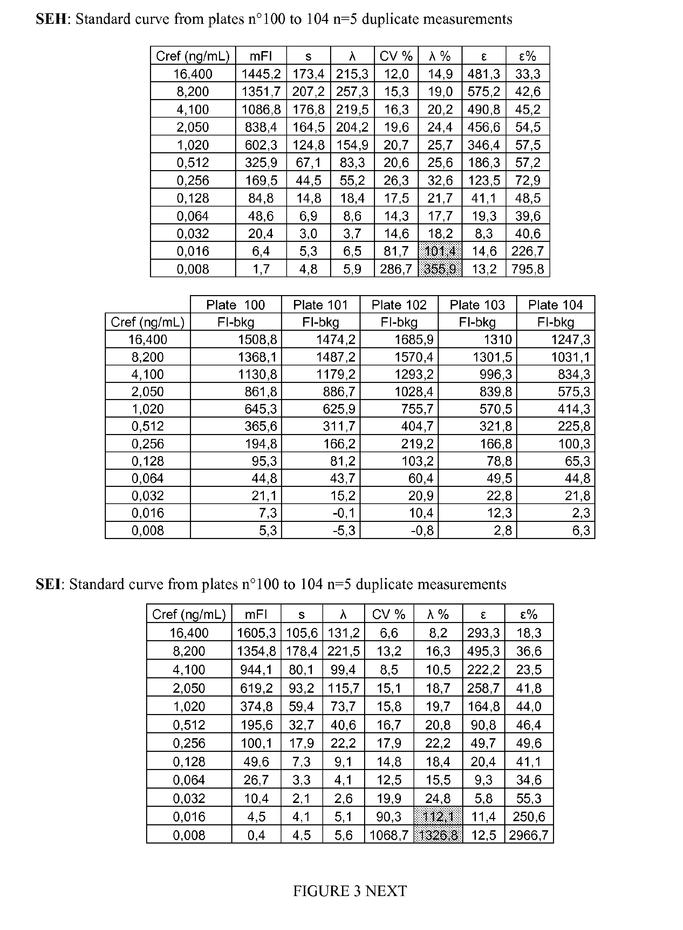

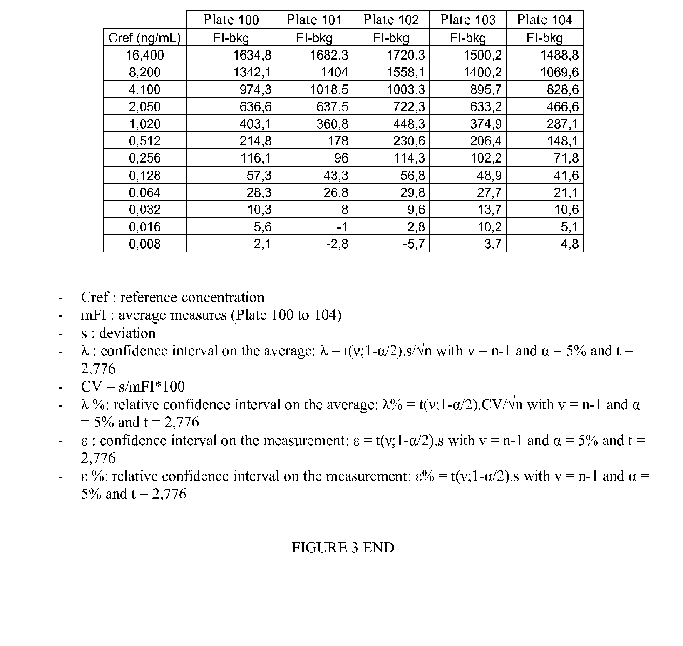

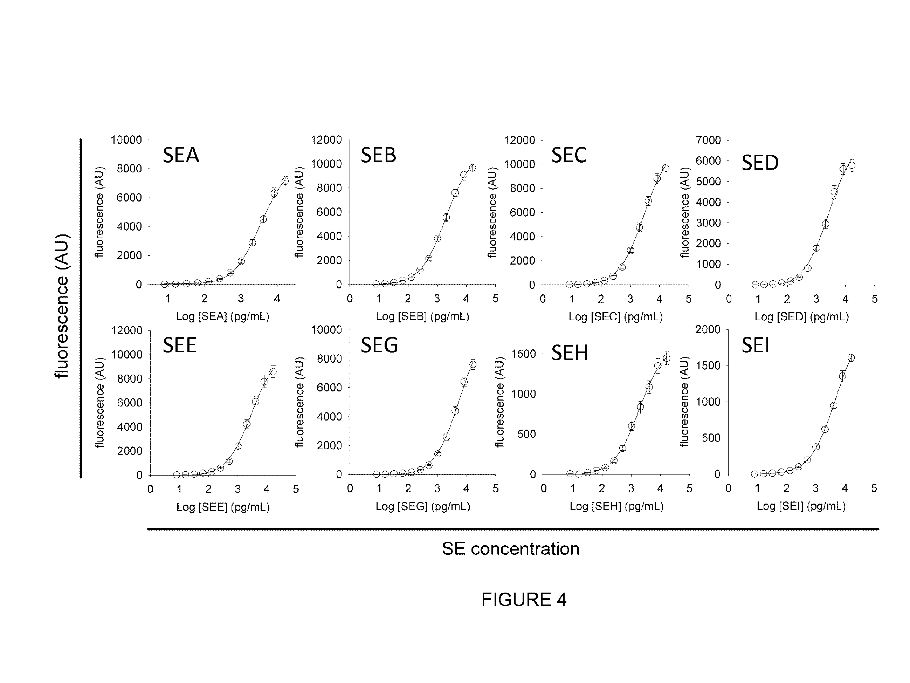

Results: Standards curves : Observed concentrations (Cobs) (Plates 100 to 104, i.e. n=5) see figures 3 and 4 : "averaged standard curves and series of assays 100 to 104.

In theory, after several types of regression tested (linear, exponential, logistic,...), the type kept was the one that reflected the best the measured magnitude (y) depending of the explanatory variable (x). If the 12 points of the standard range of the test plates (100 to 104) were linked by two types of regression: one linear and the other non linear Biologistic-5PL; the look of the non linear Biologistic 5-PL regression explained better the y=f(x) evolution than a straight line.

### 5.2.2. Calculation of LOD (limit of detection), lower LOQ (limit of quantification) and AWR (assay working range).

The aberrant points among the 6 blank wells were removed with the Grubbs test.

LOD and lower LOQ were determined according to Soejima and al. [Int. J. Food Microbiol., 93(2) : 185-194, 2004] [23] (see table 13).

If d was the estimated response at zero concentration and S the blanks standard deviation (n=6), then LOD = f-1 (d+3,3*S), with f-1 the inverse function of Fl=f(conc), provided by StatLIA, and lower LOQ=f-1 (d+1 O*S).

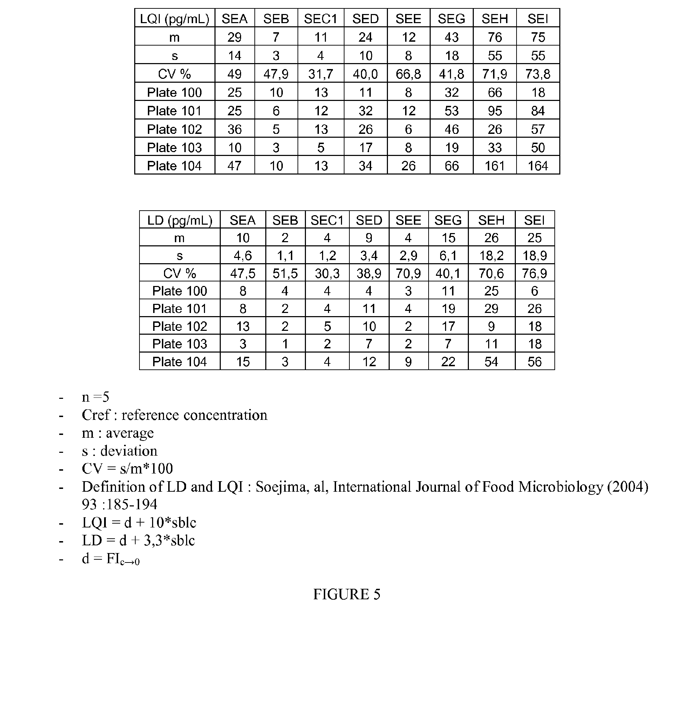

Results (plate 100 to 104, i.e. n=5) (see figure 5):

Table 13

| pg/mL | SEA | SEB | SEC1 | SED | SEE | SEG | SEH | SEI |
|---|---|---|---|---|---|---|---|---|
| **LOD** | 10 +/-4.6 | 2 +/-1.1 | 4 +/-1.2 | 9 +/-3.4 | 4 +/-2.9 | 15 +/-6.1 | 26 +/-18.2 | 25 +/-18.2 |
| **Lower LOQ** | 29 +/-13.9 | 7 +/-3.2 | 11 +/-3.6 | 24 +/-9.7 | 12 +/-8.1 | 43 +/-18.1 | 76 +/-54.8 | 75 +/-55.1 |

That method did not include a higher limit of quantification, and was dependent of the blanks (base line).

### 5.3. Linearity of the titration : Cobs = F(Cref)

Linearity of Cobs=f(Cref) tested on the 12 points of the standard range (n=5). Since $R^2$>0.99, the response could be considered as linear for indicated values (see the table 14 below).

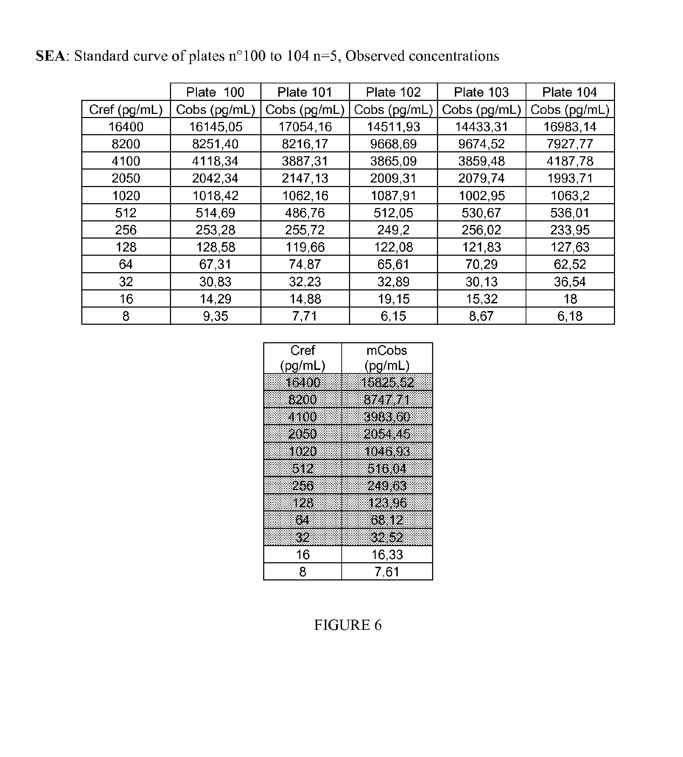

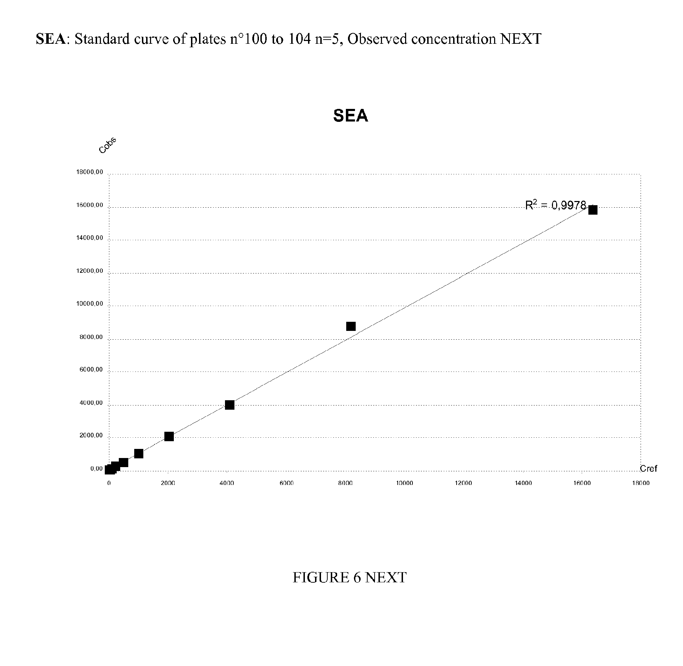

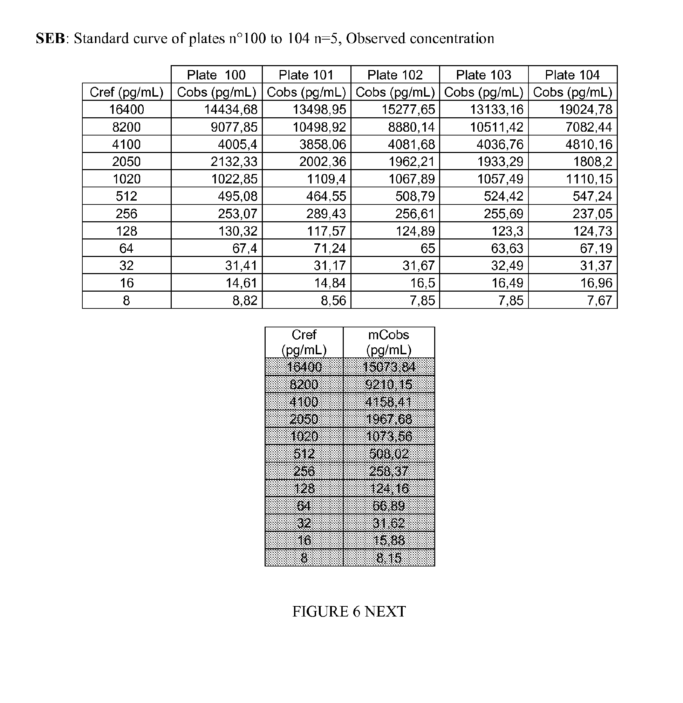

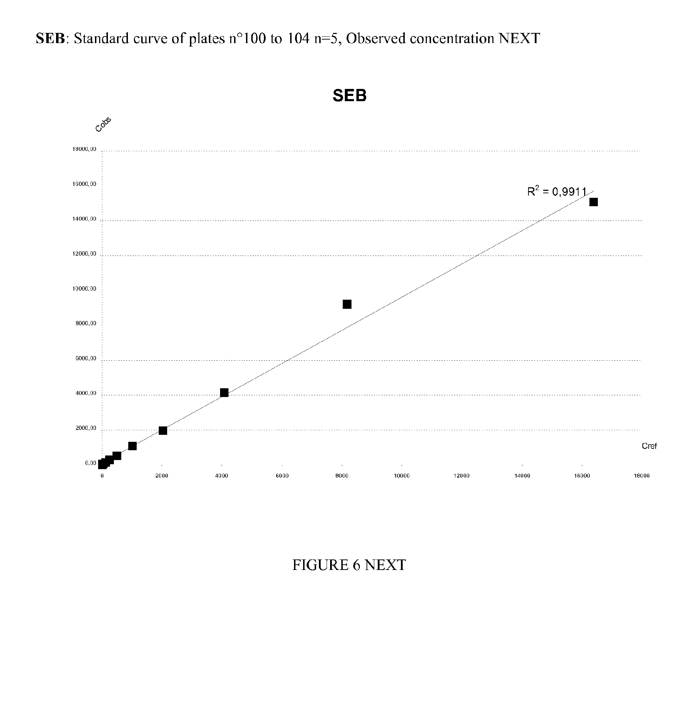

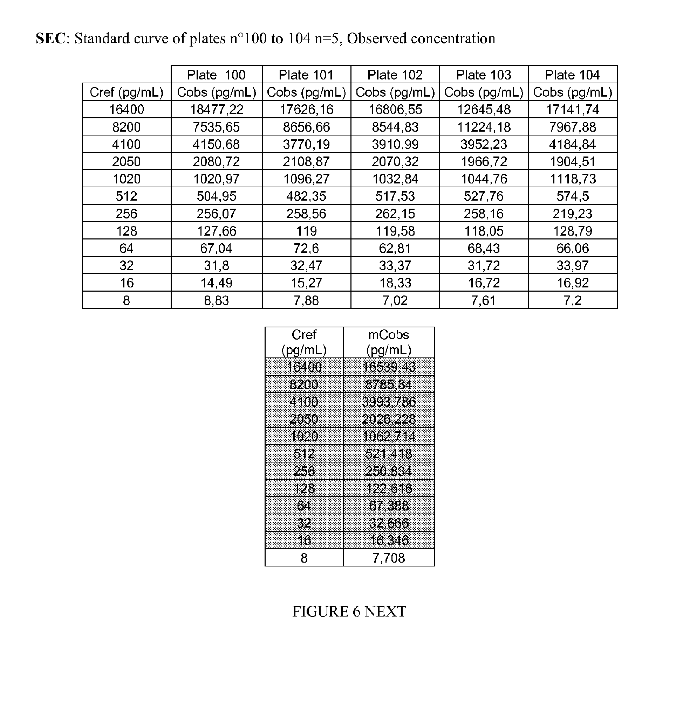

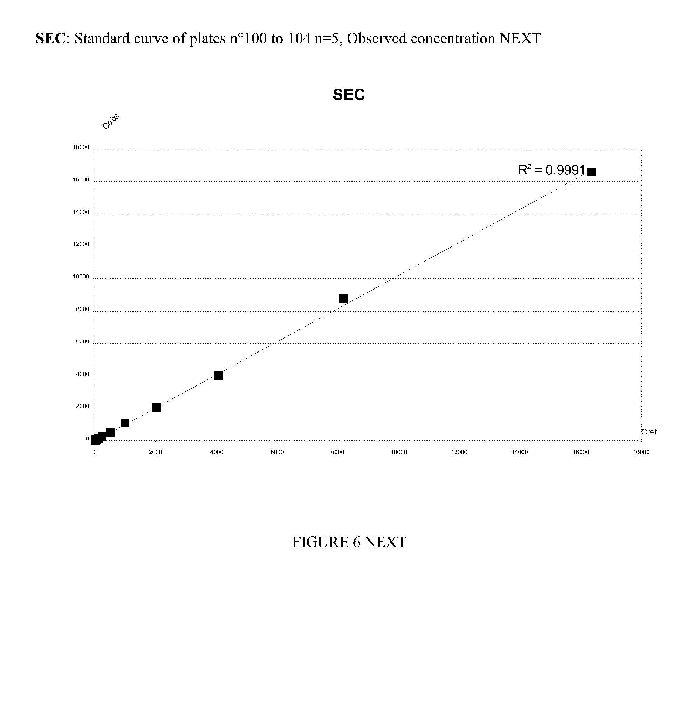

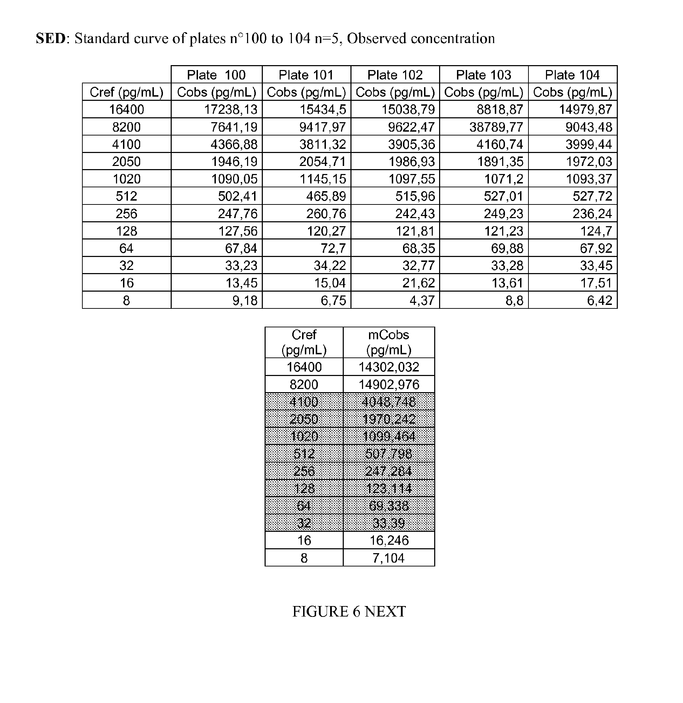

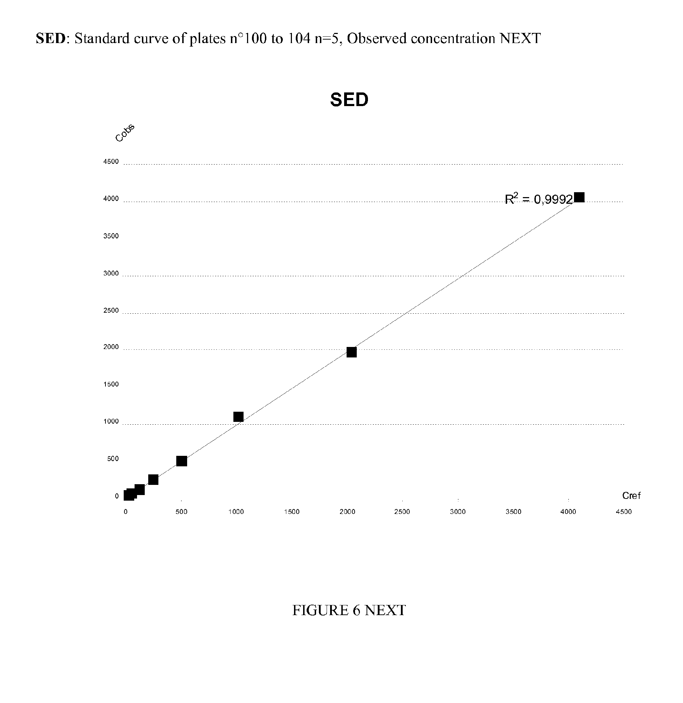

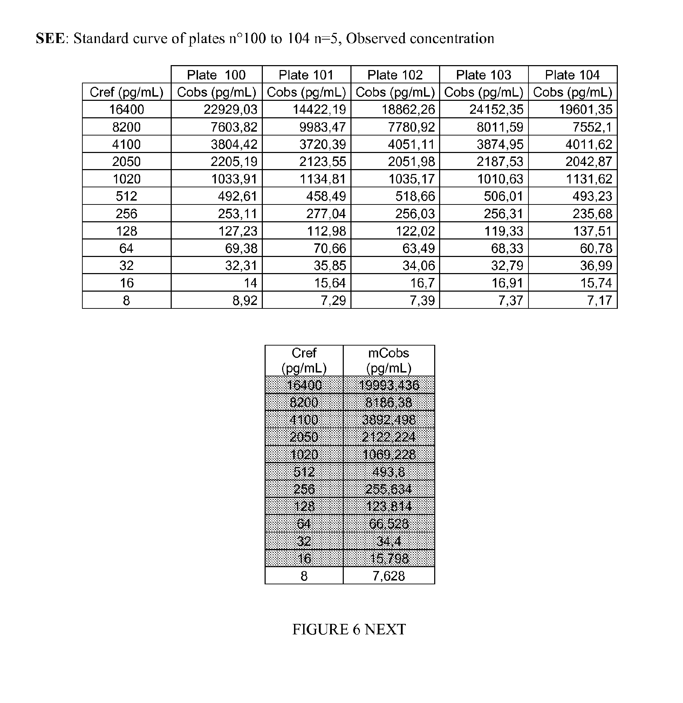

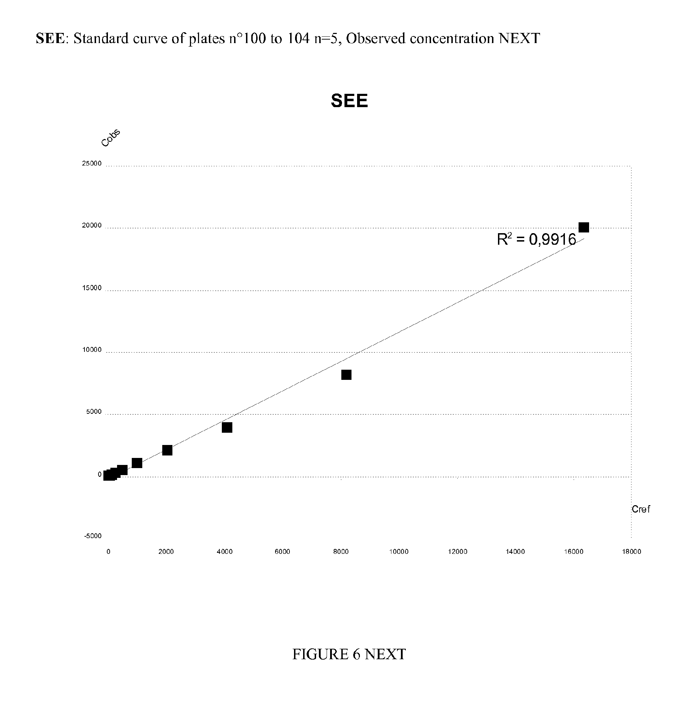

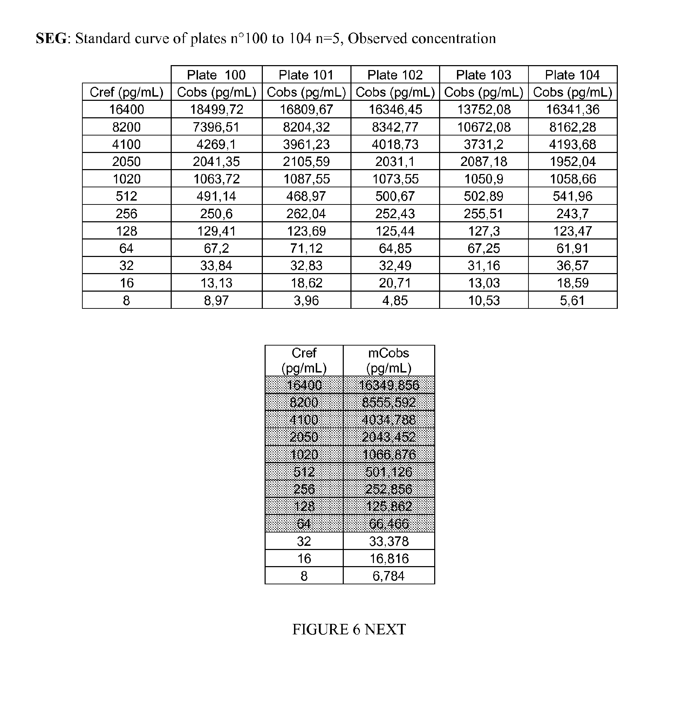

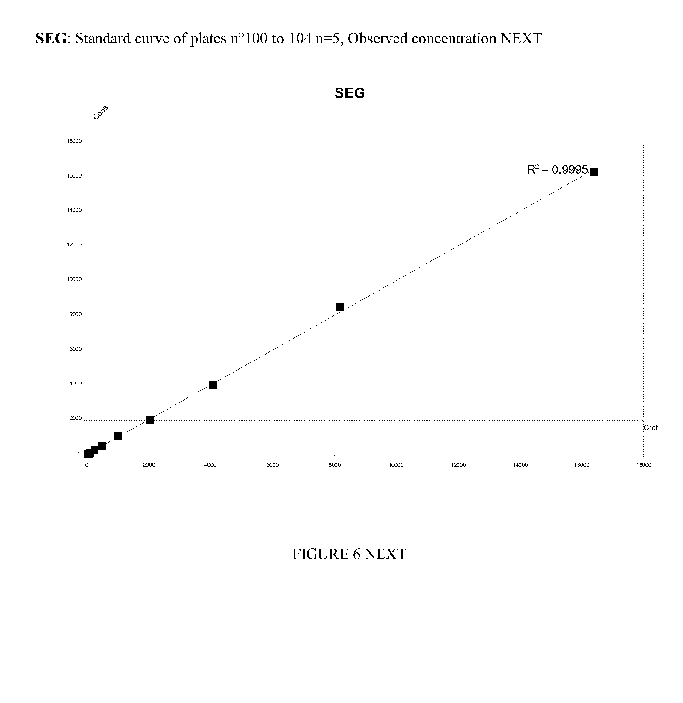

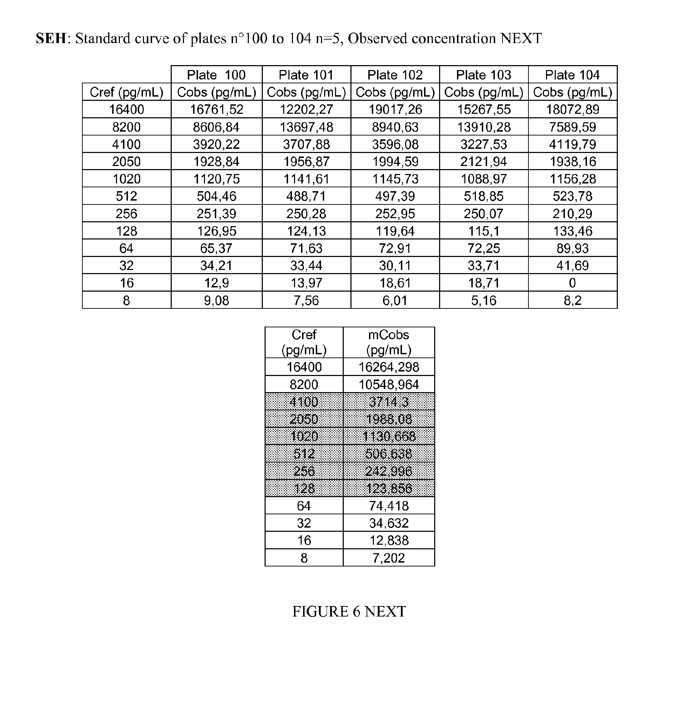

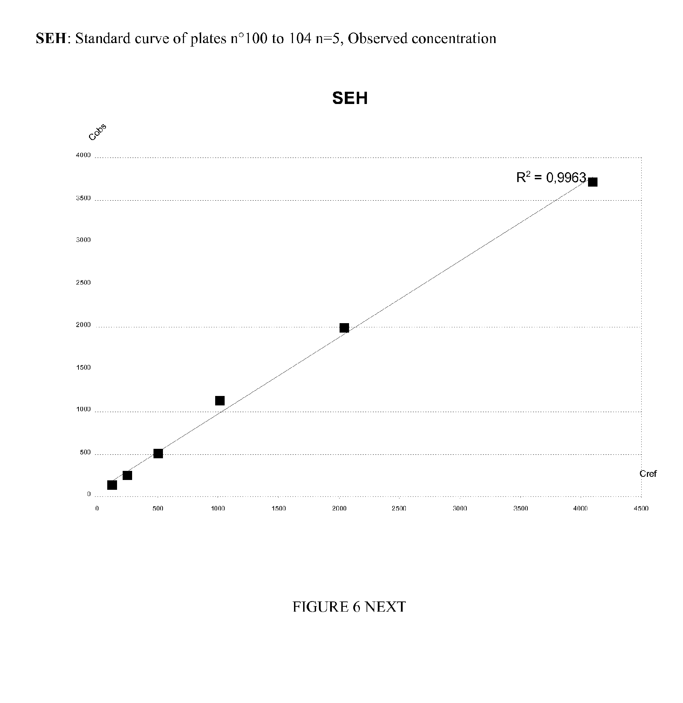

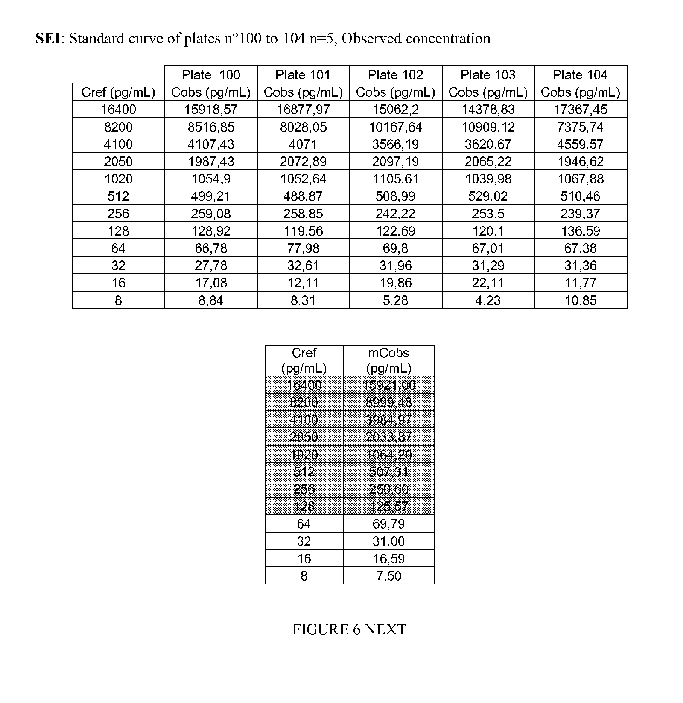

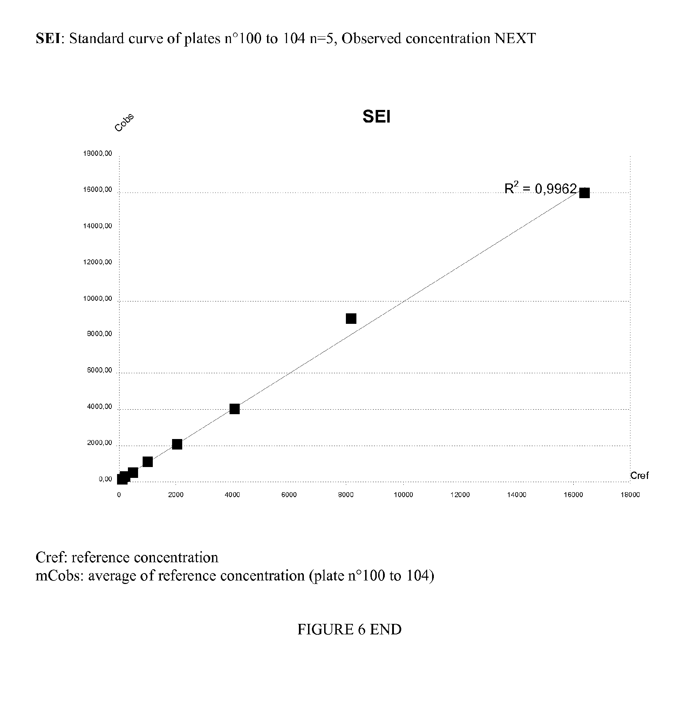

See Linearity of standard in figure 6.

Table 14

| pg/mL | SEA | SEB | SEC1 | SED | SEE | SEG | SEH | SEI |
|---|---|---|---|---|---|---|---|---|
| **$R^2$>0.99**** | 32 to 16400[ | 8 to 16400[ | 16 to 16400[ | 32 to 4100] | 16 to 16400[ | 64 to 16400[ | 128 to 4100[ | 128 to 16400[ |
| ** Whatever PBS buffer or milk considered, strictly comparable values were obtained. | | | | | | | | |

## EXAMPLE 6 : TITRATION OF THE 8 SEs IN DIFFERENT MATRIX

### <u>6.1. Protocol of matrix preparation</u>

It was adapted from the method of Macaluso and Lapeyre [Analusis, 28(7) : 610-615, 2000] [24], without the PEG 20 000 precipitation, and according to Soejima and al. [2004, fore-mentioned] [23] for the concentration by ultrafiltration and delipidation.

Before hand, the 8 SEs were added to the matrix at 4 concentrations (640

- 320 - 160 - 80 pg/mL) likely to be found in contaminated cheese and which the lowest concentration was close to the lower LOQ (particularly for SEH and SEI).

- 12.5g of munster (1st price Auchan) were cut in small pieces and added to 25mL of water in a 50mL tube (PP) at room-temperature, stirred vigorously 15sec and grinded by UltraTurrax™ during 1min. Freeze at -20°C for several days.
- The day of the preparation, homogenates were defrosted and fluidized at 37°C during several min by shaking. Then the 8 SEs were added at 640 - 320 - 160 - 80 pg/mL in 4 different homogenates, the 5th is the "matrix blank", stirred vigorously 15s and left aside for 15 min at room-temperature.

**Caseins precipitation:**

Samples were acidified with HCl (~5M) until pH 3.5 (check pH with pH indicator sticks pH2 to 9 +/- 0.5, with Hac solution 0.2M pH3.8 as a positive control). The HCl volume added was written: about 800μL were necessary. Samples were stirred and centrifuged 10 min at 7000 x *g*, at room-temperature. 20mL of the supernatant were taken between the pellet and the cream and 50mM of Tris (1mL) and 1mM of $CaCl_2$ (20μL) were added then the pH was adjusted at 7.5 +/- 0,3 with NaOH (~2M) (pH to check with Lyphan® L669, with HEPES solution 0.5M pH 7.5 as a positive control). The NaOH volume was written: about 300μL.

The resulting solution was stirred and centrifuged 10min, room-temperature at 7000 x *g*. ~15mL of the supernatant were kept (it may be slightly cloudy).

**Delipidation :**

2mL of $HCCl_3$ were added to each supernatant, stirred vigorously and centrifuged 10min at 7000 x *g* at room-temperature and the aqueous phase was kept. A disk of precipitation appeared between the two phases and the supernatant was unclouded.

**Desalting by ultrafiltration :**

4mL of each supernatant were placed in a ultrafiltration Amicon® 4 (Millipore™) system, with a nominal molecular weight limit of 10kDa, and centrifugated ~12min at 7500g at room-temperature (rotor 35°). The final volume was lower than 0.8mL. The retentate was homogenized and the volume was adjusted to 4mL with TBS (Tris-HCl 40mM + NaCl 140mM pH 7.5).

**x5 concentration by ultrafiltration:**

Each retentate was re-centrifugated with the same filter unit, then homogenized and the volume was adjusted to 800μL. That concentrate was more "viscous" than before 5x concentration.

Tween 20 at 0.05% and rabbit aspecific IgG at 10μg/mL were added to each sample, and frozen at -20°C.

Titration of the 8SEs were performed the day after according to the general instrumental protocol 4.

**6.2. Eight SEs titration in Munster- Results - Conclusion**

**6.2.1. Recovery rate in different matrix**

On the plate 037, there were 10 points (16384 to 32pg/mL, 2 in 2 of 7SEs(without SED)), prepared (in 50% of whole milk centrifugated and filtered on 0,45μm or 50% Brain Heart Infusion) in TBS + Tween 20 0,05% + rabbit IgG 100μg/mL.

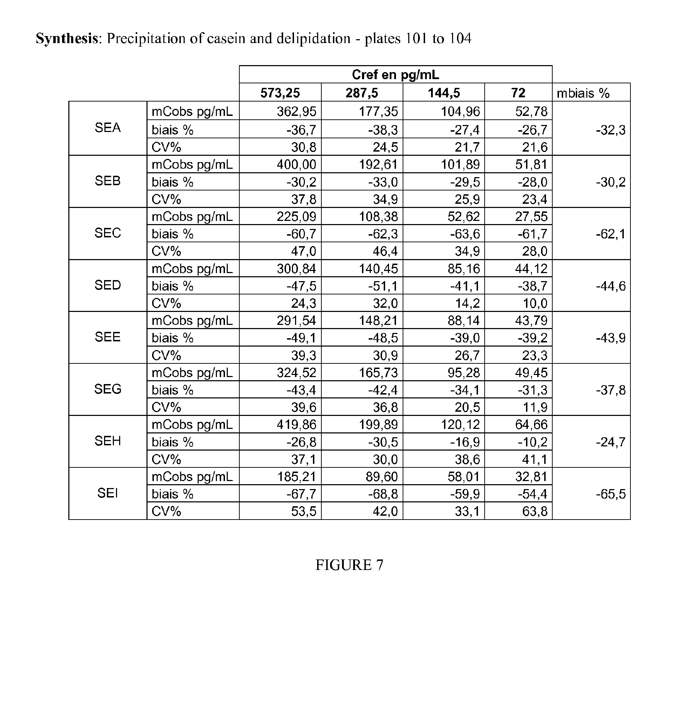

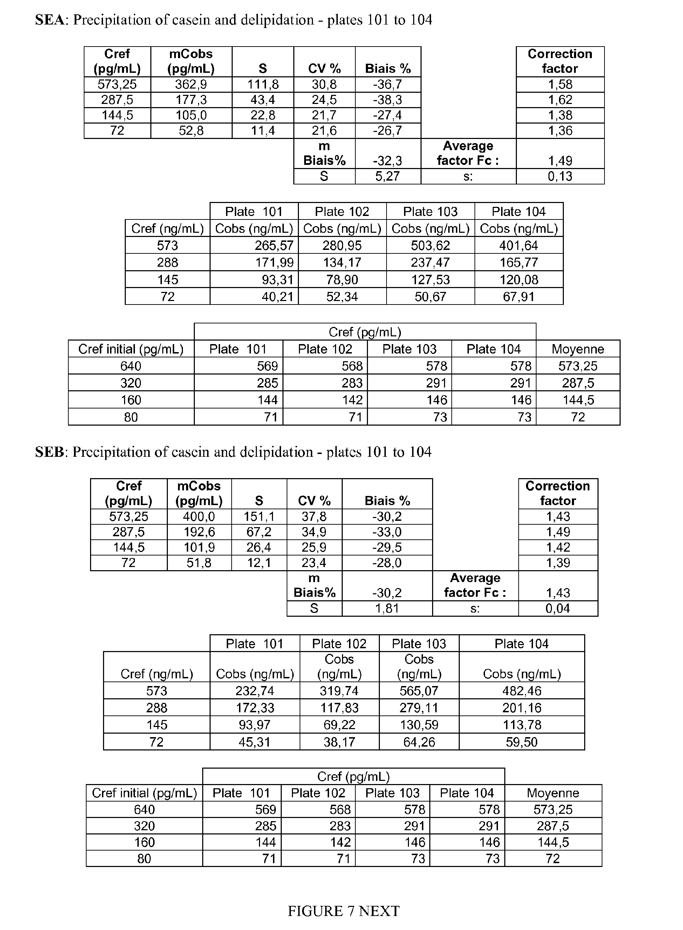

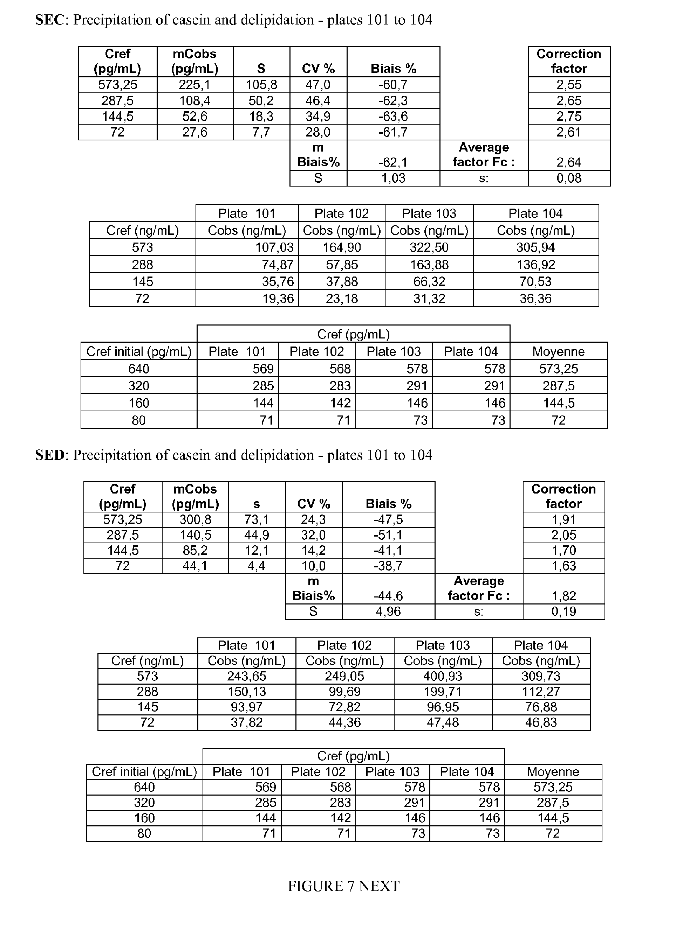

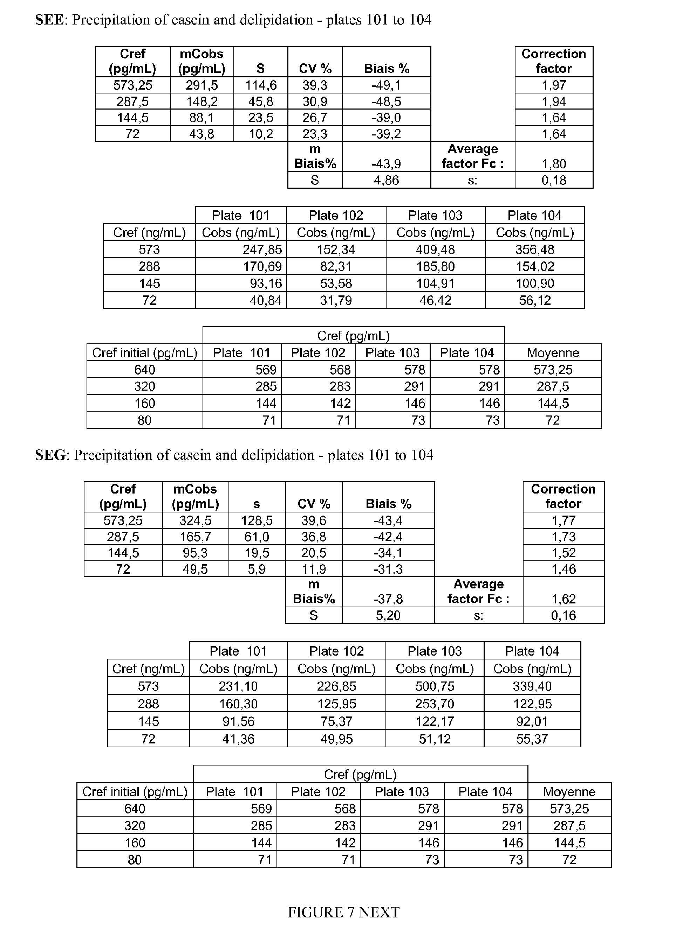

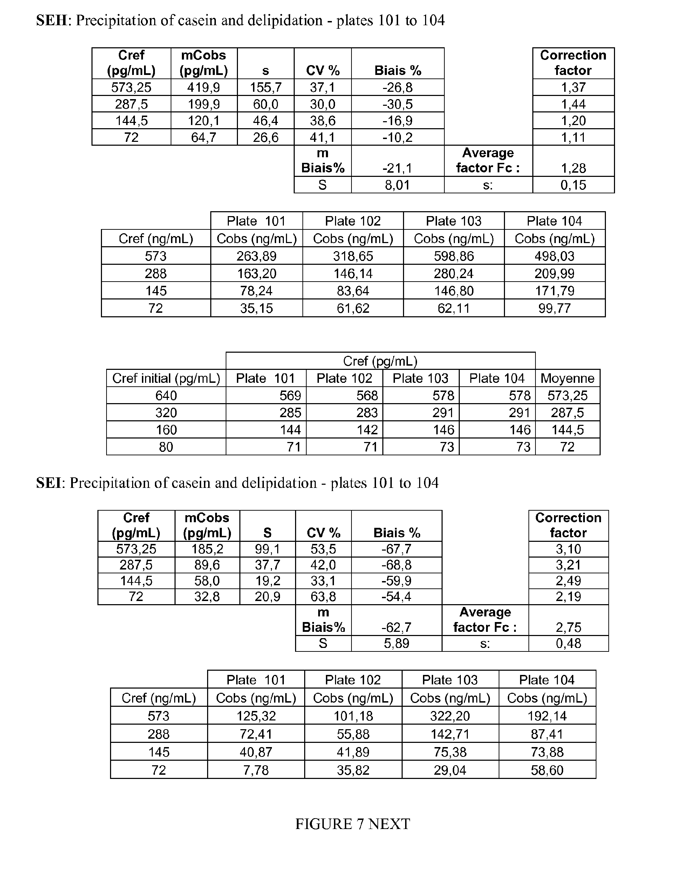

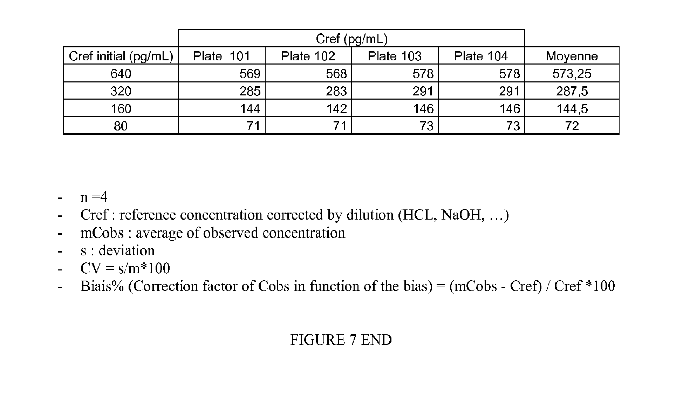

The bias % (Mu HCL) was obtained on Munster after caseins precipitation and delipidation (see also figure 7)

In order to turn the LOQ per g of cheese down, the matrix was concentrated after caseins precipitation and delipidation :

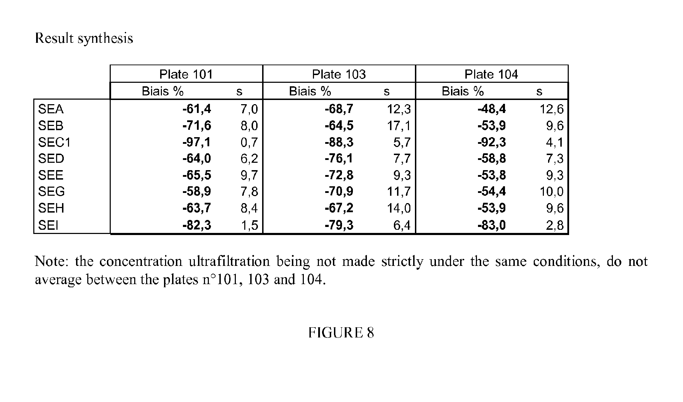

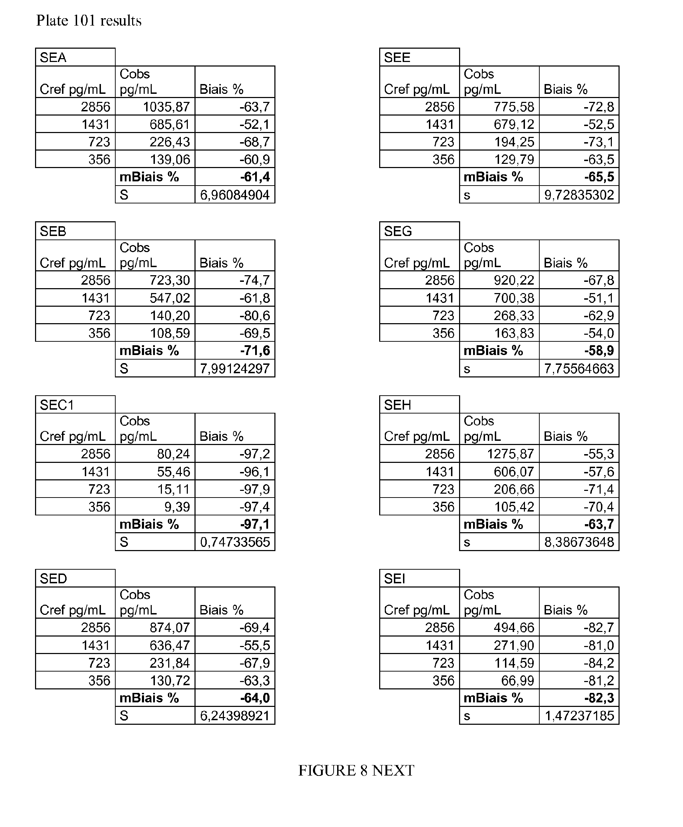

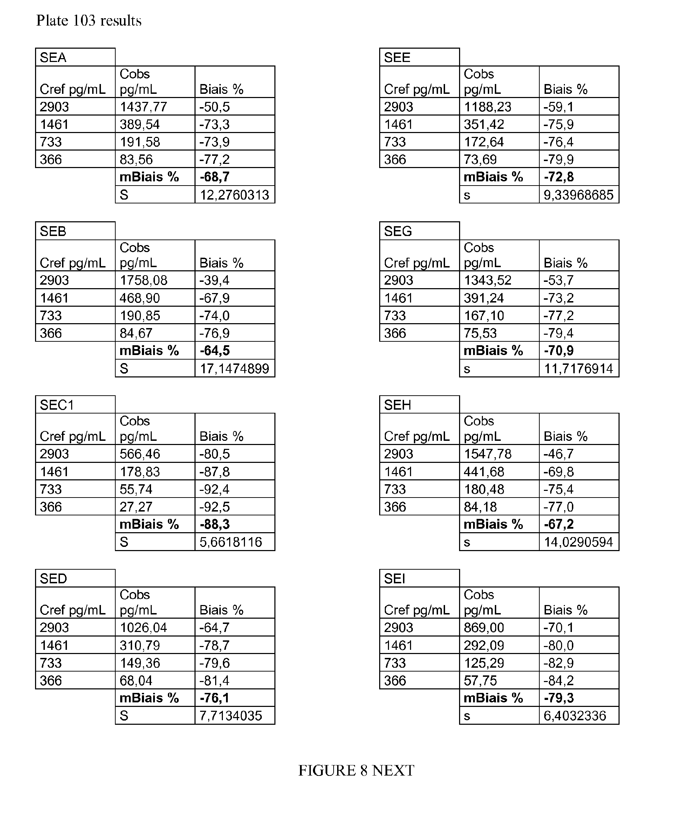

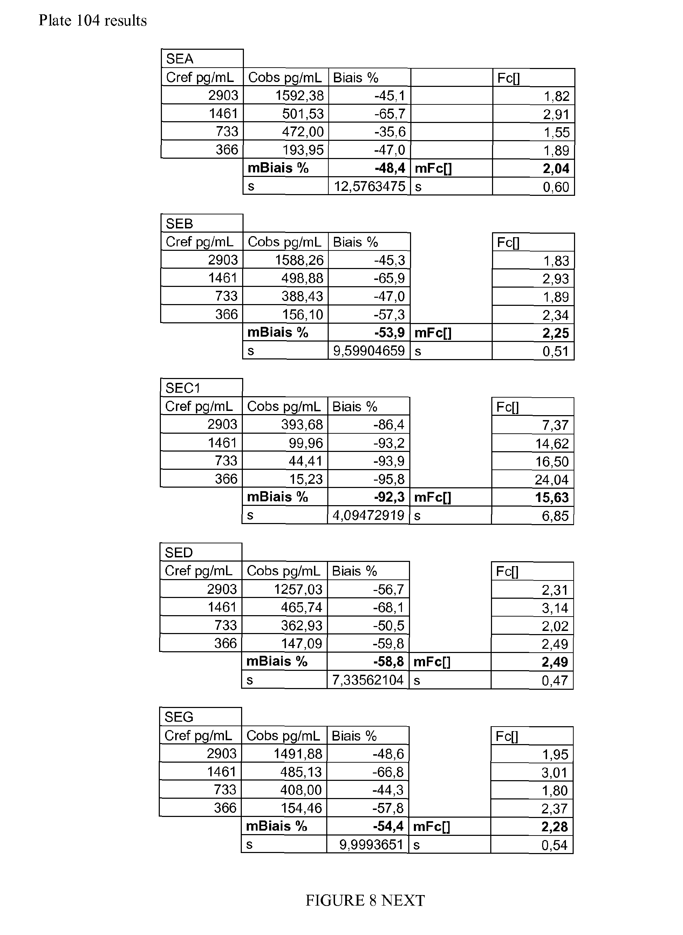

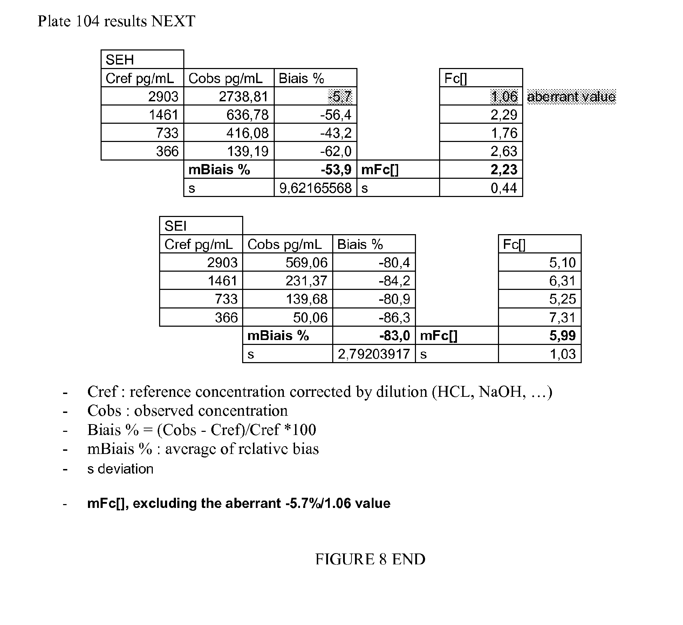

- Plate 101 : desalting then concentration as exposed in the matrix preparation protocole 6.1 (see also figure 8)
- Plate 103 : ultrafiltration of 10mL for 1h at 5000 x *g* at room-temperature with a nominal molecular weight limit of 300kDa to collect 6mL of filtrate, of which 4mLwere desalted and concentrated as exposed in the matrix preparation protocol 6.1. (see also figure 8)
- Plate 104 : ultracentrifugation of 10mL during 1h or 3h at 100 000 x g (33300 t/min 50 Ti) then 4mL were desalted and concentrated as exposed in the matrix preparation protocol 6.1. There were no significant differences between the results of 1h or 3h of centrifugation. (see also figure 8)

In the table 15 below, the relative bias (%) in relation to TBS+Tween+IgG in standard range was presented :

Table 15

| mBias % | SEA | SEB | SEC | SED | SEE | SEG | SEH | SEI |
|---|---|---|---|---|---|---|---|---|
| **Lait 50%** | -28.5 | -18.0 | -25.9 | ND | -22.5 | -26.1 | -21.2 | -62.6 |
| **BHI 50%** | -40.9 | -38.8 | -39.2 | ND | -40.1 | -43.3 | -46.1 | -68.0 |
| **Mu HCL** | -32.3 | -30.2 | -62.1 | -44.6 | -43.9 | -37.8 | -24.7 | -65.5 |
| **Mu 101** | -61.4 | -71.6 | -97.1 | -64.0 | -65.5 | -58.9 | -63.7 | -82.3 |
| **Mu 103** | -68.7 | -64.5 | -88.3 | -76.1 | -72.8 | -70.9 | -67.2 | -79.3 |
| **Mu 104** | -48.4 | -53.9 | -92.3 | -58.8 | -53.8 | -54.4 | -53.9 | -83.0 |
| ND : not determined | | | | | | | | |

If average biases were compared, the bias was mostly lower in whole milk than in munster or BHI. SEC showed an important difference between the milk and the munster, and that led to the problem of the protein stability in its secretion environment. SEI had always a high bias, probably due to the insufficient Ab sensibility of that generation.

After concentration, for the 8 SEs absolute value of the biases raise compared with casein precipitation+ delipidation : the inhibitory effect of the matrix raised with its concentration, whatever the conditions 101, 103 or 104 which had the less bad result.

**6.2.2 Proposal of the correction of the loss of 8Ses and lower LOD in Munster**

For all the SEs, an important negative bias has been evaluated, reflecting the inhibitor effect of the matrix. However, that bias could be corrected by a factor defined in the table below : (+/- s (standard deviation)) :

After casein precipitation and delipidation

Table 16

| Cf ± s | SEA | SEB | SEC1 | SED | SEE | SEG | SEH | SEI |
|---|---|---|---|---|---|---|---|---|
| **Correction factor** | 1.49 +/-0.13 | 1.43 +/-0.04 | 2.64 +/-0.08 | 1.82 +/-0.19 | 1.80 +/-0.18 | 1.62 +/-0.16 | 1.28 +/-0.15 | 2.75 +/-0.48 |

Note : the concentration Cobs had been introduced regardless the LOQ. Then, due to a negative bias, for SEH and SEI, Cobs are lower than the LOQ for the 72pg/mL point, which did not have been deleted for that study.

Allowing the dilution factor of 1/3.3 from the matrix preparation and the negative bias correlated to Cf, a new LOQ per g of cheese could be revealed: LOQ pg/g = LOQ pg/mL x 3.3 x Cf (see the table 17 below).

Table 17

| pg/g | SEA | SEB | SEC1 | SED | SEE | SEG | SEH | SEI |
|---|---|---|---|---|---|---|---|---|
| **LOQ** | 143 | 33 | 96 | 144 | 65 | 230 | 321 | 681 |

After casein precipitation delipidation and concentration (plate 104 conditions)

Table 18

| Cf* +/-s | SEA | SEB | SEC1 | SED | SEE | SEG | SEH | SEI |
|---|---|---|---|---|---|---|---|---|
| **Correction Factor *** | 2.04 +/-0.60 | 2.25 +/-0.51 | 15.63 +/-6.85 | 2.49 +/-0.47 | 2.24 +/-0.53 | 2.28 +/-0.54 | 2.23 +/-0.44 | 5.99 +/-1.03 |

Allowing the concentration factor of 5/3.3 resulting from the matrix preparation and the negative bias reflected by Cf*, a new lower LOQ per g of cheese could be revealed: Lower LOQ pg/g of cheese = lower LOQ pg/mL x 3.3/5 x Cf (see the table 19 below).

Table 19

| pg/g | SEA | SEB | SEC1 | SED | SEE | SEG | SEH | SEI |
|---|---|---|---|---|---|---|---|---|
| Lower LOQ | 39 | 10 | 113 | 39 | 18 | 65 | 112 | 297 |

Overall, the lower LOQ per g of cheese drop after concentration, apart from SEC1. But since Cf* > Cf, the result uncertainty raises.

The immunoabsorbed polyclonal antibodies developed here could be used in different tests, among which the Luminex™ technology. These antibodies conserved a high affinity and gathered the possibility to detect all SEs already designed to be responsible for collective food-born intoxinations.

**List of references**

1. Jones and Kahn, J. Bacteriol., 166 : 29-33, 1986
2. Betley and Mekalanos, J. Bacteriol., 170(1): 34-41, 1988
3. Couch and al., J. Bacteriol., 170 : 2954-2960, 1988
4. Bayles and Iandolo, J. Bacteriol., 171 : 4799-4806, 1989
5. Dingues and al., Clin. Microbiol. Rev., 13 : 16-34, 2000
6. Bergdoll and al., J. Bacteriol., 90(5): 1481-1485, 1965
7. Marr and al., Infect. Immun., 61 : 4254-4262, 1993
8. Bergdoll, Lancet, 1 : 1017-1021, 1981
9. Blomster-Hautamaa and al., J. Biol. Chem., 261 : 15783-15786, 1986
10. Lina and al., J. Infect. Dis., 189(12) : 2334-2336, 2004
11. Ren et al., J. Exp. Med., 180(5) : 1675-1683, 1994
12.Jarraud and al., J. Immunol., 166(1) : 669-677, 2001
13.Orwin and al., Infect. Immun., 69(1) : 360-366, 2001
14. Letertre and al., Mol. Cell Probes, 17 : 227-235, 2003
15.Omoe and al., J. Clin. Microbiol., 40 : 857-862, 2002
16. Su and Wong, J. Food Prot., 59(3) : 327-330, 1996
17. Munson and al., Infect. Immun., 66 : 3337-3348, 1998
18.Ono and al., Infect. Immun., 76(11) : 4999-5005, 2008
19. Smith and Johnson, Gene, 67(1) : 31-40, 1988
20. Habig and al., J. Biol. Chem., 249 : 7130-7139, 1974
21. Gampfer et al., Vaccine, 20 : 3675-3684, 2002
22. Borkowski and al., Clin. Tech. Small Anim. Pract., 14.(1): 44-49, 1999
23.Soejima and al., Int. J. Food Microbiol., 93 : 185-194, 2004
24. Macaluso et al., Analusis, 28(7 ) : 610-615, 2000

## Claims

**1.** Immunological composition comprising a mixture of at least two immunoabsorbed polyclonal antibodies each raised against a staphylococcal enterotoxin, and a pharmaceutically acceptable carrier.

**2.** Immunological composition according to claim 1, wherein each immunoabsorbed polyclonal antibody is raised against a staphylococcal enterotoxin chosen from the group consisting of the staphylococcal enterotoxins A, B, C, D, E, G, H, and I.

**3.** Method for multiplex detection of staphylococcal enterotoxins, comprising:

a) contacting a sample with an immunological composition as defined in claim 1 or 2 ;
b) detecting potential immunological complexes formed.

**4.** Use of an immunological composition as defined in claim 1 or 2, as a diagnostic tool of a staphylococcal enterotoxin contamination.

**5.** Kit for the multiplex detection of staphylococcal enterotoxins, comprising an immunological composition as defined in claim 1 or 2, and means for the detection of immunological complexes.

**6.** Method for the preparation of an immunoabsorbed polyclonal antibody raised against a staphylococcal enterotoxin, comprising :

a) providing an anti-enterotoxin polyclonal antibody previously obtained from the immunization of a non-human animal with a staphylococcal enterotoxin ;
b) purification of said anti-enterotoxin polyclonal antibody using at least two successive immunoabsorption steps against immunization-unrelated staphylococcal enterotoxins and which order is chosen to abolish cross-reactions with said immunization-unrelated staphylococcal enterotoxins from the strongest cross-reaction to the weaker cross-reaction.

**7.** Method according to claim 6 for the preparation of an immunoabsorbed anti-enterotoxin A polyclonal antibody, comprising :

a) providing an anti-enterotoxin A polyclonal antibody previously obtained from the immunization of a non-human animal with the staphylococcal enterotoxin A ;
b) purification of said anti-enterotoxin A polyclonal antibody following 5 successive immunoabsorption steps against the immunization-unrelated staphylococcal enterotoxin E, E, I, B, then D.

**8.** Method according to claim 6 for the preparation of an immunoabsorbed anti-enterotoxin B polyclonal antibody, comprising :

a) providing an anti-enterotoxin B polyclonal antibody previously obtained from the immunization of a non-human animal with the staphylococcal enterotoxin B ;
b) purification of said anti-enterotoxin B polyclonal antibody following 3 successive immunoabsorption steps against the immunization-unrelated staphylococcal enterotoxin C1, C1, then G.

**9.** Method according to claim 6 for the preparation of an immunoabsorbed anti-enterotoxin C polyclonal antibody, comprising :

a) providing an anti-enterotoxin C polyclonal antibody previously obtained from the immunization of a non-human animal with the staphylococcal enterotoxin C ;
b) purification of said anti-enterotoxin C1 polyclonal antibody following 3 successive immunoabsorption steps against immunization-unrelated staphylococcal enterotoxin B, B, then G;

**10.** Method according to claim 6 for the preparation of an immunoabsorbed anti-enterotoxin D polyclonal antibody, comprising :

a) providing an anti-enterotoxin D polyclonal antibody previously obtained from the immunization of a non-human animal with the staphylococcal enterotoxin D ;
b) purification of said anti-enterotoxin D polyclonal antibody following 3 successive immunoabsorption steps against the immunization-unrelated staphylococcal enterotoxin A, A, then E.

**11.** Method according to claim 6 for the preparation of an immunoabsorbed anti-enterotoxin E polyclonal antibody, comprising :

a) providing an anti-enterotoxin E polyclonal antibody previously obtained from the immunization of a non-human animal with the staphylococcal enterotoxin E ;
b) purification of said anti-enterotoxin E polyclonal antibody following 4 successive immunoabsorption steps against the immunization-unrelated staphylococcal enterotoxin A, A, I, then C1.

**12.** Method according to claim 6 for the preparation of an immunoabsorbed anti-enterotoxin G polyclonal antibody, comprising :

a) providing an anti-enterotoxin G polyclonal antibodiy previously obtained from the immunization of a non-human animal with the staphylococcal enterotoxin G ;
b) purification of said anti-enterotoxin A polyclonal antibody following 4 successive immunoabsorption steps against the immunization-unrelated staphylococcal enterotoxin A, B, I, then C1.

**13.** Method according to claim 6 for the preparation of an immunoabsorbed anti-enterotoxin H polyclonal antibody, comprising :

a) providing an anti-enterotoxin H polyclonal antibody previously obtained from the immunization of a non-human animal with the staphylococcal enterotoxin H ;
b) purification of said anti-enterotoxin A polyclonal antibody following 2 successive immunoabsorption steps against the immunization-unrelated staphylococcal enterotoxin B, then D.

**14.** Method according to claim 6 for the preparation of an immunoabsorbe anti-enterotoxin I polyclonal antibody, comprising :

a) providing an anti-enterotoxin I polyclonal antibody previously obtained from the immunization of a non-human animal with the staphylococcal enterotoxin I ;
b) purification of said anti-enterotoxin I polyclonal antibody following 5 successive immunoabsorption steps against immunization-unrelated staphylococcal enterotoxin E, C1, G, B, then A.

**15.** Immunoabsorbed anti-enterotoxin polyclonal antibody raised against a staphylococcal enterotoxin obtained by a method according to any one of claims 6 to 14.

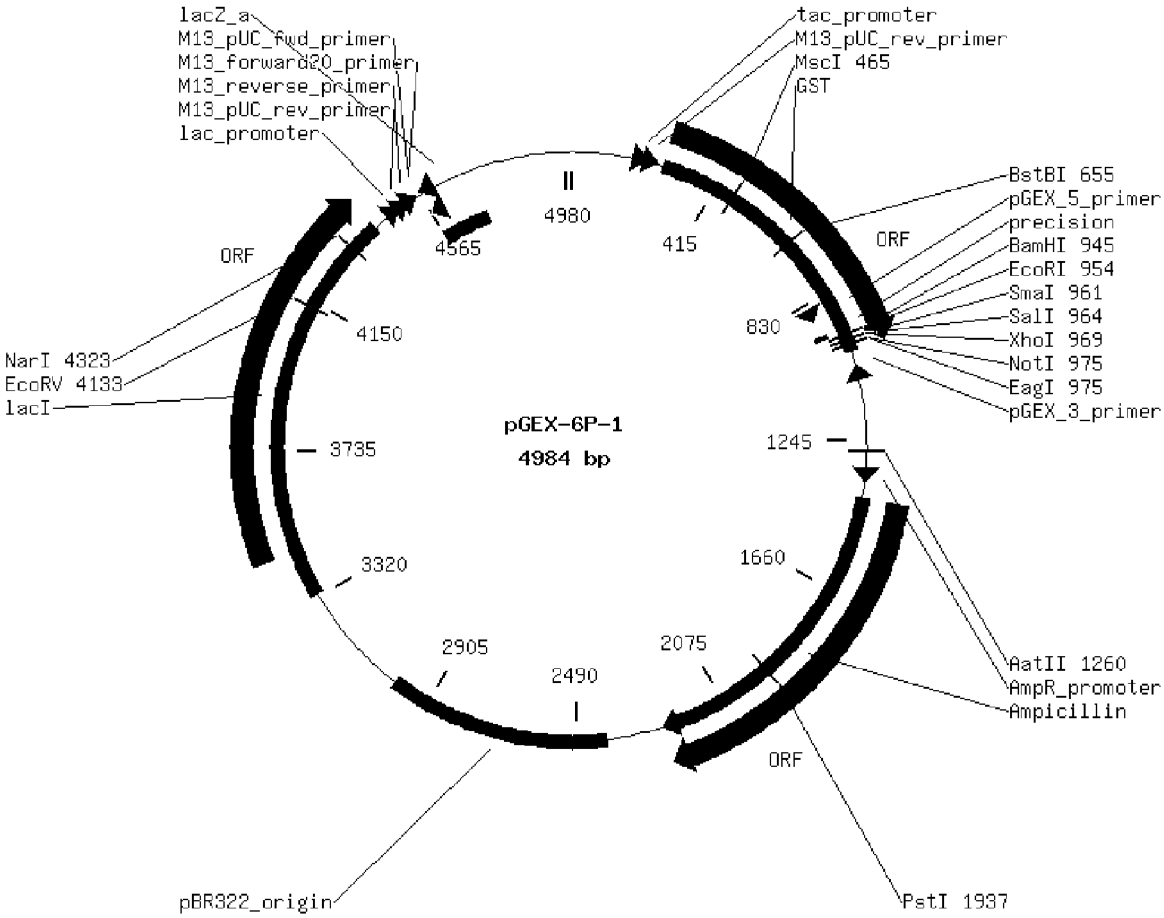
lacZ_a
M13_pUC_fwd_primer
M13_forward20_primer
M13_reverse_primer
M13_pUC_rev_primer
lac_promoter
tac_promoter
M13_pUC_rev_primer
MscI 465
GST
BstBI 655
pGEX_5_primer
precision
BamHI 945
EcoRI 954
SmaI 961
SalI 964
XhoI 969
NotI 975
EagI 975
pGEX_3_primer
ORF
4980
415
830
1245
1660
2075
2490
2905
3320
3735
4150
4565
pGEX-6P-1
4984 bp
NarI 4323
EcoRV 4133
lacI
AatII 1260
AmpR_promoter
Ampicillin
ORF
PstI 1937
pBR322_origin

FIGURE 1

SEA+5 (SEQ ID NO: 15):
GPLGSSEKSE EINEKDLRKK SELQGTALGN LKQIYYYNEK AKTENKESHD QFLQHTILFK GFFTDHSWYN DLLVDFDSKD IVDKYKGKKV DLYGAYYGYQ CAGGTPNKTA CMYGGVTLHD NNRLTEEKKV PINLWLDGKQ NTVPLETVKT NKKNVTVQEL DLQARRYLQE KYNLYNSDVF DGKVQRGLIV FHTSTEPSVN YDLFGAQGQY SNTLLRIYRD NKTINSENMH IDIYLYTS

SEB+5 (SEQ ID NO: 16):
GPLGSESQPD PKPDELHKSS KFTGLMENMK VLYDDNHVSA INVKSIDQFL YFDLIYSIKD TKLGNYDNVR VEFKNKDLAD KYKDKYVDVF GANYYYQCYF SKKTNDINSH QTDKRKTCMY GGVTEHNGNQ LDKYRSITVR VFEDGKNLLS FDVQTNKKKV TAQELDYLTR HYLVKNKKLY EFNNSPYETG YIKFIENENS FWYDMMPAPG DKFDQSKYLM MYNDNKMVDS KDVKIEVYLTTKKK

SEC1+8 (SEQ ID NO: 17):
GPLGSPEFES QPDPTPDELH KASKFTGLME NMKVLYDDHY VSATKVKSVD KFLAHDLIYN ISDKKLKNYD KVKTELLNEG LAKKYKDEVV DVYGSNYYVN CYFSSKDNVG KVTGGKTCMY GGITKHEGNH FDNGNLQNVL IRVYENKRNT ISFEVQTDKK SVTAQELDIK ARNFLINKKN LYEFNSSPYE TGYIKFIENN GNTFWYDMMP APGDKFDQSK YLMMYNDNKT VDSKSVKIEV HLTTKNG

SED (SEQ ID NO: 18):
KHSYADKNPI IGENKSTGDQ FLENTLLYKK FFTDLINFED LLINFNSKEM AQHFKSKNVD VYPIRYSINC YGGEIDRTAC TYGGVTPHEG NKLKERKKIP INLWINGVQK EVSLDKVQTD KK NVTVQELD AQARRYLQKD LKLYNNDTLG GKIQRGKIEF DSSDGSKVSY DLFDVKGDFP EKQLRIYSDN KTLSTEHLHI DIYLYEK

SEE+5 (SEQ ID NO: 19):
GPLGSSE EIN EKDLRKKSEL QRNALSNLRQ IYYYNEKAIT ENKESDDQFL ENTLLFKGFF TGHPWYNDLL VDLGSKDATN KYKGKKVDLY GAYYGYQCAG GTPNKTACMY GGVTLHDNNR LTEEKKVPIN LWIDGKQTTV PIDKVKTSKK EVTVQELDLQ ARHYLHGKFG LYNSDSFGGK VQRGLIVFHS SEGSTVSYDL FDAQGQYPDT LLRIYRDNKT INSENLHIDL YLYTT

SEG+8 (SEQ ID NO: 20):
GPLGSPEFQP DPKLDELNKV SDYKNNKGTM GNVMNLYTSP PVEGRGVINS RQFLSHDLIF PIEYKSYNEV KTELENTELA NNYKDKKVDI FGVPYFYTCI IPKSEPDINQ NFGGCCMYGG LTFNSSENER DKLITVQVTI DNRQSLGFTI TTNKNMVTIQ ELDYKARHWL TKEKKLYEFD GSAFESGYIK FTEKNNTSFW FDLFPKKELV PFVPYKFLNI YGDNKVVDSK SIKMEVFLNT H

SEH+5 (SEQ ID NO: 21):
GPLGSEDLHD KSELTDLALA NAYGQYNHPF IKENIKSDEI SGEKDLIFRN QGDSGNDLRV KFATADLAQK FKNKNVDIYG ASFYYKCEKI SENISECLYG GTTLNSEKLA QERVIGANVW VDGIQKETEL IRTNKKNVTL QELDIKIRKI LSDKYKIYYK DSEISKGLIE FDMKTPRDYS FDIYDLKGEN DYEIDKIYED NKTLKSDDIS HIDVNLYTKK KV

SEI+5 (SEQ ID NO: 22):
GPLGSQGDIG VGNLRNFYTK HDYIDLKGVT DKNLPIANQL EFSTGTNDLI SESNNWDEIS KFKGKKLDIF GIDYNGPCKS KYMYGGATLS GQYLNSARKIP INLWVNGKH KTISTDKIAT NKKLVTAQEI DVKLRRYLQE EYNIYGHNNT GKGKEYGYKS KFYSGFNNGK VLFHLNNEKS FSYDLFYTGD GLPVSFLKIY EDNKIIESEK FHLDVEISYV DSN

FIGURE 2

**SEA**: Standard curve from plates n°100 to 104 n=5 duplicate measurements

| Cref (ng/mL) | mFI | s | λ | CV % | λ % | ε | ε% |
|---|---|---|---|---|---|---|---|
| 16,400 | 7130,6 | 714,5 | 887,0 | 10,0 | 12,4 | 1983,4 | 27,8 |
| 8,200 | 6283,8 | 856,3 | 1063,1 | 13,6 | 16,9 | 2377,1 | 37,8 |
| 4,100 | 4507,5 | 620,1 | 769,8 | 13,8 | 17,1 | 1721,3 | 38,2 |
| 2,050 | 2872,4 | 501,8 | 623,0 | 17,5 | 21,7 | 1393,0 | 48,5 |
| 1,020 | 1602,2 | 301,3 | 374,0 | 18,8 | 23,3 | 836,3 | 52,2 |
| 0,512 | 805,8 | 139,6 | 173,3 | 17,3 | 21,5 | 387,5 | 48,1 |
| 0,256 | 388,0 | 71,1 | 88,2 | 18,3 | 22,7 | 197,3 | 50,9 |
| 0,128 | 187,5 | 27,6 | 34,2 | 14,7 | 18,3 | 76,5 | 40,8 |
| 0,064 | 101,0 | 15,7 | 19,4 | 15,5 | 19,3 | 43,5 | 43,1 |
| 0,032 | 46,5 | 5,3 | 6,5 | 11,3 | 14,1 | 14,6 | 31,5 |
| 0,016 | 22,9 | 5,8 | 7,1 | 25,2 | 31,3 | 16,0 | 69,9 |
| 0,008 | 10,4 | 8,3 | 10,2 | 79,4 | 98,5 | 22,9 | 220,3 |

| | Plate 100 | Plate 101 | Plate 102 | Plate 103 | Plate 104 |
|---|---|---|---|---|---|
| Cref (ng/mL) | FI-bkg | FI-bkg | FI-bkg | FI-bkg | FI-bkg |
| 16,400 | 7481,3 | 7395,7 | 7779,6 | 7058,5 | 5938 |
| 8,200 | 6469 | 6274,9 | 7266,8 | 6498 | 4910,3 |
| 4,100 | 4885,5 | 4422,4 | 5207,1 | 4461,8 | 3560,8 |
| 2,050 | 3124,5 | 2893,9 | 3374,8 | 2926 | 2043 |
| 1,020 | 1745,3 | 1551,9 | 1986,8 | 1561 | 1165,8 |
| 0,512 | 906,8 | 719,4 | 945,6 | 848,5 | 608,5 |
| 0,256 | 439,8 | 371,4 | 444,8 | 411,8 | 272 |
| 0,128 | 216,5 | 167,4 | 205,6 | 197,3 | 150,5 |
| 0,064 | 109,8 | 101,4 | 102,6 | 116 | 75 |
| 0,032 | 48,8 | 39,9 | 45,1 | 54 | 44,5 |
| 0,016 | 22,5 | 15,7 | 21,8 | 31,8 | 22,5 |
| 0,008 | 15 | 5,9 | 0,8 | 22 | 8,3 |

**SEB**: Standard curve from plates n°100 to 104 n=5 duplicate measurements

| Cref (ng/mL) | mFI | s | λ | CV % | λ % | ε | ε% |
|---|---|---|---|---|---|---|---|
| 16,400 | 9665,4 | 762,4 | 946,5 | 7,9 | 9,8 | 2116,5 | 21,9 |
| 8,200 | 9099,0 | 1021,6 | 1268,3 | 11,2 | 13,9 | 2836,0 | 31,2 |
| 4,100 | 7591,1 | 667,5 | 828,7 | 8,8 | 10,9 | 1852,9 | 24,4 |
| 2,050 | 5545,8 | 782,5 | 971,4 | 14,1 | 17,5 | 2172,1 | 39,2 |
| 1,020 | 3812,8 | 453,3 | 562,7 | 11,9 | 14,8 | 1258,3 | 33,0 |
| 0,512 | 2132,6 | 288,1 | 357,7 | 13,5 | 16,8 | 799,8 | 37,5 |
| 0,256 | 1178,7 | 178,7 | 221,9 | 15,2 | 18,8 | 496,1 | 42,1 |
| 0,128 | 590,9 | 87,4 | 108,5 | 14,8 | 18,4 | 242,6 | 41,1 |
| 0,064 | 321,2 | 31,2 | 38,8 | 9,7 | 12,1 | 86,7 | 27,0 |
| 0,032 | 152,1 | 16,9 | 21,0 | 11,1 | 13,8 | 47,0 | 30,9 |
| 0,016 | 75,2 | 7,6 | 9,5 | 10,2 | 12,6 | 21,2 | 28,2 |
| 0,008 | 36,5 | 6,5 | 8,1 | 17,9 | 22,3 | 18,2 | 49,8 |

FIGURE 3

| | Plate 100 | Plate 101 | Plate 102 | Plate 103 | Plate 104 |
|---|---|---|---|---|---|
| Cref (ng/mL) | FI-bkg | FI-bkg | FI-bkg | FI-bkg | FI-bkg |
| 16,400 | 10024,8 | 9938,3 | 10467,6 | 9422,2 | 8474,2 |
| 8,200 | 9342,3 | 9652,3 | 9946,8 | 9209,4 | 7344,4 |
| 4,100 | 7657,3 | 7610,6 | 8504,8 | 7562,2 | 6620,7 |
| 2,050 | 6010,5 | 5589,8 | 6338,3 | 5510,4 | 4280,2 |
| 1,020 | 4013,8 | 3782,8 | 4370,1 | 3768,4 | 3128,7 |
| 0,512 | 2357 | 1849,8 | 2450,3 | 2186,4 | 1819,7 |
| 0,256 | 1300,8 | 1204,6 | 1334,3 | 1171,4 | 882,2 |
| 0,128 | 674,3 | 513,8 | 676,8 | 599,7 | 489,9 |
| 0,064 | 338 | 316,6 | 357,8 | 319,7 | 273,7 |
| 0,032 | 151,5 | 140,8 | 172,8 | 164,4 | 131,2 |
| 0,016 | 71,8 | 67,1 | 85,8 | 80,2 | 70,9 |
| 0,008 | 47 | 38,1 | 34,8 | 32,2 | 30,4 |

**SEC**: Standard curve from plates n°100 to 104 n=5 duplicate measurements

| Cref (ng/mL) | mFI | s | λ | CV % | λ % | ε | ε% |
|---|---|---|---|---|---|---|---|
| 16,400 | 9670,6 | 723,4 | 898,0 | 7,5 | 9,3 | 2008,1 | 20,8 |
| 8,200 | 8820,7 | 872,4 | 1083,1 | 9,9 | 12,3 | 2421,8 | 27,5 |
| 4,100 | 6957,8 | 829,3 | 1029,6 | 11,9 | 14,8 | 2302,2 | 33,1 |
| 2,050 | 4765,8 | 800,7 | 994,0 | 16,8 | 20,9 | 2222,6 | 46,6 |
| 1,020 | 2850,2 | 357,4 | 443,7 | 12,5 | 15,6 | 992,2 | 34,8 |
| 0,512 | 1462,7 | 188,2 | 233,6 | 12,9 | 16,0 | 522,4 | 35,7 |
| 0,256 | 708,4 | 131,9 | 163,8 | 18,6 | 23,1 | 366,2 | 51,7 |
| 0,128 | 333,8 | 39,3 | 48,8 | 11,8 | 14,6 | 109,0 | 32,7 |
| 0,064 | 177,9 | 14,0 | 17,4 | 7,9 | 9,8 | 39,0 | 21,9 |
| 0,032 | 83,3 | 7,6 | 9,5 | 9,1 | 11,4 | 21,1 | 25,4 |
| 0,016 | 40,4 | 6,0 | 7,5 | 14,9 | 18,5 | 16,7 | 41,4 |
| 0,008 | 18,1 | 4,7 | 5,9 | 26,1 | 32,4 | 13,1 | 72,4 |

| | Plate 100 | Plate 101 | Plate 102 | Plate 103 | Plate 104 |
|---|---|---|---|---|---|
| Cref (ng/mL) | FI-bkg | FI-bkg | FI-bkg | FI-bkg | FI-bkg |
| 16,400 | 10282,4 | 9933,4 | 10312,4 | 9121,8 | 8702,8 |
| 8,200 | 9085,2 | 9144,4 | 9573,2 | 8988,3 | 7312,3 |
| 4,100 | 7575,9 | 6998,6 | 7714,9 | 6879,1 | 5620,6 |
| 2,050 | 5277,4 | 4912,4 | 5497,7 | 4695,1 | 3446,6 |
| 1,020 | 3056,4 | 2848,1 | 3212,4 | 2864,6 | 2269,6 |
| 0,512 | 1577,2 | 1275,4 | 1687,7 | 1511,6 | 1261,6 |
| 0,256 | 785,2 | 668,9 | 851,7 | 730,6 | 505,6 |
| 0,128 | 374,4 | 295,4 | 376,2 | 322,6 | 300,3 |
| 0,064 | 187,4 | 174,9 | 189,4 | 182,8 | 154,8 |
| 0,032 | 84,4 | 74,1 | 94,7 | 83,8 | 79,3 |
| 0,016 | 37,2 | 32,9 | 47,4 | 45,6 | 38,8 |
| 0,008 | 22,7 | 15,9 | 12,9 | 23,6 | 15,6 |

FIGURE 3 NEXT

**SED**: Standard curve from plates n°100 to 104 n=5 duplicate measurements

| Cref (ng/mL) | mFI | s | λ | CV % | λ % | ε | ε% |
|---|---|---|---|---|---|---|---|
| 16,400 | 5780,4 | 626,8 | 778,1 | 10,8 | 13,5 | 1739,9 | 30,1 |
| 8,200 | 5610,8 | 597,4 | 741,7 | 10,6 | 13,2 | 1658,5 | 29,6 |
| 4,100 | 4497,9 | 672,3 | 834,6 | 14,9 | 18,6 | 1866,3 | 41,5 |
| 2,050 | 2951,3 | 512,4 | 636,1 | 17,4 | 21,6 | 1422,3 | 48,2 |
| 1,020 | 1775,5 | 309,8 | 384,6 | 17,4 | 21,7 | 860,0 | 48,4 |
| 0,512 | 803,6 | 138,5 | 172,0 | 17,2 | 21,4 | 384,5 | 47,8 |
| 0,256 | 367,8 | 53,3 | 66,2 | 14,5 | 18,0 | 148,0 | 40,2 |
| 0,128 | 170,4 | 22,2 | 27,6 | 13,1 | 16,2 | 61,7 | 36,2 |
| 0,064 | 90,3 | 7,6 | 9,4 | 8,4 | 10,5 | 21,1 | 23,4 |
| 0,032 | 40,4 | 3,9 | 4,9 | 9,7 | 12,1 | 10,9 | 27,1 |
| 0,016 | 18,7 | 5,3 | 6,6 | 28,4 | 35,2 | 14,7 | 78,8 |
| 0,008 | 7,6 | 7,8 | 9,7 | 102,7 | 127,6 | 21,7 | 285,2 |

| | Plate 100 | Plate 101 | Plate 102 | Plate 103 | Plate 104 |
|---|---|---|---|---|---|
| Cref (ng/mL) | FI-bkg | FI-bkg | FI-bkg | FI-bkg | FI-bkg |
| 16,400 | 6015,5 | 6099,9 | 6529 | 5176,4 | 5081,3 |
| 8,200 | 5702 | 5818,6 | 6315 | 5543,2 | 4675 |
| 4,100 | 5047 | 4442,4 | 5155,3 | 4374,7 | 3470,3 |
| 2,050 | 3243,8 | 2958,4 | 3539,8 | 2841,7 | 2172,8 |
| 1,020 | 1947,5 | 1745,4 | 2139,5 | 1738,2 | 1307 |
| 0,512 | 875,8 | 684,4 | 982,5 | 828,7 | 646,5 |
| 0,256 | 406,8 | 363,4 | 423,5 | 358,9 | 286,3 |
| 0,128 | 196 | 153,6 | 192,3 | 161,4 | 148,5 |
| 0,064 | 97,3 | 86,6 | 97,5 | 90,4 | 79,5 |
| 0,032 | 43,5 | 35,4 | 39,5 | 45,2 | 38,5 |
| 0,016 | 15 | 11,6 | 22,8 | 24,2 | 20 |
| 0,008 | 9,3 | 2,1 | -0,5 | 19,7 | 7,5 |

**SEE**: Standard curve from plates n°100 to 104 n=5 duplicate measurements

| Cref (ng/mL) | mFI | s | λ | CV % | λ % | ε | ε% |
|---|---|---|---|---|---|---|---|
| 16,400 | 8580,6 | 1100,0 | 1365,6 | 12,8 | 15,9 | 3053,5 | 35,6 |
| 8,200 | 7764,9 | 1165,8 | 1447,3 | 15,0 | 18,6 | 3236,2 | 41,7 |
| 4,100 | 6092,7 | 1019,6 | 1265,8 | 16,7 | 20,8 | 2830,5 | 46,5 |
| 2,050 | 4233,1 | 835,1 | 1036,7 | 19,7 | 24,5 | 2318,1 | 54,8 |
| 1,020 | 2393,7 | 467,7 | 580,6 | 19,5 | 24,3 | 1298,3 | 54,2 |
| 0,512 | 1154,4 | 289,4 | 359,3 | 25,1 | 31,1 | 803,4 | 69,6 |
| 0,256 | 595,0 | 140,5 | 174,4 | 23,6 | 29,3 | 390,0 | 65,6 |
| 0,128 | 280,9 | 55,3 | 68,6 | 19,7 | 24,4 | 153,4 | 54,6 |
| 0,064 | 149,4 | 30,6 | 38,0 | 20,5 | 25,4 | 85,0 | 56,9 |
| 0,032 | 74,9 | 10,1 | 12,5 | 13,4 | 16,7 | 27,9 | 37,3 |
| 0,016 | 33,8 | 5,8 | 7,2 | 17,2 | 21,3 | 16,1 | 47,7 |
| 0,008 | 16,1 | 4,3 | 5,4 | 26,9 | 33,4 | 12,0 | 74,7 |

FIGURE 3 NEXT

| | Plate 100 | Plate 101 | Plate 102 | Plate 103 | Plate 104 |
|---|---|---|---|---|---|
| Cref (ng/mL) | FI-bkg | FI-bkg | FI-bkg | FI-bkg | FI-bkg |
| 16,400 | 9209,2 | 8769,1 | 9901,4 | 7947,3 | 7076,1 |
| 8,200 | 8182,9 | 8409,6 | 8913,2 | 7395,1 | 5923,6 |
| 4,100 | 6583,2 | 6060,1 | 7352,2 | 5888,3 | 4579,8 |
| 2,050 | 4877,2 | 4147,4 | 5098,2 | 4071,6 | 2971,1 |
| 1,020 | 2689,2 | 2410,9 | 2983,7 | 2068,3 | 1816,3 |
| 0,512 | 1328,2 | 1004,9 | 1557,2 | 1061,6 | 820,3 |
| 0,256 | 674,4 | 607,9 | 759,9 | 544,3 | 388,3 |
| 0,128 | 329,4 | 244,6 | 349,7 | 256,3 | 224,3 |
| 0,064 | 174,2 | 150,4 | 174,9 | 148,1 | 99,6 |
| 0,032 | 77,7 | 72,6 | 89,9 | 72,1 | 62,3 |
| 0,016 | 31,9 | 27,4 | 41,7 | 37,8 | 30,3 |
| 0,008 | 19,7 | 8,6 | 16,9 | 17,1 | 18,1 |

**SEG**: Standard curve from plates n°100 to 104 n=5 duplicate measurements

| Cref (ng/mL) | mFI | s | λ | CV % | λ % | ε | ε% |
|---|---|---|---|---|---|---|---|
| 16,400 | 7603,4 | 758,1 | 941,2 | 10,0 | 12,4 | 2104,5 | 27,7 |
| 8,200 | 6399,1 | 760,7 | 944,4 | 11,9 | 14,8 | 2111,7 | 33,0 |
| 4,100 | 4401,1 | 669,7 | 831,4 | 15,2 | 18,9 | 1859,1 | 42,2 |
| 2,050 | 2604,8 | 502,0 | 623,2 | 19,3 | 23,9 | 1393,6 | 53,5 |
| 1,020 | 1409,4 | 269,6 | 334,7 | 19,1 | 23,7 | 748,3 | 53,1 |
| 0,512 | 644,8 | 106,4 | 132,0 | 16,5 | 20,5 | 295,2 | 45,8 |
| 0,256 | 314,2 | 50,7 | 63,0 | 16,1 | 20,0 | 140,8 | 44,8 |
| 0,128 | 149,2 | 22,0 | 27,3 | 14,7 | 18,3 | 60,9 | 40,8 |
| 0,064 | 75,5 | 10,1 | 12,5 | 13,3 | 16,6 | 27,9 | 37,0 |
| 0,032 | 36,4 | 7,3 | 9,1 | 20,1 | 24,9 | 20,2 | 55,7 |
| 0,016 | 18,1 | 5,6 | 7,0 | 31,0 | 38,5 | 15,6 | 86,1 |
| 0,008 | 7,6 | 11,2 | 13,9 | 148,0 | 183,7 | 31,1 | 410,8 |

| | Plate 100 | Plate 101 | Plate 102 | Plate 103 | Plate 104 |
|---|---|---|---|---|---|
| Cref (ng/mL) | FI-bkg | FI-bkg | FI-bkg | FI-bkg | FI-bkg |
| 16,400 | 8177,3 | 7609,7 | 8482,4 | 7131,2 | 6616,5 |
| 8,200 | 6422,1 | 6370,7 | 7246,7 | 6765,4 | 5190,5 |
| 4,100 | 4864,8 | 4330,7 | 5148,7 | 4257,7 | 3403,8 |
| 2,050 | 2822,3 | 2597,2 | 3109,9 | 2721,7 | 1773 |
| 1,020 | 1547,1 | 1363,2 | 1718,9 | 1424,9 | 992,8 |
| 0,512 | 703,6 | 559,7 | 777,2 | 667,2 | 516,3 |
| 0,256 | 344,3 | 297,2 | 365,9 | 328,4 | 235,3 |
| 0,128 | 169,1 | 130,2 | 164,4 | 161,2 | 121 |
| 0,064 | 83,3 | 70,4 | 73,9 | 87,4 | 62,3 |
| 0,032 | 39,8 | 29,4 | 28,7 | 45,9 | 38 |
| 0,016 | 14,6 | 15,2 | 13,2 | 26,7 | 20,8 |
| 0,008 | 9,8 | 1,7 | -6,1 | 24,2 | 8,3 |

FIGURE 3 NEXT

**SEH**: Standard curve from plates n°100 to 104 n=5 duplicate measurements

| Cref (ng/mL) | mFI | s | λ | CV % | λ % | ε | ε% |
|---|---|---|---|---|---|---|---|
| 16,400 | 1445,2 | 173,4 | 215,3 | 12,0 | 14,9 | 481,3 | 33,3 |
| 8,200 | 1351,7 | 207,2 | 257,3 | 15,3 | 19,0 | 575,2 | 42,6 |
| 4,100 | 1086,8 | 176,8 | 219,5 | 16,3 | 20,2 | 490,8 | 45,2 |
| 2,050 | 838,4 | 164,5 | 204,2 | 19,6 | 24,4 | 456,6 | 54,5 |
| 1,020 | 602,3 | 124,8 | 154,9 | 20,7 | 25,7 | 346,4 | 57,5 |
| 0,512 | 325,9 | 67,1 | 83,3 | 20,6 | 25,6 | 186,3 | 57,2 |
| 0,256 | 169,5 | 44,5 | 55,2 | 26,3 | 32,6 | 123,5 | 72,9 |
| 0,128 | 84,8 | 14,8 | 18,4 | 17,5 | 21,7 | 41,1 | 48,5 |
| 0,064 | 48,6 | 6,9 | 8,6 | 14,3 | 17,7 | 19,3 | 39,6 |
| 0,032 | 20,4 | 3,0 | 3,7 | 14,6 | 18,2 | 8,3 | 40,6 |
| 0,016 | 6,4 | 5,3 | 6,5 | 81,7 | 101,4 | 14,6 | 226,7 |
| 0,008 | 1,7 | 4,8 | 5,9 | 286,7 | 355,9 | 13,2 | 795,8 |

| | Plate 100 | Plate 101 | Plate 102 | Plate 103 | Plate 104 |
|---|---|---|---|---|---|
| Cref (ng/mL) | FI-bkg | FI-bkg | FI-bkg | FI-bkg | FI-bkg |
| 16,400 | 1508,8 | 1474,2 | 1685,9 | 1310 | 1247,3 |
| 8,200 | 1368,1 | 1487,2 | 1570,4 | 1301,5 | 1031,1 |
| 4,100 | 1130,8 | 1179,2 | 1293,2 | 996,3 | 834,3 |
| 2,050 | 861,8 | 886,7 | 1028,4 | 839,8 | 575,3 |
| 1,020 | 645,3 | 625,9 | 755,7 | 570,5 | 414,3 |
| 0,512 | 365,6 | 311,7 | 404,7 | 321,8 | 225,8 |
| 0,256 | 194,8 | 166,2 | 219,2 | 166,8 | 100,3 |
| 0,128 | 95,3 | 81,2 | 103,2 | 78,8 | 65,3 |
| 0,064 | 44,8 | 43,7 | 60,4 | 49,5 | 44,8 |
| 0,032 | 21,1 | 15,2 | 20,9 | 22,8 | 21,8 |
| 0,016 | 7,3 | -0,1 | 10,4 | 12,3 | 2,3 |
| 0,008 | 5,3 | -5,3 | -0,8 | 2,8 | 6,3 |

**SEI**: Standard curve from plates n°100 to 104 n=5 duplicate measurements

| Cref (ng/mL) | mFI | s | λ | CV % | λ % | ε | ε% |
|---|---|---|---|---|---|---|---|
| 16,400 | 1605,3 | 105,6 | 131,2 | 6,6 | 8,2 | 293,3 | 18,3 |
| 8,200 | 1354,8 | 178,4 | 221,5 | 13,2 | 16,3 | 495,3 | 36,6 |
| 4,100 | 944,1 | 80,1 | 99,4 | 8,5 | 10,5 | 222,2 | 23,5 |
| 2,050 | 619,2 | 93,2 | 115,7 | 15,1 | 18,7 | 258,7 | 41,8 |
| 1,020 | 374,8 | 59,4 | 73,7 | 15,8 | 19,7 | 164,8 | 44,0 |
| 0,512 | 195,6 | 32,7 | 40,6 | 16,7 | 20,8 | 90,8 | 46,4 |
| 0,256 | 100,1 | 17,9 | 22,2 | 17,9 | 22,2 | 49,7 | 49,6 |
| 0,128 | 49,6 | 7,3 | 9,1 | 14,8 | 18,4 | 20,4 | 41,1 |
| 0,064 | 26,7 | 3,3 | 4,1 | 12,5 | 15,5 | 9,3 | 34,6 |
| 0,032 | 10,4 | 2,1 | 2,6 | 19,9 | 24,8 | 5,8 | 55,3 |
| 0,016 | 4,5 | 4,1 | 5,1 | 90,3 | 112,1 | 11,4 | 250,6 |
| 0,008 | 0,4 | 4,5 | 5,6 | 1068,7 | 1326,8 | 12,5 | 2966,7 |

FIGURE 3 NEXT

| | Plate 100 | Plate 101 | Plate 102 | Plate 103 | Plate 104 |
|---|---|---|---|---|---|
| Cref (ng/mL) | FI-bkg | FI-bkg | FI-bkg | FI-bkg | FI-bkg |
| 16,400 | 1634,8 | 1682,3 | 1720,3 | 1500,2 | 1488,8 |
| 8,200 | 1342,1 | 1404 | 1558,1 | 1400,2 | 1069,6 |
| 4,100 | 974,3 | 1018,5 | 1003,3 | 895,7 | 828,6 |
| 2,050 | 636,6 | 637,5 | 722,3 | 633,2 | 466,6 |
| 1,020 | 403,1 | 360,8 | 448,3 | 374,9 | 287,1 |
| 0,512 | 214,8 | 178 | 230,6 | 206,4 | 148,1 |
| 0,256 | 116,1 | 96 | 114,3 | 102,2 | 71,8 |
| 0,128 | 57,3 | 43,3 | 56,8 | 48,9 | 41,6 |
| 0,064 | 28,3 | 26,8 | 29,8 | 27,7 | 21,1 |
| 0,032 | 10,3 | 8 | 9,6 | 13,7 | 10,6 |
| 0,016 | 5,6 | -1 | 2,8 | 10,2 | 5,1 |
| 0,008 | 2,1 | -2,8 | -5,7 | 3,7 | 4,8 |

- Cref : reference concentration
- mFI : average measures (Plate 100 to 104)
- s : deviation
- λ : confidence interval on the average: $\lambda = t(\nu;1-\alpha/2).s/\sqrt{n}$ with $\nu = n-1$ and $\alpha = 5\%$ and t = 2,776
- CV = s/mFI*100
- λ %: relative confidence interval on the average: $\lambda\% = t(\nu;1-\alpha/2).CV/\sqrt{n}$ with $\nu = n-1$ and $\alpha = 5\%$ and t = 2,776
- ε : confidence interval on the measurement: $\varepsilon = t(\nu;1-\alpha/2).s$ with $\nu = n-1$ and $\alpha = 5\%$ and t = 2,776
- ε %: relative confidence interval on the measurement: $\varepsilon\% = t(\nu;1-\alpha/2).s$ with $\nu = n-1$ and $\alpha = 5\%$ and t = 2,776

FIGURE 3 END

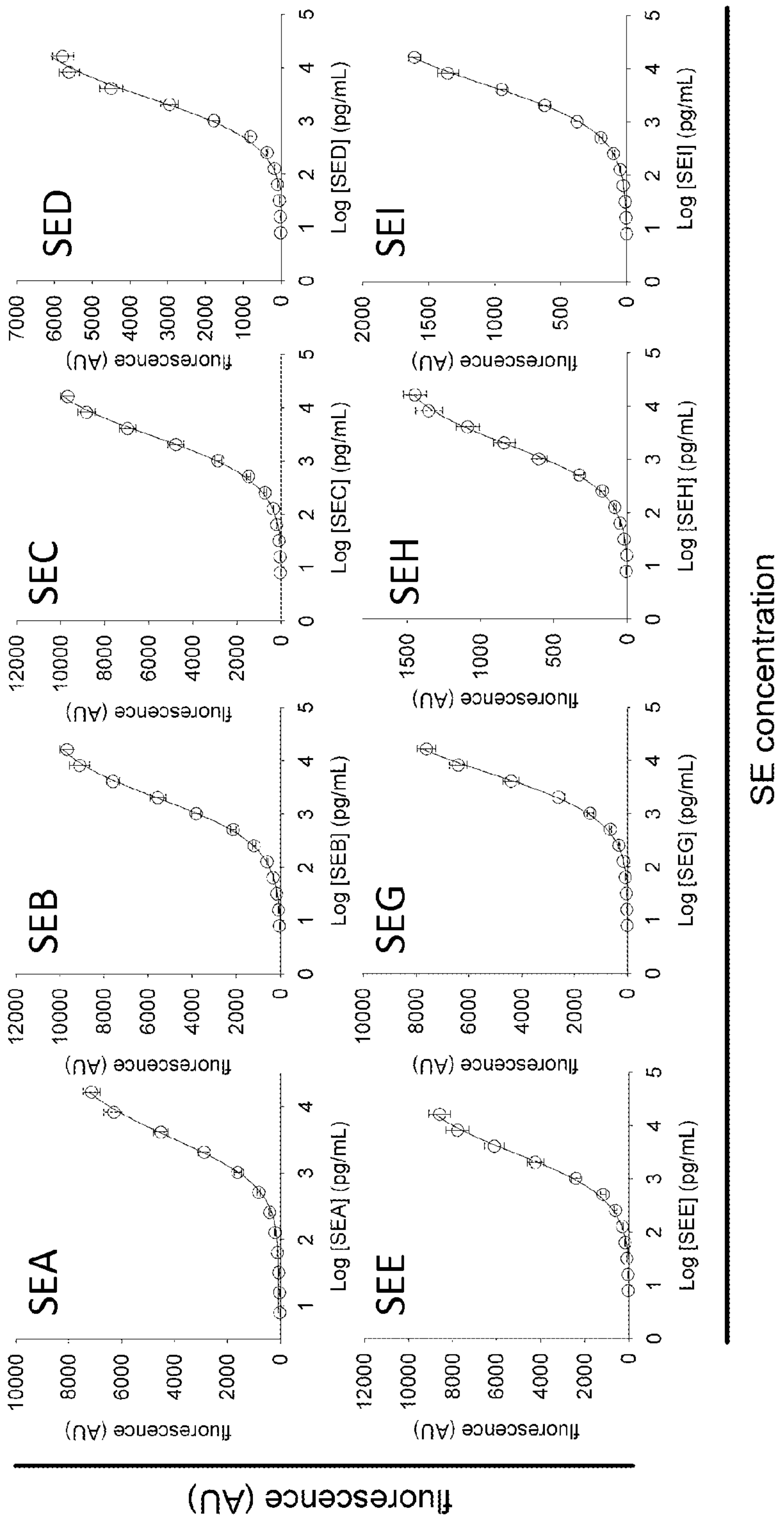
fluorescence (AU)
SE concentration
SEA
SEB
SEC
SED
SEE
SEG
SEH
SEI
Log [SEA] (pg/mL)
Log [SEB] (pg/mL)
Log [SEC] (pg/mL)
Log [SED] (pg/mL)
Log [SEE] (pg/mL)
Log [SEG] (pg/mL)
Log [SEH] (pg/mL)
Log [SEI] (pg/mL)

FIGURE 4

| LQI (pg/mL) | SEA | SEB | SEC1 | SED | SEE | SEG | SEH | SEI |
|---|---|---|---|---|---|---|---|---|
| m | 29 | 7 | 11 | 24 | 12 | 43 | 76 | 75 |
| s | 14 | 3 | 4 | 10 | 8 | 18 | 55 | 55 |
| CV % | 49 | 47,9 | 31,7 | 40,0 | 66,8 | 41,8 | 71,9 | 73,8 |
| Plate 100 | 25 | 10 | 13 | 11 | 8 | 32 | 66 | 18 |
| Plate 101 | 25 | 6 | 12 | 32 | 12 | 53 | 95 | 84 |
| Plate 102 | 36 | 5 | 13 | 26 | 6 | 46 | 26 | 57 |
| Plate 103 | 10 | 3 | 5 | 17 | 8 | 19 | 33 | 50 |
| Plate 104 | 47 | 10 | 13 | 34 | 26 | 66 | 161 | 164 |

| LD (pg/mL) | SEA | SEB | SEC1 | SED | SEE | SEG | SEH | SEI |
|---|---|---|---|---|---|---|---|---|
| m | 10 | 2 | 4 | 9 | 4 | 15 | 26 | 25 |
| s | 4,6 | 1,1 | 1,2 | 3,4 | 2,9 | 6,1 | 18,2 | 18,9 |
| CV % | 47,5 | 51,5 | 30,3 | 38,9 | 70,9 | 40,1 | 70,6 | 76,9 |
| Plate 100 | 8 | 4 | 4 | 4 | 3 | 11 | 25 | 6 |
| Plate 101 | 8 | 2 | 4 | 11 | 4 | 19 | 29 | 26 |
| Plate 102 | 13 | 2 | 5 | 10 | 2 | 17 | 9 | 18 |
| Plate 103 | 3 | 1 | 2 | 7 | 2 | 7 | 11 | 18 |
| Plate 104 | 15 | 3 | 4 | 12 | 9 | 22 | 54 | 56 |

- n =5
- Cref : reference concentration
- m : average
- s : deviation
- CV = s/m*100
- Definition of LD and LQI : Soejima, al, International Journal of Food Microbiology (2004) 93 :185-194
- LQI = d + 10*sblc
- LD = d + 3,3*sblc
- d = $FI_{c \to 0}$

FIGURE 5

**SEA**: Standard curve of plates n°100 to 104 n=5, Observed concentrations

| | Plate 100 | Plate 101 | Plate 102 | Plate 103 | Plate 104 |
|---|---|---|---|---|---|
| Cref (pg/mL) | Cobs (pg/mL) | Cobs (pg/mL) | Cobs (pg/mL) | Cobs (pg/mL) | Cobs (pg/mL) |
| 16400 | 16145,05 | 17054,16 | 14511,93 | 14433,31 | 16983,14 |
| 8200 | 8251,40 | 8216,17 | 9668,69 | 9674,52 | 7927,77 |
| 4100 | 4118,34 | 3887,31 | 3865,09 | 3859,48 | 4187,78 |
| 2050 | 2042,34 | 2147,13 | 2009,31 | 2079,74 | 1993,71 |
| 1020 | 1018,42 | 1062,16 | 1087,91 | 1002,95 | 1063,2 |
| 512 | 514,69 | 486,76 | 512,05 | 530,67 | 536,01 |
| 256 | 253,28 | 255,72 | 249,2 | 256,02 | 233,95 |
| 128 | 128,58 | 119,66 | 122,08 | 121,83 | 127,63 |
| 64 | 67,31 | 74,87 | 65,61 | 70,29 | 62,52 |
| 32 | 30,83 | 32,23 | 32,89 | 30,13 | 36,54 |
| 16 | 14,29 | 14,88 | 19,15 | 15,32 | 18 |
| 8 | 9,35 | 7,71 | 6,15 | 8,67 | 6,18 |

| Cref (pg/mL) | mCobs (pg/mL) |
|---|---|
| 16400 | 15825,52 |
| 8200 | 8747,71 |
| 4100 | 3983,60 |
| 2050 | 2054,45 |
| 1020 | 1046,93 |
| 512 | 516,04 |
| 256 | 249,63 |
| 128 | 123,96 |
| 64 | 68,12 |
| 32 | 32,52 |
| 16 | 16,33 |
| 8 | 7,61 |

FIGURE 6

**SEA**: Standard curve of plates n°100 to 104 n=5, Observed concentration NEXT

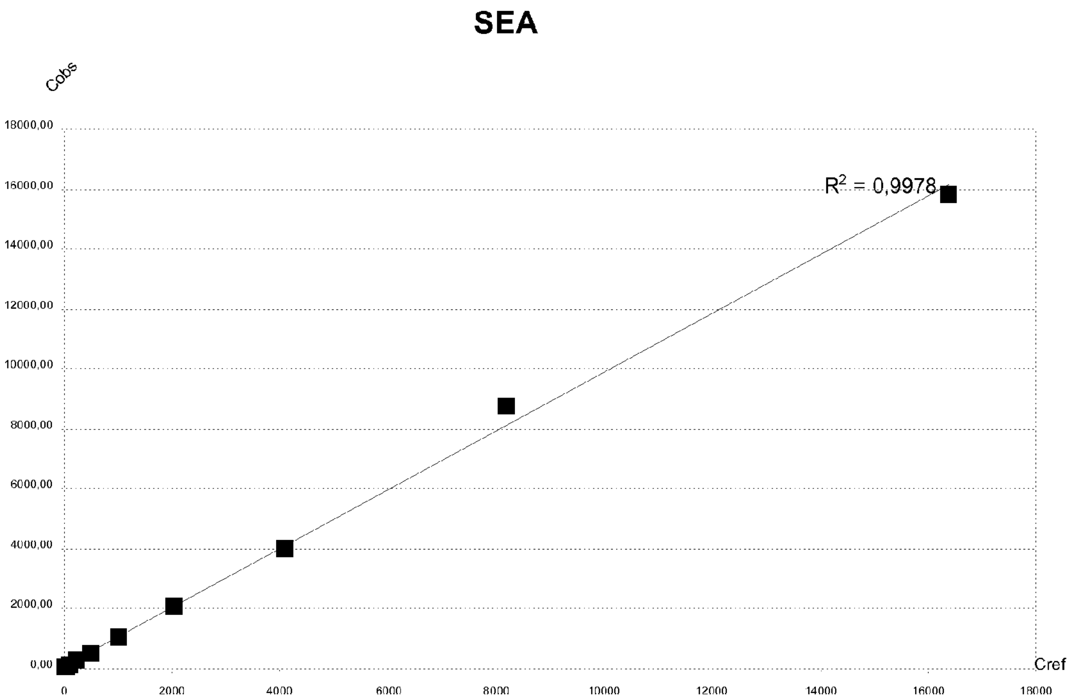


FIGURE 6 NEXT

**SEB**: Standard curve of plates n°100 to 104 n=5, Observed concentration

| | Plate 100 | Plate 101 | Plate 102 | Plate 103 | Plate 104 |
|---|---|---|---|---|---|
| Cref (pg/mL) | Cobs (pg/mL) | Cobs (pg/mL) | Cobs (pg/mL) | Cobs (pg/mL) | Cobs (pg/mL) |
| 16400 | 14434,68 | 13498,95 | 15277,65 | 13133,16 | 19024,78 |
| 8200 | 9077,85 | 10498,92 | 8880,14 | 10511,42 | 7082,44 |
| 4100 | 4005,4 | 3858,06 | 4081,68 | 4036,76 | 4810,16 |
| 2050 | 2132,33 | 2002,36 | 1962,21 | 1933,29 | 1808,2 |
| 1020 | 1022,85 | 1109,4 | 1067,89 | 1057,49 | 1110,15 |
| 512 | 495,08 | 464,55 | 508,79 | 524,42 | 547,24 |
| 256 | 253,07 | 289,43 | 256,61 | 255,69 | 237,05 |
| 128 | 130,32 | 117,57 | 124,89 | 123,3 | 124,73 |
| 64 | 67,4 | 71,24 | 65 | 63,63 | 67,19 |
| 32 | 31,41 | 31,17 | 31,67 | 32,49 | 31,37 |
| 16 | 14,61 | 14,84 | 16,5 | 16,49 | 16,96 |
| 8 | 8,82 | 8,56 | 7,85 | 7,85 | 7,67 |

| Cref (pg/mL) | mCobs (pg/mL) |
|---|---|
| 16400 | 15073,84 |
| 8200 | 9210,15 |
| 4100 | 4158,41 |
| 2050 | 1967,68 |
| 1020 | 1073,56 |
| 512 | 508,02 |
| 256 | 258,37 |
| 128 | 124,16 |
| 64 | 66,89 |
| 32 | 31,62 |
| 16 | 15,88 |
| 8 | 8,15 |

FIGURE 6 NEXT

**SEB**: Standard curve of plates n°100 to 104 n=5, Observed concentration NEXT

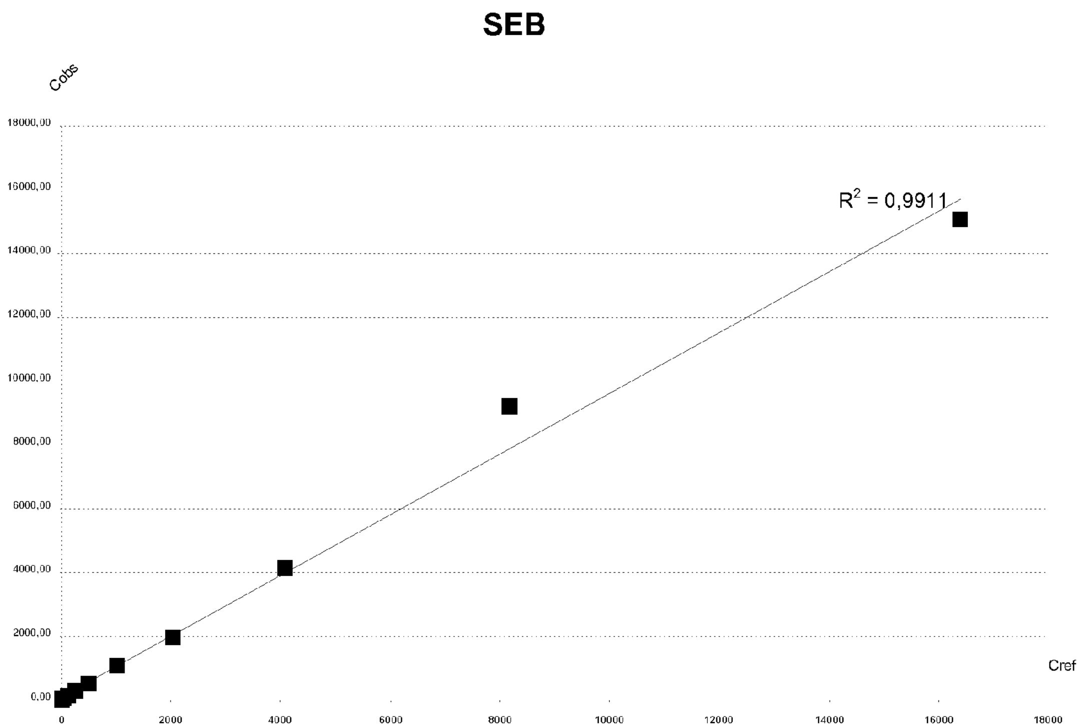


FIGURE 6 NEXT

**SEC**: Standard curve of plates n°100 to 104 n=5, Observed concentration

| | Plate 100 | Plate 101 | Plate 102 | Plate 103 | Plate 104 |
|---|---|---|---|---|---|
| Cref (pg/mL) | Cobs (pg/mL) | Cobs (pg/mL) | Cobs (pg/mL) | Cobs (pg/mL) | Cobs (pg/mL) |
| 16400 | 18477,22 | 17626,16 | 16806,55 | 12645,48 | 17141,74 |
| 8200 | 7535,65 | 8656,66 | 8544,83 | 11224,18 | 7967,88 |
| 4100 | 4150,68 | 3770,19 | 3910,99 | 3952,23 | 4184,84 |
| 2050 | 2080,72 | 2108,87 | 2070,32 | 1966,72 | 1904,51 |
| 1020 | 1020,97 | 1096,27 | 1032,84 | 1044,76 | 1118,73 |
| 512 | 504,95 | 482,35 | 517,53 | 527,76 | 574,5 |
| 256 | 256,07 | 258,56 | 262,15 | 258,16 | 219,23 |
| 128 | 127,66 | 119 | 119,58 | 118,05 | 128,79 |
| 64 | 67,04 | 72,6 | 62,81 | 68,43 | 66,06 |
| 32 | 31,8 | 32,47 | 33,37 | 31,72 | 33,97 |
| 16 | 14,49 | 15,27 | 18,33 | 16,72 | 16,92 |
| 8 | 8,83 | 7,88 | 7,02 | 7,61 | 7,2 |

| Cref (pg/mL) | mCobs (pg/mL) |
|---|---|
| 16400 | 16539,43 |
| 8200 | 8785,84 |
| 4100 | 3993,786 |
| 2050 | 2026,228 |
| 1020 | 1062,714 |
| 512 | 521,418 |
| 256 | 250,834 |
| 128 | 122,616 |
| 64 | 67,388 |
| 32 | 32,666 |
| 16 | 16,346 |
| 8 | 7,708 |

FIGURE 6 NEXT

**SEC**: Standard curve of plates n°100 to 104 n=5, Observed concentration NEXT

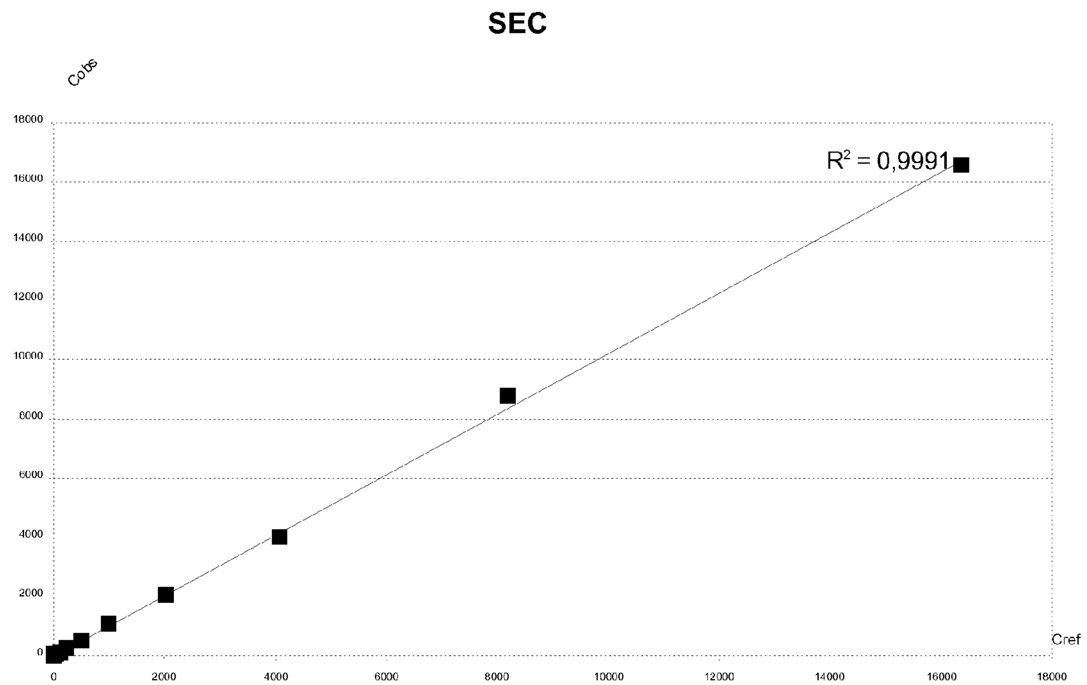


FIGURE 6 NEXT

**SED**: Standard curve of plates n°100 to 104 n=5, Observed concentration

| | Plate 100 | Plate 101 | Plate 102 | Plate 103 | Plate 104 |
|---|---|---|---|---|---|
| Cref (pg/mL) | Cobs (pg/mL) | Cobs (pg/mL) | Cobs (pg/mL) | Cobs (pg/mL) | Cobs (pg/mL) |
| 16400 | 17238,13 | 15434,5 | 15038,79 | 8818,87 | 14979,87 |
| 8200 | 7641,19 | 9417,97 | 9622,47 | 38789,77 | 9043,48 |
| 4100 | 4366,88 | 3811,32 | 3905,36 | 4160,74 | 3999,44 |
| 2050 | 1946,19 | 2054,71 | 1986,93 | 1891,35 | 1972,03 |
| 1020 | 1090,05 | 1145,15 | 1097,55 | 1071,2 | 1093,37 |
| 512 | 502,41 | 465,89 | 515,96 | 527,01 | 527,72 |
| 256 | 247,76 | 260,76 | 242,43 | 249,23 | 236,24 |
| 128 | 127,56 | 120,27 | 121,81 | 121,23 | 124,7 |
| 64 | 67,84 | 72,7 | 68,35 | 69,88 | 67,92 |
| 32 | 33,23 | 34,22 | 32,77 | 33,28 | 33,45 |
| 16 | 13,45 | 15,04 | 21,62 | 13,61 | 17,51 |
| 8 | 9,18 | 6,75 | 4,37 | 8,8 | 6,42 |

| Cref (pg/mL) | mCobs (pg/mL) |
|---|---|
| 16400 | 14302,032 |
| 8200 | 14902,976 |
| 4100 | 4048,748 |
| 2050 | 1970,242 |
| 1020 | 1099,464 |
| 512 | 507,798 |
| 256 | 247,284 |
| 128 | 123,114 |
| 64 | 69,338 |
| 32 | 33,39 |
| 16 | 16,246 |
| 8 | 7,104 |

FIGURE 6 NEXT

**SED**: Standard curve of plates n°100 to 104 n=5, Observed concentration NEXT

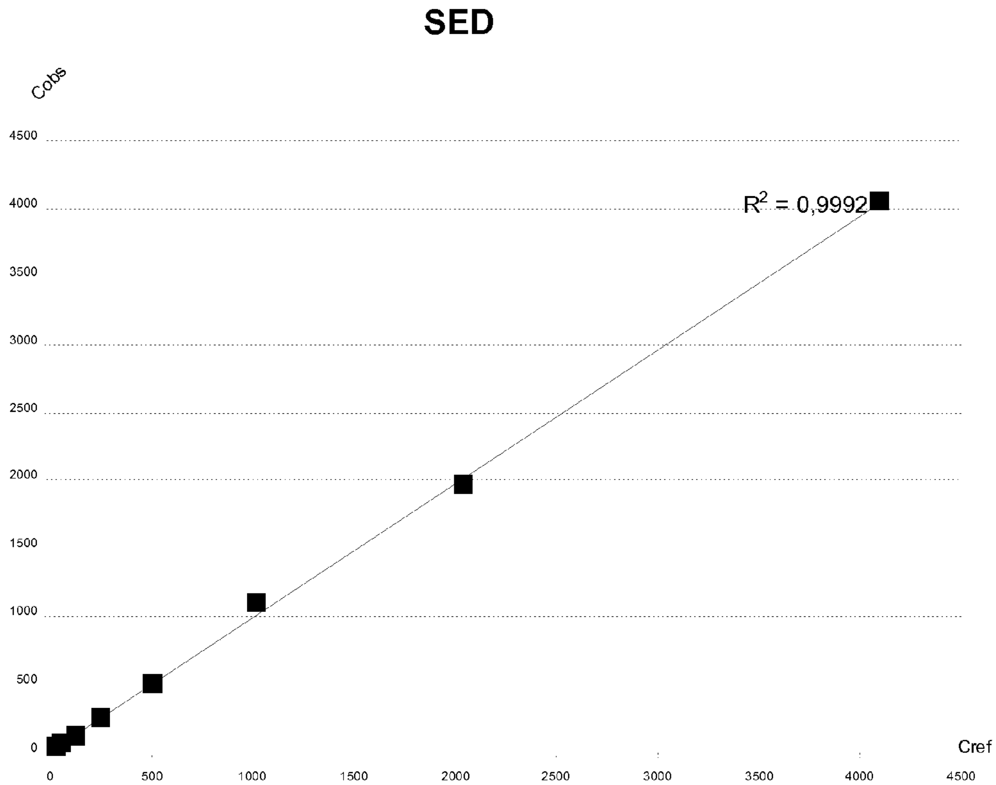


FIGURE 6 NEXT

**SEE**: Standard curve of plates n°100 to 104 n=5, Observed concentration

| | Plate 100 | Plate 101 | Plate 102 | Plate 103 | Plate 104 |
|---|---|---|---|---|---|
| Cref (pg/mL) | Cobs (pg/mL) | Cobs (pg/mL) | Cobs (pg/mL) | Cobs (pg/mL) | Cobs (pg/mL) |
| 16400 | 22929,03 | 14422,19 | 18862,26 | 24152,35 | 19601,35 |
| 8200 | 7603,82 | 9983,47 | 7780,92 | 8011,59 | 7552,1 |
| 4100 | 3804,42 | 3720,39 | 4051,11 | 3874,95 | 4011,62 |
| 2050 | 2205,19 | 2123,55 | 2051,98 | 2187,53 | 2042,87 |
| 1020 | 1033,91 | 1134,81 | 1035,17 | 1010,63 | 1131,62 |
| 512 | 492,61 | 458,49 | 518,66 | 506,01 | 493,23 |
| 256 | 253,11 | 277,04 | 256,03 | 256,31 | 235,68 |
| 128 | 127,23 | 112,98 | 122,02 | 119,33 | 137,51 |
| 64 | 69,38 | 70,66 | 63,49 | 68,33 | 60,78 |
| 32 | 32,31 | 35,85 | 34,06 | 32,79 | 36,99 |
| 16 | 14 | 15,64 | 16,7 | 16,91 | 15,74 |
| 8 | 8,92 | 7,29 | 7,39 | 7,37 | 7,17 |

| Cref (pg/mL) | mCobs (pg/mL) |
|---|---|
| 16400 | 19993,436 |
| 8200 | 8186,38 |
| 4100 | 3892,498 |
| 2050 | 2122,224 |
| 1020 | 1069,228 |
| 512 | 493,8 |
| 256 | 255,634 |
| 128 | 123,814 |
| 64 | 66,528 |
| 32 | 34,4 |
| 16 | 15,798 |
| 8 | 7,628 |

FIGURE 6 NEXT

**SEE**: Standard curve of plates n°100 to 104 n=5, Observed concentration NEXT

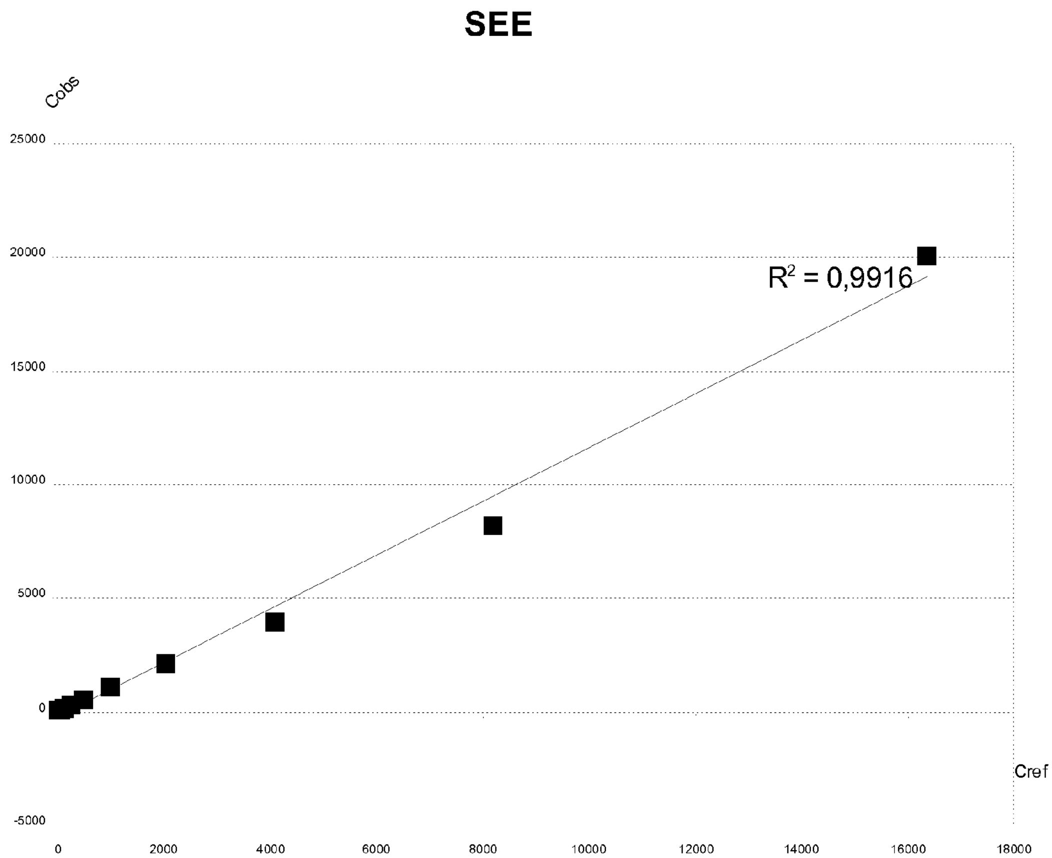


FIGURE 6 NEXT

**SEG**: Standard curve of plates n°100 to 104 n=5, Observed concentration

| | Plate 100 | Plate 101 | Plate 102 | Plate 103 | Plate 104 |
|---|---|---|---|---|---|
| Cref (pg/mL) | Cobs (pg/mL) | Cobs (pg/mL) | Cobs (pg/mL) | Cobs (pg/mL) | Cobs (pg/mL) |
| 16400 | 18499,72 | 16809,67 | 16346,45 | 13752,08 | 16341,36 |
| 8200 | 7396,51 | 8204,32 | 8342,77 | 10672,08 | 8162,28 |
| 4100 | 4269,1 | 3961,23 | 4018,73 | 3731,2 | 4193,68 |
| 2050 | 2041,35 | 2105,59 | 2031,1 | 2087,18 | 1952,04 |
| 1020 | 1063,72 | 1087,55 | 1073,55 | 1050,9 | 1058,66 |
| 512 | 491,14 | 468,97 | 500,67 | 502,89 | 541,96 |
| 256 | 250,6 | 262,04 | 252,43 | 255,51 | 243,7 |
| 128 | 129,41 | 123,69 | 125,44 | 127,3 | 123,47 |
| 64 | 67,2 | 71,12 | 64,85 | 67,25 | 61,91 |
| 32 | 33,84 | 32,83 | 32,49 | 31,16 | 36,57 |
| 16 | 13,13 | 18,62 | 20,71 | 13,03 | 18,59 |
| 8 | 8,97 | 3,96 | 4,85 | 10,53 | 5,61 |

| Cref (pg/mL) | mCobs (pg/mL) |
|---|---|
| 16400 | 16349,856 |
| 8200 | 8555,592 |
| 4100 | 4034,788 |
| 2050 | 2043,452 |
| 1020 | 1066,876 |
| 512 | 501,126 |
| 256 | 252,856 |
| 128 | 125,862 |
| 64 | 66,466 |
| 32 | 33,378 |
| 16 | 16,816 |
| 8 | 6,784 |

FIGURE 6 NEXT

**SEG**: Standard curve of plates n°100 to 104 n=5, Observed concentration NEXT

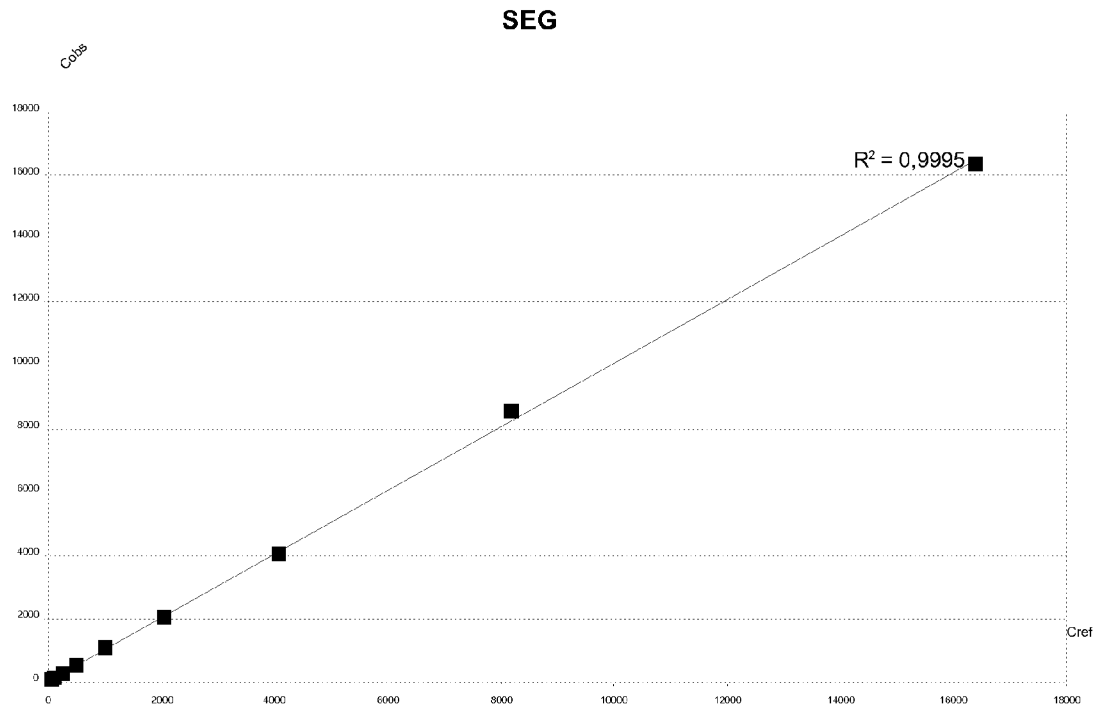


FIGURE 6 NEXT

**SEH**: Standard curve of plates n°100 to 104 n=5, Observed concentration NEXT

| | Plate 100 | Plate 101 | Plate 102 | Plate 103 | Plate 104 |
|---|---|---|---|---|---|
| Cref (pg/mL) | Cobs (pg/mL) | Cobs (pg/mL) | Cobs (pg/mL) | Cobs (pg/mL) | Cobs (pg/mL) |
| 16400 | 16761,52 | 12202,27 | 19017,26 | 15267,55 | 18072,89 |
| 8200 | 8606,84 | 13697,48 | 8940,63 | 13910,28 | 7589,59 |
| 4100 | 3920,22 | 3707,88 | 3596,08 | 3227,53 | 4119,79 |
| 2050 | 1928,84 | 1956,87 | 1994,59 | 2121,94 | 1938,16 |
| 1020 | 1120,75 | 1141,61 | 1145,73 | 1088,97 | 1156,28 |
| 512 | 504,46 | 488,71 | 497,39 | 518,85 | 523,78 |
| 256 | 251,39 | 250,28 | 252,95 | 250,07 | 210,29 |
| 128 | 126,95 | 124,13 | 119,64 | 115,1 | 133,46 |
| 64 | 65,37 | 71,63 | 72,91 | 72,25 | 89,93 |
| 32 | 34,21 | 33,44 | 30,11 | 33,71 | 41,69 |
| 16 | 12,9 | 13,97 | 18,61 | 18,71 | 0 |
| 8 | 9,08 | 7,56 | 6,01 | 5,16 | 8,2 |

| Cref (pg/mL) | mCobs (pg/mL) |
|---|---|
| 16400 | 16264,298 |
| 8200 | 10548,964 |
| 4100 | 3714,3 |
| 2050 | 1988,08 |
| 1020 | 1130,668 |
| 512 | 506,638 |
| 256 | 242,996 |
| 128 | 123,856 |
| 64 | 74,418 |
| 32 | 34,632 |
| 16 | 12,838 |
| 8 | 7,202 |

FIGURE 6 NEXT

**SEH**: Standard curve of plates n°100 to 104 n=5, Observed concentration

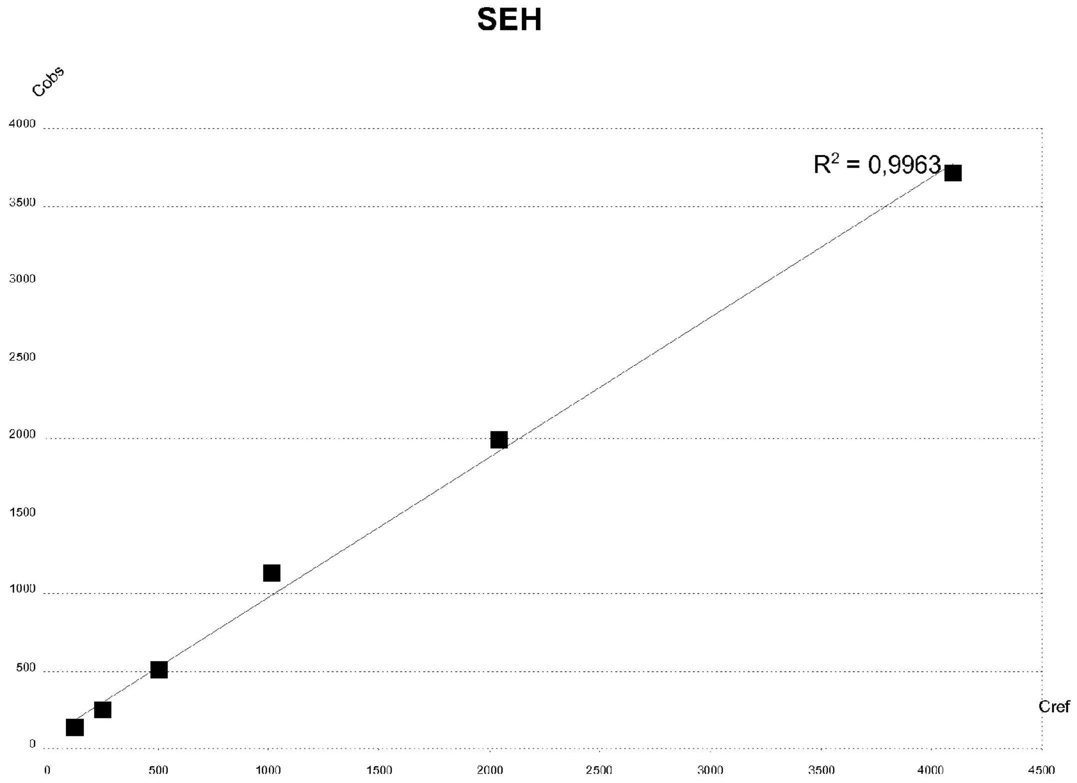


FIGURE 6 NEXT

**SEI**: Standard curve of plates n°100 to 104 n=5, Observed concentration

| | Plate 100 | Plate 101 | Plate 102 | Plate 103 | Plate 104 |
|---|---|---|---|---|---|
| Cref (pg/mL) | Cobs (pg/mL) | Cobs (pg/mL) | Cobs (pg/mL) | Cobs (pg/mL) | Cobs (pg/mL) |
| 16400 | 15918,57 | 16877,97 | 15062,2 | 14378,83 | 17367,45 |
| 8200 | 8516,85 | 8028,05 | 10167,64 | 10909,12 | 7375,74 |
| 4100 | 4107,43 | 4071 | 3566,19 | 3620,67 | 4559,57 |
| 2050 | 1987,43 | 2072,89 | 2097,19 | 2065,22 | 1946,62 |
| 1020 | 1054,9 | 1052,64 | 1105,61 | 1039,98 | 1067,88 |
| 512 | 499,21 | 488,87 | 508,99 | 529,02 | 510,46 |
| 256 | 259,08 | 258,85 | 242,22 | 253,5 | 239,37 |
| 128 | 128,92 | 119,56 | 122,69 | 120,1 | 136,59 |
| 64 | 66,78 | 77,98 | 69,8 | 67,01 | 67,38 |
| 32 | 27,78 | 32,61 | 31,96 | 31,29 | 31,36 |
| 16 | 17,08 | 12,11 | 19,86 | 22,11 | 11,77 |
| 8 | 8,84 | 8,31 | 5,28 | 4,23 | 10,85 |

| Cref (pg/mL) | mCobs (pg/mL) |
|---|---|
| 16400 | 15921,00 |
| 8200 | 8999,48 |
| 4100 | 3984,97 |
| 2050 | 2033,87 |
| 1020 | 1064,20 |
| 512 | 507,31 |
| 256 | 250,60 |
| 128 | 125,57 |
| 64 | 69,79 |
| 32 | 31,00 |
| 16 | 16,59 |
| 8 | 7,50 |

FIGURE 6 NEXT

**SEI**: Standard curve of plates n°100 to 104 n=5, Observed concentration NEXT

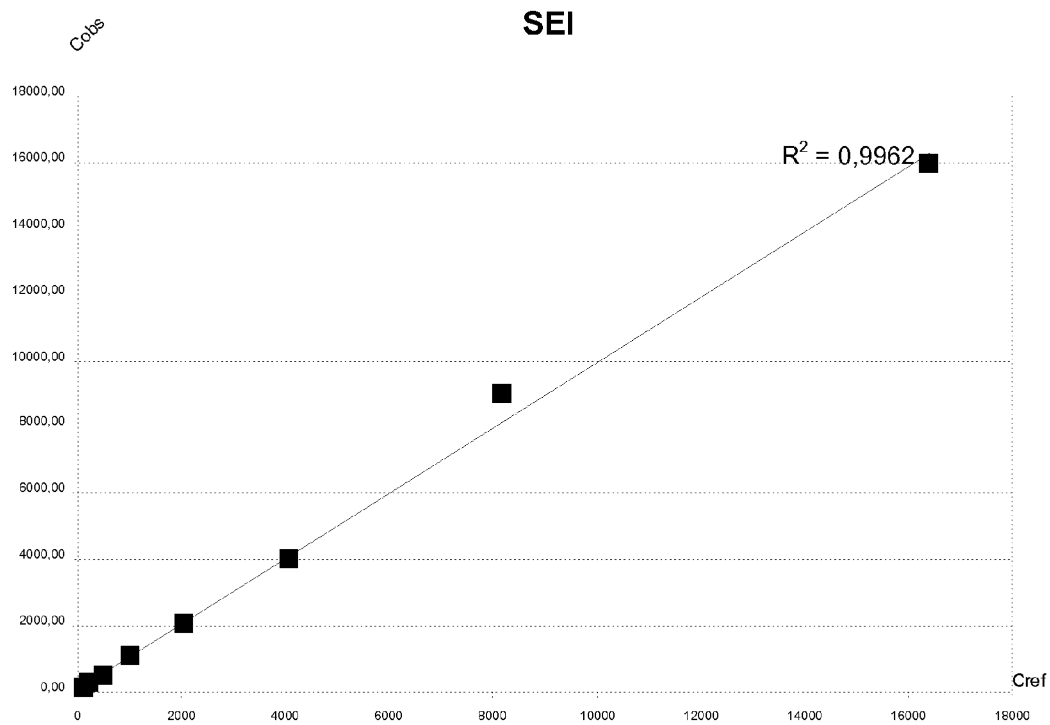


Cref: reference concentration
mCobs: average of reference concentration (plate n°100 to 104)

FIGURE 6 END

**Synthesis**: Precipitation of casein and delipidation - plates 101 to 104

| | | Cref en pg/mL | | | | |
|---|---|---|---|---|---|---|
| | | **573,25** | **287,5** | **144,5** | **72** | mbiais % |
| SEA | mCobs pg/mL | 362,95 | 177,35 | 104,96 | 52,78 | -32,3 |
| | biais % | -36,7 | -38,3 | -27,4 | -26,7 | |
| | CV% | 30,8 | 24,5 | 21,7 | 21,6 | |
| SEB | mCobs pg/mL | 400,00 | 192,61 | 101,89 | 51,81 | -30,2 |
| | biais % | -30,2 | -33,0 | -29,5 | -28,0 | |
| | CV% | 37,8 | 34,9 | 25,9 | 23,4 | |
| SEC | mCobs pg/mL | 225,09 | 108,38 | 52,62 | 27,55 | -62,1 |
| | biais % | -60,7 | -62,3 | -63,6 | -61,7 | |
| | CV% | 47,0 | 46,4 | 34,9 | 28,0 | |
| SED | mCobs pg/mL | 300,84 | 140,45 | 85,16 | 44,12 | -44,6 |
| | biais % | -47,5 | -51,1 | -41,1 | -38,7 | |
| | CV% | 24,3 | 32,0 | 14,2 | 10,0 | |
| SEE | mCobs pg/mL | 291,54 | 148,21 | 88,14 | 43,79 | -43,9 |
| | biais % | -49,1 | -48,5 | -39,0 | -39,2 | |
| | CV% | 39,3 | 30,9 | 26,7 | 23,3 | |
| SEG | mCobs pg/mL | 324,52 | 165,73 | 95,28 | 49,45 | -37,8 |
| | biais % | -43,4 | -42,4 | -34,1 | -31,3 | |
| | CV% | 39,6 | 36,8 | 20,5 | 11,9 | |
| SEH | mCobs pg/mL | 419,86 | 199,89 | 120,12 | 64,66 | -24,7 |
| | biais % | -26,8 | -30,5 | -16,9 | -10,2 | |
| | CV% | 37,1 | 30,0 | 38,6 | 41,1 | |
| SEI | mCobs pg/mL | 185,21 | 89,60 | 58,01 | 32,81 | -65,5 |
| | biais % | -67,7 | -68,8 | -59,9 | -54,4 | |
| | CV% | 53,5 | 42,0 | 33,1 | 63,8 | |

FIGURE 7

**SEA**: Precipitation of casein and delipidation - plates 101 to 104

| Cref (pg/mL) | mCobs (pg/mL) | S | CV % | Biais % | | Correction factor |
|---|---|---|---|---|---|---|
| 573,25 | 362,9 | 111,8 | 30,8 | -36,7 | | 1,58 |
| 287,5 | 177,3 | 43,4 | 24,5 | -38,3 | | 1,62 |
| 144,5 | 105,0 | 22,8 | 21,7 | -27,4 | | 1,38 |
| 72 | 52,8 | 11,4 | 21,6 | -26,7 | | 1,36 |
| | | | m Biais% | -32,3 | Average factor Fc : | 1,49 |
| | | | S | 5,27 | s: | 0,13 |

| | Plate 101 | Plate 102 | Plate 103 | Plate 104 |
|---|---|---|---|---|
| Cref (ng/mL) | Cobs (ng/mL) | Cobs (ng/mL) | Cobs (ng/mL) | Cobs (ng/mL) |
| 573 | 265,57 | 280,95 | 503,62 | 401,64 |
| 288 | 171,99 | 134,17 | 237,47 | 165,77 |
| 145 | 93,31 | 78,90 | 127,53 | 120,08 |
| 72 | 40,21 | 52,34 | 50,67 | 67,91 |

| | Cref (pg/mL) | | | | |
|---|---|---|---|---|---|
| Cref initial (pg/mL) | Plate 101 | Plate 102 | Plate 103 | Plate 104 | Moyenne |
| 640 | 569 | 568 | 578 | 578 | 573,25 |
| 320 | 285 | 283 | 291 | 291 | 287,5 |
| 160 | 144 | 142 | 146 | 146 | 144,5 |
| 80 | 71 | 71 | 73 | 73 | 72 |

**SEB**: Precipitation of casein and delipidation - plates 101 to 104

| Cref (pg/mL) | mCobs (pg/mL) | S | CV % | Biais % | | Correction factor |
|---|---|---|---|---|---|---|
| 573,25 | 400,0 | 151,1 | 37,8 | -30,2 | | 1,43 |
| 287,5 | 192,6 | 67,2 | 34,9 | -33,0 | | 1,49 |
| 144,5 | 101,9 | 26,4 | 25,9 | -29,5 | | 1,42 |
| 72 | 51,8 | 12,1 | 23,4 | -28,0 | | 1,39 |
| | | | m Biais% | -30,2 | Average factor Fc : | 1,43 |
| | | | S | 1,81 | s: | 0,04 |

| | Plate 101 | Plate 102 | Plate 103 | Plate 104 |
|---|---|---|---|---|
| Cref (ng/mL) | Cobs (ng/mL) | Cobs (ng/mL) | Cobs (ng/mL) | Cobs (ng/mL) |
| 573 | 232,74 | 319,74 | 565,07 | 482,46 |
| 288 | 172,33 | 117,83 | 279,11 | 201,16 |
| 145 | 93,97 | 69,22 | 130,59 | 113,78 |
| 72 | 45,31 | 38,17 | 64,26 | 59,50 |

| | Cref (pg/mL) | | | | |
|---|---|---|---|---|---|
| Cref initial (pg/mL) | Plate 101 | Plate 102 | Plate 103 | Plate 104 | Moyenne |
| 640 | 569 | 568 | 578 | 578 | 573,25 |
| 320 | 285 | 283 | 291 | 291 | 287,5 |
| 160 | 144 | 142 | 146 | 146 | 144,5 |
| 80 | 71 | 71 | 73 | 73 | 72 |

FIGURE 7 NEXT

**SEC**: Precipitation of casein and delipidation - plates 101 to 104

| Cref (pg/mL) | mCobs (pg/mL) | S | CV % | Biais % | | Correction factor |
|---|---|---|---|---|---|---|
| 573,25 | 225,1 | 105,8 | 47,0 | -60,7 | | 2,55 |
| 287,5 | 108,4 | 50,2 | 46,4 | -62,3 | | 2,65 |
| 144,5 | 52,6 | 18,3 | 34,9 | -63,6 | | 2,75 |
| 72 | 27,6 | 7,7 | 28,0 | -61,7 | | 2,61 |
| | | | m Biais% | -62,1 | Average factor Fc : | 2,64 |
| | | | S | 1,03 | s: | 0,08 |

| | Plate 101 | Plate 102 | Plate 103 | Plate 104 |
|---|---|---|---|---|
| Cref (ng/mL) | Cobs (ng/mL) | Cobs (ng/mL) | Cobs (ng/mL) | Cobs (ng/mL) |
| 573 | 107,03 | 164,90 | 322,50 | 305,94 |
| 288 | 74,87 | 57,85 | 163,88 | 136,92 |
| 145 | 35,76 | 37,88 | 66,32 | 70,53 |
| 72 | 19,36 | 23,18 | 31,32 | 36,36 |

| | Cref (pg/mL) | | | | |
|---|---|---|---|---|---|
| Cref initial (pg/mL) | Plate 101 | Plate 102 | Plate 103 | Plate 104 | Moyenne |
| 640 | 569 | 568 | 578 | 578 | 573,25 |
| 320 | 285 | 283 | 291 | 291 | 287,5 |
| 160 | 144 | 142 | 146 | 146 | 144,5 |
| 80 | 71 | 71 | 73 | 73 | 72 |

**SED**: Precipitation of casein and delipidation - plates 101 to 104

| Cref (pg/mL) | mCobs (pg/mL) | s | CV % | Biais % | | Correction factor |
|---|---|---|---|---|---|---|
| 573,25 | 300,8 | 73,1 | 24,3 | -47,5 | | 1,91 |
| 287,5 | 140,5 | 44,9 | 32,0 | -51,1 | | 2,05 |
| 144,5 | 85,2 | 12,1 | 14,2 | -41,1 | | 1,70 |
| 72 | 44,1 | 4,4 | 10,0 | -38,7 | | 1,63 |
| | | | m Biais% | -44,6 | Average factor Fc : | 1,82 |
| | | | S | 4,96 | s: | 0,19 |

| | Plate 101 | Plate 102 | Plate 103 | Plate 104 |
|---|---|---|---|---|
| Cref (ng/mL) | Cobs (ng/mL) | Cobs (ng/mL) | Cobs (ng/mL) | Cobs (ng/mL) |
| 573 | 243,65 | 249,05 | 400,93 | 309,73 |
| 288 | 150,13 | 99,69 | 199,71 | 112,27 |
| 145 | 93,97 | 72,82 | 96,95 | 76,88 |
| 72 | 37,82 | 44,36 | 47,48 | 46,83 |

| | Cref (pg/mL) | | | | |
|---|---|---|---|---|---|
| Cref initial (pg/mL) | Plate 101 | Plate 102 | Plate 103 | Plate 104 | Moyenne |
| 640 | 569 | 568 | 578 | 578 | 573,25 |
| 320 | 285 | 283 | 291 | 291 | 287,5 |
| 160 | 144 | 142 | 146 | 146 | 144,5 |
| 80 | 71 | 71 | 73 | 73 | 72 |

FIGURE 7 NEXT

**SEE**: Precipitation of casein and delipidation - plates 101 to 104

| Cref (pg/mL) | mCobs (pg/mL) | S | CV % | Biais % | | Correction factor |
|---|---|---|---|---|---|---|
| 573,25 | 291,5 | 114,6 | 39,3 | -49,1 | | 1,97 |
| 287,5 | 148,2 | 45,8 | 30,9 | -48,5 | | 1,94 |
| 144,5 | 88,1 | 23,5 | 26,7 | -39,0 | | 1,64 |
| 72 | 43,8 | 10,2 | 23,3 | -39,2 | | 1,64 |
| | | | m Biais% | -43,9 | Average factor Fc : | 1,80 |
| | | | S | 4,86 | s: | 0,18 |

| | Plate 101 | Plate 102 | Plate 103 | Plate 104 |
|---|---|---|---|---|
| Cref (ng/mL) | Cobs (ng/mL) | Cobs (ng/mL) | Cobs (ng/mL) | Cobs (ng/mL) |
| 573 | 247,85 | 152,34 | 409,48 | 356,48 |
| 288 | 170,69 | 82,31 | 185,80 | 154,02 |
| 145 | 93,16 | 53,58 | 104,91 | 100,90 |
| 72 | 40,84 | 31,79 | 46,42 | 56,12 |

| | Cref (pg/mL) | | | | |
|---|---|---|---|---|---|
| Cref initial (pg/mL) | Plate 101 | Plate 102 | Plate 103 | Plate 104 | Moyenne |
| 640 | 569 | 568 | 578 | 578 | 573,25 |
| 320 | 285 | 283 | 291 | 291 | 287,5 |
| 160 | 144 | 142 | 146 | 146 | 144,5 |
| 80 | 71 | 71 | 73 | 73 | 72 |

**SEG**: Precipitation of casein and delipidation - plates 101 to 104

| Cref (pg/mL) | mCobs (pg/mL) | s | CV % | Biais % | | Correction factor |
|---|---|---|---|---|---|---|
| 573,25 | 324,5 | 128,5 | 39,6 | -43,4 | | 1,77 |
| 287,5 | 165,7 | 61,0 | 36,8 | -42,4 | | 1,73 |
| 144,5 | 95,3 | 19,5 | 20,5 | -34,1 | | 1,52 |
| 72 | 49,5 | 5,9 | 11,9 | -31,3 | | 1,46 |
| | | | m Biais% | -37,8 | Average factor Fc : | 1,62 |
| | | | S | 5,20 | s: | 0,16 |

| | Plate 101 | Plate 102 | Plate 103 | Plate 104 |
|---|---|---|---|---|
| Cref (ng/mL) | Cobs (ng/mL) | Cobs (ng/mL) | Cobs (ng/mL) | Cobs (ng/mL) |
| 573 | 231,10 | 226,85 | 500,75 | 339,40 |
| 288 | 160,30 | 125,95 | 253,70 | 122,95 |
| 145 | 91,56 | 75,37 | 122,17 | 92,01 |
| 72 | 41,36 | 49,95 | 51,12 | 55,37 |

| | Cref (pg/mL) | | | | |
|---|---|---|---|---|---|
| Cref initial (pg/mL) | Plate 101 | Plate 102 | Plate 103 | Plate 104 | Moyenne |
| 640 | 569 | 568 | 578 | 578 | 573,25 |
| 320 | 285 | 283 | 291 | 291 | 287,5 |
| 160 | 144 | 142 | 146 | 146 | 144,5 |
| 80 | 71 | 71 | 73 | 73 | 72 |

FIGURE 7 NEXT

**SEH**: Precipitation of casein and delipidation - plates 101 to 104

| **Cref (pg/mL)** | **mCobs (pg/mL)** | **s** | **CV %** | **Biais %** | | **Correction factor** |
|---|---|---|---|---|---|---|
| 573,25 | 419,9 | 155,7 | 37,1 | -26,8 | | 1,37 |
| 287,5 | 199,9 | 60,0 | 30,0 | -30,5 | | 1,44 |
| 144,5 | 120,1 | 46,4 | 38,6 | -16,9 | | 1,20 |
| 72 | 64,7 | 26,6 | 41,1 | -10,2 | | 1,11 |
| | | | **m Biais%** | -21,1 | **Average factor Fc :** | 1,28 |
| | | | S | 8,01 | s: | 0,15 |

| | Plate 101 | Plate 102 | Plate 103 | Plate 104 |
|---|---|---|---|---|
| Cref (ng/mL) | Cobs (ng/mL) | Cobs (ng/mL) | Cobs (ng/mL) | Cobs (ng/mL) |
| 573 | 263,89 | 318,65 | 598,86 | 498,03 |
| 288 | 163,20 | 146,14 | 280,24 | 209,99 |
| 145 | 78,24 | 83,64 | 146,80 | 171,79 |
| 72 | 35,15 | 61,62 | 62,11 | 99,77 |

| | Cref (pg/mL) | | | | |
|---|---|---|---|---|---|
| Cref initial (pg/mL) | Plate 101 | Plate 102 | Plate 103 | Plate 104 | Moyenne |
| 640 | 569 | 568 | 578 | 578 | 573,25 |
| 320 | 285 | 283 | 291 | 291 | 287,5 |
| 160 | 144 | 142 | 146 | 146 | 144,5 |
| 80 | 71 | 71 | 73 | 73 | 72 |

**SEI**: Precipitation of casein and delipidation - plates 101 to 104

| **Cref (pg/mL)** | **mCobs (pg/mL)** | **S** | **CV %** | **Biais %** | | **Correction factor** |
|---|---|---|---|---|---|---|
| 573,25 | 185,2 | 99,1 | 53,5 | -67,7 | | 3,10 |
| 287,5 | 89,6 | 37,7 | 42,0 | -68,8 | | 3,21 |
| 144,5 | 58,0 | 19,2 | 33,1 | -59,9 | | 2,49 |
| 72 | 32,8 | 20,9 | 63,8 | -54,4 | | 2,19 |
| | | | **m Biais%** | -62,7 | **Average factor Fc :** | 2,75 |
| | | | S | 5,89 | s: | 0,48 |

| | Plate 101 | Plate 102 | Plate 103 | Plate 104 |
|---|---|---|---|---|
| Cref (ng/mL) | Cobs (ng/mL) | Cobs (ng/mL) | Cobs (ng/mL) | Cobs (ng/mL) |
| 573 | 125,32 | 101,18 | 322,20 | 192,14 |
| 288 | 72,41 | 55,88 | 142,71 | 87,41 |
| 145 | 40,87 | 41,89 | 75,38 | 73,88 |
| 72 | 7,78 | 35,82 | 29,04 | 58,60 |

FIGURE 7 NEXT

| | Cref (pg/mL) | | | | |
|---|---|---|---|---|---|
| Cref initial (pg/mL) | Plate 101 | Plate 102 | Plate 103 | Plate 104 | Moyenne |
| 640 | 569 | 568 | 578 | 578 | 573,25 |
| 320 | 285 | 283 | 291 | 291 | 287,5 |
| 160 | 144 | 142 | 146 | 146 | 144,5 |
| 80 | 71 | 71 | 73 | 73 | 72 |

- n =4
- Cref : reference concentration corrected by dilution (HCL, NaOH, ...)
- mCobs : average of observed concentration
- s : deviation
- CV = s/m*100
- Biais% (Correction factor of Cobs in function of the bias) = (mCobs - Cref) / Cref *100

FIGURE 7 END

Result synthesis

| | Plate 101 | | Plate 103 | | Plate 104 | |
|---|---|---|---|---|---|---|
| | Biais % | s | Biais % | s | Biais % | s |
| SEA | **-61,4** | 7,0 | **-68,7** | 12,3 | **-48,4** | 12,6 |
| SEB | **-71,6** | 8,0 | **-64,5** | 17,1 | **-53,9** | 9,6 |
| SEC1 | **-97,1** | 0,7 | **-88,3** | 5,7 | **-92,3** | 4,1 |
| SED | **-64,0** | 6,2 | **-76,1** | 7,7 | **-58,8** | 7,3 |
| SEE | **-65,5** | 9,7 | **-72,8** | 9,3 | **-53,8** | 9,3 |
| SEG | **-58,9** | 7,8 | **-70,9** | 11,7 | **-54,4** | 10,0 |
| SEH | **-63,7** | 8,4 | **-67,2** | 14,0 | **-53,9** | 9,6 |
| SEI | **-82,3** | 1,5 | **-79,3** | 6,4 | **-83,0** | 2,8 |

Note: the concentration ultrafiltration being not made strictly under the same conditions, do not average between the plates n°101, 103 and 104.

FIGURE 8

Plate 101 results

| SEA | | |
|---|---|---|
| Cref pg/mL | Cobs pg/mL | Biais % |
| 2856 | 1035,87 | -63,7 |
| 1431 | 685,61 | -52,1 |
| 723 | 226,43 | -68,7 |
| 356 | 139,06 | -60,9 |
| | **mBiais %** | **-61,4** |
| | S | 6,96084904 |

| SEE | | |
|---|---|---|
| Cref pg/mL | Cobs pg/mL | Biais % |
| 2856 | 775,58 | -72,8 |
| 1431 | 679,12 | -52,5 |
| 723 | 194,25 | -73,1 |
| 356 | 129,79 | -63,5 |
| | **mBiais %** | **-65,5** |
| | s | 9,72835302 |

| SEB | | |
|---|---|---|
| Cref pg/mL | Cobs pg/mL | Biais % |
| 2856 | 723,30 | -74,7 |
| 1431 | 547,02 | -61,8 |
| 723 | 140,20 | -80,6 |
| 356 | 108,59 | -69,5 |
| | **mBiais %** | **-71,6** |
| | S | 7,99124297 |

| SEG | | |
|---|---|---|
| Cref pg/mL | Cobs pg/mL | Biais % |
| 2856 | 920,22 | -67,8 |
| 1431 | 700,38 | -51,1 |
| 723 | 268,33 | -62,9 |
| 356 | 163,83 | -54,0 |
| | **mBiais %** | **-58,9** |
| | s | 7,75564663 |

| SEC1 | | |
|---|---|---|
| Cref pg/mL | Cobs pg/mL | Biais % |
| 2856 | 80,24 | -97,2 |
| 1431 | 55,46 | -96,1 |
| 723 | 15,11 | -97,9 |
| 356 | 9,39 | -97,4 |
| | **mBiais %** | **-97,1** |
| | S | 0,74733565 |

| SEH | | |
|---|---|---|
| Cref pg/mL | Cobs pg/mL | Biais % |
| 2856 | 1275,87 | -55,3 |
| 1431 | 606,07 | -57,6 |
| 723 | 206,66 | -71,4 |
| 356 | 105,42 | -70,4 |
| | **mBiais %** | **-63,7** |
| | s | 8,38673648 |

| SED | | |
|---|---|---|
| Cref pg/mL | Cobs pg/mL | Biais % |
| 2856 | 874,07 | -69,4 |
| 1431 | 636,47 | -55,5 |
| 723 | 231,84 | -67,9 |
| 356 | 130,72 | -63,3 |
| | **mBiais %** | **-64,0** |
| | S | 6,24398921 |

| SEI | | |
|---|---|---|
| Cref pg/mL | Cobs pg/mL | Biais % |
| 2856 | 494,66 | -82,7 |
| 1431 | 271,90 | -81,0 |
| 723 | 114,59 | -84,2 |
| 356 | 66,99 | -81,2 |
| | **mBiais %** | **-82,3** |
| | s | 1,47237185 |

FIGURE 8 NEXT

Plate 103 results

| SEA | | |
|---|---|---|
| Cref pg/mL | Cobs pg/mL | Biais % |
| 2903 | 1437,77 | -50,5 |
| 1461 | 389,54 | -73,3 |
| 733 | 191,58 | -73,9 |
| 366 | 83,56 | -77,2 |
| | **mBiais %** | **-68,7** |
| | S | 12,2760313 |

| SEE | | |
|---|---|---|
| Cref pg/mL | Cobs pg/mL | Biais % |
| 2903 | 1188,23 | -59,1 |
| 1461 | 351,42 | -75,9 |
| 733 | 172,64 | -76,4 |
| 366 | 73,69 | -79,9 |
| | **mBiais %** | **-72,8** |
| | s | 9,33968685 |

| SEB | | |
|---|---|---|
| Cref pg/mL | Cobs pg/mL | Biais % |
| 2903 | 1758,08 | -39,4 |
| 1461 | 468,90 | -67,9 |
| 733 | 190,85 | -74,0 |
| 366 | 84,67 | -76,9 |
| | **mBiais %** | **-64,5** |
| | S | 17,1474899 |

| SEG | | |
|---|---|---|
| Cref pg/mL | Cobs pg/mL | Biais % |
| 2903 | 1343,52 | -53,7 |
| 1461 | 391,24 | -73,2 |
| 733 | 167,10 | -77,2 |
| 366 | 75,53 | -79,4 |
| | **mBiais %** | **-70,9** |
| | s | 11,7176914 |

| SEC1 | | |
|---|---|---|
| Cref pg/mL | Cobs pg/mL | Biais % |
| 2903 | 566,46 | -80,5 |
| 1461 | 178,83 | -87,8 |
| 733 | 55,74 | -92,4 |
| 366 | 27,27 | -92,5 |
| | **mBiais %** | **-88,3** |
| | S | 5,6618116 |

| SEH | | |
|---|---|---|
| Cref pg/mL | Cobs pg/mL | Biais % |
| 2903 | 1547,78 | -46,7 |
| 1461 | 441,68 | -69,8 |
| 733 | 180,48 | -75,4 |
| 366 | 84,18 | -77,0 |
| | **mBiais %** | **-67,2** |
| | s | 14,0290594 |

| SED | | |
|---|---|---|
| Cref pg/mL | Cobs pg/mL | Biais % |
| 2903 | 1026,04 | -64,7 |
| 1461 | 310,79 | -78,7 |
| 733 | 149,36 | -79,6 |
| 366 | 68,04 | -81,4 |
| | **mBiais %** | **-76,1** |
| | S | 7,7134035 |

| SEI | | |
|---|---|---|
| Cref pg/mL | Cobs pg/mL | Biais % |
| 2903 | 869,00 | -70,1 |
| 1461 | 292,09 | -80,0 |
| 733 | 125,29 | -82,9 |
| 366 | 57,75 | -84,2 |
| | **mBiais %** | **-79,3** |
| | s | 6,4032336 |

FIGURE 8 NEXT

Plate 104 results

| SEA | | | | |
|---|---|---|---|---|
| Cref pg/mL | Cobs pg/mL | Biais % | | Fc[] |
| 2903 | 1592,38 | -45,1 | | 1,82 |
| 1461 | 501,53 | -65,7 | | 2,91 |
| 733 | 472,00 | -35,6 | | 1,55 |
| 366 | 193,95 | -47,0 | | 1,89 |
| | **mBiais %** | **-48,4** | **mFc[]** | **2,04** |
| | s | 12,5763475 | s | 0,60 |

| SEB | | | | |
|---|---|---|---|---|
| Cref pg/mL | Cobs pg/mL | Biais % | | Fc[] |
| 2903 | 1588,26 | -45,3 | | 1,83 |
| 1461 | 498,88 | -65,9 | | 2,93 |
| 733 | 388,43 | -47,0 | | 1,89 |
| 366 | 156,10 | -57,3 | | 2,34 |
| | **mBiais %** | **-53,9** | **mFc[]** | **2,25** |
| | s | 9,59904659 | s | 0,51 |

| SEC1 | | | | |
|---|---|---|---|---|
| Cref pg/mL | Cobs pg/mL | Biais % | | Fc[] |
| 2903 | 393,68 | -86,4 | | 7,37 |
| 1461 | 99,96 | -93,2 | | 14,62 |
| 733 | 44,41 | -93,9 | | 16,50 |
| 366 | 15,23 | -95,8 | | 24,04 |
| | **mBiais %** | **-92,3** | **mFc[]** | **15,63** |
| | s | 4,09472919 | s | 6,85 |

| SED | | | | |
|---|---|---|---|---|
| Cref pg/mL | Cobs pg/mL | Biais % | | Fc[] |
| 2903 | 1257,03 | -56,7 | | 2,31 |
| 1461 | 465,74 | -68,1 | | 3,14 |
| 733 | 362,93 | -50,5 | | 2,02 |
| 366 | 147,09 | -59,8 | | 2,49 |
| | **mBiais %** | **-58,8** | **mFc[]** | **2,49** |
| | s | 7,33562104 | s | 0,47 |

| SEG | | | | |
|---|---|---|---|---|
| Cref pg/mL | Cobs pg/mL | Biais % | | Fc[] |
| 2903 | 1491,88 | -48,6 | | 1,95 |
| 1461 | 485,13 | -66,8 | | 3,01 |
| 733 | 408,00 | -44,3 | | 1,80 |
| 366 | 154,46 | -57,8 | | 2,37 |
| | **mBiais %** | **-54,4** | **mFc[]** | **2,28** |
| | s | 9,9993651 | s | 0,54 |

FIGURE 8 NEXT

Plate 104 results NEXT

| SEH | | | | | |
|---|---|---|---|---|---|
| Cref pg/mL | Cobs pg/mL | Biais % | | Fc[] | |
| 2903 | 2738,81 | -5,7 | | 1,06 | aberrant value |
| 1461 | 636,78 | -56,4 | | 2,29 | |
| 733 | 416,08 | -43,2 | | 1,76 | |
| 366 | 139,19 | -62,0 | | 2,63 | |
| | **mBiais %** | **-53,9** | **mFc[]** | **2,23** | |
| | s | 9,62165568 | s | 0,44 | |

| SEI | | | | |
|---|---|---|---|---|
| Cref pg/mL | Cobs pg/mL | Biais % | | Fc[] |
| 2903 | 569,06 | -80,4 | | 5,10 |
| 1461 | 231,37 | -84,2 | | 6,31 |
| 733 | 139,68 | -80,9 | | 5,25 |
| 366 | 50,06 | -86,3 | | 7,31 |
| | **mBiais %** | **-83,0** | **mFc[]** | **5,99** |
| | s | 2,79203917 | s | 1,03 |

- Cref : reference concentration corrected by dilution (HCL, NaOH, …)
- Cobs : observed concentration
- Biais % = (Cobs - Cref)/Cref *100
- mBiais % : average of relative bias
- s deviation

- **mFc[], excluding the aberrant -5.7%/1.06 value**

FIGURE 8 END

Europäisches Patentamt
European Patent Office
Office européen des brevets

# EUROPEAN SEARCH REPORT

Application Number
EP 11 15 6131

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ERIC A. E. GARBER ET AL: "Simultaneous Multiplex Detection and Confirmation of the Proteinaceous Toxins Abrin, Ricin, Botulinum Toxins, and Staphylococcus Enterotoxins A, B, and C in Food", JOURNAL OF AGRICULTURAL AND FOOD CHEMISTRY, vol. 58, no. 11, 9 June 2010 (2010-06-09), pages 6600-6607, XP55003120, ISSN: 0021-8561, DOI: 10.1021/jf100789n * the whole document * ----- | 1-5 | INV. C07K16/12 G01N33/569 |
| A | A. Yu. Rubina ET AL: "Simultaneous Detection of Seven Staphylococcal Enterotoxins: Development of Hydrogel Biochips for Analytical and Practical Application", Analytical Chemistry, vol. 82, no. 21, 1 November 2010 (2010-11-01), pages 8881-8889, XP55003417, ISSN: 0003-2700, DOI: 10.1021/ac1016634 * the whole document * ----- | 1-15 | TECHNICAL FIELDS SEARCHED (IPC) C07K G01N |
| A | LAPEYRE CHRISTIANE ET AL: "Enzyme immunoassay of staphylococcal enterotoxins in dairy products with cleanup and concentration by immunoaffinity column", JOURNAL OF AOAC INTERNATIONAL, vol. 84, no. 5, September 2001 (2001-09), pages 1587-1592, XP9150590, ISSN: 1060-3271 * the whole document * ----- | 1-15 | |
| | -/-- | | |

The present search report has been drawn up for all claims

1

| Place of search | Date of completion of the search | Examiner |
|---|---|---|
| Munich | 25 July 2011 | Kalsner, Inge |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

## EUROPEAN SEARCH REPORT

Application Number
EP 11 15 6131

| Category | Citation of document with indication, where appropriate, of relevant passages | Relevant to claim | CLASSIFICATION OF THE APPLICATION (IPC) |
|---|---|---|---|
| X | DE OLIVEIRA TEREZA CRISTINA R M ET AL: "Low cost production and purification of polyclonal antibodies to staphylococcal enterotoxin A", REVISTA DE MICROBIOLOGIA, vol. 30, no. 2, June 1999 (1999-06), pages 120-124, XP002653016, ISSN: 0001-3714 * the whole document * ----- | 15 | |
| A | PAULY DIANA ET AL: "Simultaneous quantification of five bacterial and plant toxins from complex matrices using a multiplexed fluorescent magnetic suspension assay", THE ANALYST ENGLAND,, vol. 134, no. 10, 1 October 2009 (2009-10-01), pages 2028-2039, XP009150509, DOI: DOI:10.1039/B911525K * abstract * ----- | 1-15 | TECHNICAL FIELDS SEARCHED (IPC) |

The present search report has been drawn up for all claims

1

| Place of search | Date of completion of the search | Examiner |
|---|---|---|
| Munich | 25 July 2011 | Kalsner, Inge |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **JONES ; KAHN.** *J. Bacteriol.,* 1986, vol. 166, 29-33
- **BETLEY ; MEKALANOS.** *J. Bacteriol.,* 1988, vol. 170 (1), 34-41
- **COUCH.** *J. Bacteriol.,* 1988, vol. 170, 2954-2960
- **BAYLES ; LANDOLO.** *J. Bacteriol.,* 1989, vol. 171, 4799-4806
- **DINGUES.** *Clin. Microbiol. Rev.,* 2000, vol. 13, 16-34
- **BERGDOLL.** *J. Bacteriol.,* 1965, vol. 90 (5), 1481-1485
- **MARR.** *Infect. Immun.,* 1993, vol. 61, 4254-4262
- **BERGDOLL.** *Lancet,* 1981, vol. 1, 1017-1021
- **BLOMSTER-HAUTAMAA.** *J. Biol. Chem.,* 1986, vol. 261, 15783-15786
- **LINA.** *J. Infect. Dis.,* 2004, vol. 189 (12), 2334-2336
- **REN et al.** *J. Exp. Med.,* 1994, vol. 180 (5), 1675-1683
- **JARRAUD.** *J. Immunol.,* 2001, vol. 166 (1), 669-677
- **ORWIN.** *Infect. Immun.,* 2001, vol. 69 (1), 360-366
- **LETERTRE.** *Mol. Cell Probes,* 2003, vol. 17, 227-235
- **OMOE.** *J. Clin. Microbiol.,* 2002, vol. 40, 857-862
- **SU ; WONG.** *J. Food Prot.,* 1996, vol. 59 (3), 327-330
- **MUNSON.** *Infect. Immun.,* 1998, vol. 66, 3337-3348
- **ONO.** *Infect. Immun.,* 2008, vol. 76 (11), 4999-5005
- **SMITH ; JOHNSON.** *Gene,* 1988, vol. 67 (1), 31-40
- **HABIG.** *J. Biol. Chem.,* 1974, vol. 249, 7130-7139
- **GAMPFER.** *Vaccine,* 2002, vol. 20, 3675-3684
- **BORKOWSKI.** *Clin. Tech. Small Anim. Pract.,* 1999, vol. 14 (1), 44-49
- **GAMPFER et al.** *Vaccine,* 2002, vol. 20, 3675-3684
- **SOEJIMA.** *Int. J. Food Microbiol.,* 2004, vol. 93, 185-194
- **MACALUSO et al.** *Analusis,* 2000, vol. 28 (7), 610-615

SEQUENCE LISTING

<110> UNIVERSITE DE STRASBOURG
PREVOST, Gilles
KELLER, Daniel
MASOUD, Khaldoun
BARASINO, Charline

<120> IMMUNOABSORBED ANTI-STAPHYLOCOCCAL ENTEROTOXINS POLYCLONAL ANTIBODIES, PREPARATION AND USES THEREOF

<130> BIP209387EP00

<160> 22

<170> PatentIn version 3.5

<210> 1
<211> 37
<212> DNA
<213> Artificial Sequence

<220>
<223> Primer BamH1-setBf

<400> 1
agtaaggatc cgagagagtc aaccagatcc taaacca 37

<210> 2
<211> 42
<212> DNA
<213> Artificial Sequence

<220>
<223> Primer EcoR1-setBr

<400> 2
aagtatagaa ttcactaaca cttcatattt actttctaaa at 42

<210> 3
<211> 39
<212> DNA
<213> Artificial Sequence

<220>
<223> primer EcoR1-setC1f

<400> 3
aagtatagaa ttcgagagcc aaccagaccc tacgccaga 39

<210> 4
<211> 39
<212> DNA
<213> Artificial Sequence

<220>
<223> Primer EcoR1-setC1r

<400> 4
aagtatagaa ttctactttc actttttata tcaaaatgg 39

<210> 5
<211> 42
<212> DNA
<213> Artificial Sequence

<220>
<223> Primer EcoR1-setDf

<400> 5
aagtatagaa ttcaaacatt cttatgcaga taaaaatcca at 42

<210> 6
<211> 39
<212> DNA
<213> Artificial Sequence

<220>
<223> Primer EcoR1-setDr

<400> 6
aagtatagaa ttcttgaaat ggctttagtg tctgatgtt 39

<210> 7
<211> 37
<212> DNA
<213> Artificial Sequence

<220>
<223> Primer EcoR1-setEf

<400> 7
agtaaggatc cagcgaagaa ataaatgaaa aagattt 37

<210> 8
<211> 39
<212> DNA
<213> Artificial Sequence

<220>
<223> Primer EcoR1-setEr

<400> 8
aagtatagaa ttcagttgtg tataaataca aatcaatat 39

<210> 9
<211> 36
<212> DNA
<213> Artificial Sequence

<220>
<223> Primer EcoR1-seGf

<400> 9
agtaaggatc ccaacccgat cctaaattag acgaac 36

<210> 10
<211> 42
<212> DNA
<213> Artificial Sequence

<220>
<223> Primer EcoR1-SeGr

<400> 10
aagtatgaat tctcagtgag tattaagaaa tacttccatt tt 42

<210> 11

<211> 37
<212> DNA
<213> Artificial Sequence

<220>
<223> Primer EcoR1-setHf

<400> 11
agtaaggatc cgaagattta cacgataaaa gtgagtt 37

<210> 12
<211> 39
<212> DNA
<213> Artificial Sequence

<220>
<223> Primer EcoR1-setHr

<400> 12
aagtatagaa ttctgatcta gaaattttca ttgattata 39

<210> 13
<211> 37
<212> DNA
<213> Artificial Sequence

<220>
<223> Primer BamH1-setIf

<400> 13
agtaaggatc ccaaggtgat attggtgtag gtaactt 37

<210> 14
<211> 38
<212> DNA
<213> Artificial Sequence

<220>
<223> Primer EcoR1-setIr

<400> 14
aagtatagaa ttcgttacta tctacatatg atatttcg 38

<210> 15
<211> 238
<212> PRT
<213> Staphylococcus aureus

<400> 15

Gly Pro Leu Gly Ser Ser Glu Lys Ser Glu Glu Ile Asn Glu Lys Asp
1               5                   10                  15

Leu Arg Lys Lys Ser Glu Leu Gln Gly Thr Ala Leu Gly Asn Leu Lys
            20                  25                  30

Gln Ile Tyr Tyr Tyr Asn Glu Lys Ala Lys Thr Glu Asn Lys Glu Ser
        35                  40                  45

His Asp Gln Phe Leu Gln His Thr Ile Leu Phe Lys Gly Phe Phe Thr
    50                  55                  60

Asp His Ser Trp Tyr Asn Asp Leu Leu Val Asp Phe Asp Ser Lys Asp
65 70 75 80

Ile Val Asp Lys Tyr Lys Gly Lys Lys Val Asp Leu Tyr Gly Ala Tyr
85 90 95

Tyr Gly Tyr Gln Cys Ala Gly Gly Thr Pro Asn Lys Thr Ala Cys Met
100 105 110

Tyr Gly Gly Val Thr Leu His Asp Asn Asn Arg Leu Thr Glu Glu Lys
115 120 125

Lys Val Pro Ile Asn Leu Trp Leu Asp Gly Lys Gln Asn Thr Val Pro
130 135 140

Leu Glu Thr Val Lys Thr Asn Lys Lys Asn Val Thr Val Gln Glu Leu
145 150 155 160

Asp Leu Gln Ala Arg Arg Tyr Leu Gln Glu Lys Tyr Asn Leu Tyr Asn
165 170 175

Ser Asp Val Phe Asp Gly Lys Val Gln Arg Gly Leu Ile Val Phe His
180 185 190

Thr Ser Thr Glu Pro Ser Val Asn Tyr Asp Leu Phe Gly Ala Gln Gly
195 200 205

Gln Tyr Ser Asn Thr Leu Leu Arg Ile Tyr Arg Asp Asn Lys Thr Ile
210 215 220

Asn Ser Glu Asn Met His Ile Asp Ile Tyr Leu Tyr Thr Ser
225 230 235

<210> 16
<211> 244
<212> PRT
<213> Staphylococcus aureus

<400> 16

Gly Pro Leu Gly Ser Glu Ser Gln Pro Asp Pro Lys Pro Asp Glu Leu
1 5 10 15

His Lys Ser Ser Lys Phe Thr Gly Leu Met Glu Asn Met Lys Val Leu
20 25 30

Tyr Asp Asp Asn His Val Ser Ala Ile Asn Val Lys Ser Ile Asp Gln
35 40 45

Phe Leu Tyr Phe Asp Leu Ile Tyr Ser Ile Lys Asp Thr Lys Leu Gly
50 55 60

Asn Tyr Asp Asn Val Arg Val Glu Phe Lys Asn Lys Asp Leu Ala Asp

65 70 75 80

Lys Tyr Lys Asp Lys Tyr Val Asp Val Phe Gly Ala Asn Tyr Tyr Tyr
85 90 95

Gln Cys Tyr Phe Ser Lys Lys Thr Asn Asp Ile Asn Ser His Gln Thr
100 105 110

Asp Lys Arg Lys Thr Cys Met Tyr Gly Gly Val Thr Glu His Asn Gly
115 120 125

Asn Gln Leu Asp Lys Tyr Arg Ser Ile Thr Val Arg Val Phe Glu Asp
130 135 140

Gly Lys Asn Leu Leu Ser Phe Asp Val Gln Thr Asn Lys Lys Lys Val
145 150 155 160

Thr Ala Gln Glu Leu Asp Tyr Leu Thr Arg His Tyr Leu Val Lys Asn
165 170 175

Lys Lys Leu Tyr Glu Phe Asn Asn Ser Pro Tyr Glu Thr Gly Tyr Ile
180 185 190

Lys Phe Ile Glu Asn Glu Asn Ser Phe Trp Tyr Asp Met Met Pro Ala
195 200 205

Pro Gly Asp Lys Phe Asp Gln Ser Lys Tyr Leu Met Met Tyr Asn Asp
210 215 220

Asn Lys Met Val Asp Ser Lys Asp Val Lys Ile Glu Val Tyr Leu Thr
225 230 235 240

Thr Lys Lys Lys

<210> 17
<211> 247
<212> PRT
<213> Staphylococcus aureus

<400> 17

Gly Pro Leu Gly Ser Pro Glu Phe Glu Ser Gln Pro Asp Pro Thr Pro
1 5 10 15

Asp Glu Leu His Lys Ala Ser Lys Phe Thr Gly Leu Met Glu Asn Met
20 25 30

Lys Val Leu Tyr Asp Asp His Tyr Val Ser Ala Thr Lys Val Lys Ser
35 40 45

Val Asp Lys Phe Leu Ala His Asp Leu Ile Tyr Asn Ile Ser Asp Lys
50 55 60

Lys Leu Lys Asn Tyr Asp Lys Val Lys Thr Glu Leu Leu Asn Glu Gly
65 70 75 80

Leu Ala Lys Lys Tyr Lys Asp Glu Val Val Asp Val Tyr Gly Ser Asn
85 90 95

Tyr Tyr Val Asn Cys Tyr Phe Ser Ser Lys Asp Asn Val Gly Lys Val
100 105 110

Thr Gly Gly Lys Thr Cys Met Tyr Gly Gly Ile Thr Lys His Glu Gly
115 120 125

Asn His Phe Asp Asn Gly Asn Leu Gln Asn Val Leu Ile Arg Val Tyr
130 135 140

Glu Asn Lys Arg Asn Thr Ile Ser Phe Glu Val Gln Thr Asp Lys Lys
145 150 155 160

Ser Val Thr Ala Gln Glu Leu Asp Ile Lys Ala Arg Asn Phe Leu Ile
165 170 175

Asn Lys Lys Asn Leu Tyr Glu Phe Asn Ser Ser Pro Tyr Glu Thr Gly
180 185 190

Tyr Ile Lys Phe Ile Glu Asn Asn Gly Asn Thr Phe Trp Tyr Asp Met
195 200 205

Met Pro Ala Pro Gly Asp Lys Phe Asp Gln Ser Lys Tyr Leu Met Met
210 215 220

Tyr Asn Asp Asn Lys Thr Val Asp Ser Lys Ser Val Lys Ile Glu Val
225 230 235 240

His Leu Thr Thr Lys Asn Gly
245

<210> 18
<211> 207
<212> PRT
<213> Staphylococcus aureus

<400> 18

Lys His Ser Tyr Ala Asp Lys Asn Pro Ile Ile Gly Glu Asn Lys Ser
1 5 10 15

Thr Gly Asp Gln Phe Leu Glu Asn Thr Leu Leu Tyr Lys Lys Phe Phe
20 25 30

Thr Asp Leu Ile Asn Phe Glu Asp Leu Leu Ile Asn Phe Asn Ser Lys
35 40 45

Glu Met Ala Gln His Phe Lys Ser Lys Asn Val Asp Val Tyr Pro Ile

```
    50                  55                  60

Arg Tyr Ser Ile Asn Cys Tyr Gly Gly Glu Ile Asp Arg Thr Ala Cys
65                  70                  75                  80

Thr Tyr Gly Gly Val Thr Pro His Glu Gly Asn Lys Leu Lys Glu Arg
                85                  90                  95

Lys Lys Ile Pro Ile Asn Leu Trp Ile Asn Gly Val Gln Lys Glu Val
            100                 105                 110

Ser Leu Asp Lys Val Gln Thr Asp Lys Lys Asn Val Thr Val Gln Glu
        115                 120                 125

Leu Asp Ala Gln Ala Arg Arg Tyr Leu Gln Lys Asp Leu Lys Leu Tyr
    130                 135                 140

Asn Asn Asp Thr Leu Gly Gly Lys Ile Gln Arg Gly Lys Ile Glu Phe
145                 150                 155                 160

Asp Ser Ser Asp Gly Ser Lys Val Ser Tyr Asp Leu Phe Asp Val Lys
                165                 170                 175

Gly Asp Phe Pro Glu Lys Gln Leu Arg Ile Tyr Ser Asp Asn Lys Thr
            180                 185                 190

Leu Ser Thr Glu His Leu His Ile Asp Ile Tyr Leu Tyr Glu Lys
        195                 200                 205

<210>  19
<211>  235
<212>  PRT
<213>  Staphylococcus aureus

<400>  19

Gly Pro Leu Gly Ser Ser Glu Glu Ile Asn Glu Lys Asp Leu Arg Lys
1               5                   10                  15

Lys Ser Glu Leu Gln Arg Asn Ala Leu Ser Asn Leu Arg Gln Ile Tyr
            20                  25                  30

Tyr Tyr Asn Glu Lys Ala Ile Thr Glu Asn Lys Glu Ser Asp Asp Gln
        35                  40                  45

Phe Leu Glu Asn Thr Leu Leu Phe Lys Gly Phe Phe Thr Gly His Pro
    50                  55                  60

Trp Tyr Asn Asp Leu Leu Val Asp Leu Gly Ser Lys Asp Ala Thr Asn
65                  70                  75                  80

Lys Tyr Lys Gly Lys Lys Val Asp Leu Tyr Gly Ala Tyr Tyr Gly Tyr
                85                  90                  95
```

Gln Cys Ala Gly Gly Thr Pro Asn Lys Thr Ala Cys Met Tyr Gly Gly
100 105 110

Val Thr Leu His Asp Asn Asn Arg Leu Thr Glu Glu Lys Lys Val Pro
115 120 125

Ile Asn Leu Trp Ile Asp Gly Lys Gln Thr Thr Val Pro Ile Asp Lys
130 135 140

Val Lys Thr Ser Lys Lys Glu Val Thr Val Gln Glu Leu Asp Leu Gln
145 150 155 160

Ala Arg His Tyr Leu His Gly Lys Phe Gly Leu Tyr Asn Ser Asp Ser
165 170 175

Phe Gly Gly Lys Val Gln Arg Gly Leu Ile Val Phe His Ser Ser Glu
180 185 190

Gly Ser Thr Val Ser Tyr Asp Leu Phe Asp Ala Gln Gly Gln Tyr Pro
195 200 205

Asp Thr Leu Leu Arg Ile Tyr Arg Asp Asn Lys Thr Ile Asn Ser Glu
210 215 220

Asn Leu His Ile Asp Leu Tyr Leu Tyr Thr Thr
225 230 235

<210> 20
<211> 241
<212> PRT
<213> Staphylococcus aureus

<400> 20

Gly Pro Leu Gly Ser Pro Glu Phe Gln Pro Asp Pro Lys Leu Asp Glu
1 5 10 15

Leu Asn Lys Val Ser Asp Tyr Lys Asn Asn Lys Gly Thr Met Gly Asn
20 25 30

Val Met Asn Leu Tyr Thr Ser Pro Pro Val Glu Gly Arg Gly Val Ile
35 40 45

Asn Ser Arg Gln Phe Leu Ser His Asp Leu Ile Phe Pro Ile Glu Tyr
50 55 60

Lys Ser Tyr Asn Glu Val Lys Thr Glu Leu Glu Asn Thr Glu Leu Ala
65 70 75 80

Asn Asn Tyr Lys Asp Lys Lys Val Asp Ile Phe Gly Val Pro Tyr Phe
85 90 95

Tyr Thr Cys Ile Ile Pro Lys Ser Glu Pro Asp Ile Asn Gln Asn Phe

100 105 110

Gly Gly Cys Cys Met Tyr Gly Gly Leu Thr Phe Asn Ser Ser Glu Asn
115 120 125

Glu Arg Asp Lys Leu Ile Thr Val Gln Val Thr Ile Asp Asn Arg Gln
130 135 140

Ser Leu Gly Phe Thr Ile Thr Thr Asn Lys Asn Met Val Thr Ile Gln
145 150 155 160

Glu Leu Asp Tyr Lys Ala Arg His Trp Leu Thr Lys Glu Lys Lys Leu
165 170 175

Tyr Glu Phe Asp Gly Ser Ala Phe Glu Ser Gly Tyr Ile Lys Phe Thr
180 185 190

Glu Lys Asn Asn Thr Ser Phe Trp Phe Asp Leu Phe Pro Lys Lys Glu
195 200 205

Leu Val Pro Phe Val Pro Tyr Lys Phe Leu Asn Ile Tyr Gly Asp Asn
210 215 220

Lys Val Val Asp Ser Lys Ser Ile Lys Met Glu Val Phe Leu Asn Thr
225 230 235 240

His

<210> 21
<211> 222
<212> PRT
<213> Staphylococcus aureus

<400> 21

Gly Pro Leu Gly Ser Glu Asp Leu His Asp Lys Ser Glu Leu Thr Asp
1 5 10 15

Leu Ala Leu Ala Asn Ala Tyr Gly Gln Tyr Asn His Pro Phe Ile Lys
20 25 30

Glu Asn Ile Lys Ser Asp Glu Ile Ser Gly Glu Lys Asp Leu Ile Phe
35 40 45

Arg Asn Gln Gly Asp Ser Gly Asn Asp Leu Arg Val Lys Phe Ala Thr
50 55 60

Ala Asp Leu Ala Gln Lys Phe Lys Asn Lys Asn Val Asp Ile Tyr Gly
65 70 75 80

Ala Ser Phe Tyr Tyr Lys Cys Glu Lys Ile Ser Glu Asn Ile Ser Glu
85 90 95

Cys Leu Tyr Gly Gly Thr Thr Leu Asn Ser Glu Lys Leu Ala Gln Glu
100 105 110

Arg Val Ile Gly Ala Asn Val Trp Val Asp Gly Ile Gln Lys Glu Thr
115 120 125

Glu Leu Ile Arg Thr Asn Lys Lys Asn Val Thr Leu Gln Glu Leu Asp
130 135 140

Ile Lys Ile Arg Lys Ile Leu Ser Asp Lys Tyr Lys Ile Tyr Tyr Lys
145 150 155 160

Asp Ser Glu Ile Ser Lys Gly Leu Ile Glu Phe Asp Met Lys Thr Pro
165 170 175

Arg Asp Tyr Ser Phe Asp Ile Tyr Asp Leu Lys Gly Glu Asn Asp Tyr
180 185 190

Glu Ile Asp Lys Ile Tyr Glu Asp Asn Lys Thr Leu Lys Ser Asp Asp
195 200 205

Ile Ser His Ile Asp Val Asn Leu Tyr Thr Lys Lys Lys Val
210 215 220

<210> 22
<211> 223
<212> PRT
<213> Staphylococcus aureus

<400> 22

Gly Pro Leu Gly Ser Gln Gly Asp Ile Gly Val Gly Asn Leu Arg Asn
1 5 10 15

Phe Tyr Thr Lys His Asp Tyr Ile Asp Leu Lys Gly Val Thr Asp Lys
20 25 30

Asn Leu Pro Ile Ala Asn Gln Leu Glu Phe Ser Thr Gly Thr Asn Asp
35 40 45

Leu Ile Ser Glu Ser Asn Asn Trp Asp Glu Ile Ser Lys Phe Lys Gly
50 55 60

Lys Lys Leu Asp Ile Phe Gly Ile Asp Tyr Asn Gly Pro Cys Lys Ser
65 70 75 80

Lys Tyr Met Tyr Gly Gly Ala Thr Leu Ser Gly Gln Tyr Leu Asn Ser
85 90 95

Ala Arg Lys Ile Pro Ile Asn Leu Trp Val Asn Gly Lys His Lys Thr
100 105 110

Ile Ser Thr Asp Lys Ile Ala Thr Asn Lys Lys Leu Val Thr Ala Gln

```
            115                 120                 125

Glu Ile Asp Val Lys Leu Arg Arg Tyr Leu Gln Glu Glu Tyr Asn Ile
    130                 135                 140

Tyr Gly His Asn Asn Thr Gly Lys Gly Lys Glu Tyr Gly Tyr Lys Ser
145                 150                 155                 160

Lys Phe Tyr Ser Gly Phe Asn Asn Gly Lys Val Leu Phe His Leu Asn
                165                 170                 175

Asn Glu Lys Ser Phe Ser Tyr Asp Leu Phe Tyr Thr Gly Asp Gly Leu
            180                 185                 190

Pro Val Ser Phe Leu Lys Ile Tyr Glu Asp Asn Lys Ile Ile Glu Ser
        195                 200                 205

Glu Lys Phe His Leu Asp Val Glu Ile Ser Tyr Val Asp Ser Asn
    210                 215                 220
```